# NEW EUROPEAN PATENT SPECIFICATION

(11) **EP 1 592 793 B2**
(45) Date of publication and mention of the opposition decision: **07.05.2014**
(45) Mention of the grant of the patent: 15.07.2009
(21) Application number: 04709580.7
(22) Date of filing: 10.02.2004
(51) Int. Cl.: C12N 15/113, A61K 31/7088, A61P 35/00

(54) **OLIGOMERIC COMPOUNDS FOR THE MODULATION OF SURVIVIN EXPRESSION**
OLIGOMERE VERBINDUNGEN ZUR MODULATION DER EXPRESSION VON SURVIVIN
COMPOSES OLIGOMERES MODULANT L'EXPRESSION DE LA SURVIVINE

(30) Priority: 10.02.2003 DK 200300183; 18.11.2003 DK 200301708
(43) Date of publication of application: 09.11.2005
(73) Proprietor: Santaris Pharma A/S, 2970 Hørsholm (DK)
(72) Inventor: HANSEN, Jens Bo Rode, DK-2900 Hellerup (DK); THRUE, Charlotte, Albaek, DK-1327 Copenhagen K (DK); PETERSEN, Kamille, Dumong, DK-2800 Lyngby (DK); WESTERGAARD, Majken, DK-3460 Birkerod (DK); WISSENBACH, Margit, DK-3480 Fredensborg (DK)
(74) Representative: Harding, Charles Thomas
(86) International application number: PCT/DK2004/000096
(87) International publication number: WO 2004/069991

(56) References cited:
- WO-A-01/48190
- WO-A-03/091384
- WO-A1-91/12811
- WO-A2-03/102019
- US-A1- 2002 137 708
- US-A1- 2004 005 567
- SHANKAR SAI LATHA ET AL.: "Survivin inhibition induces human neural tumor cell death through caspase-independent and -dependent pathways" JOURNAL OF NEUROCHEMISTRY, vol. 79, 2001, pages 426-436, XP002297490
- OLIE ROBERT A. ET AL.: "A novel antisense oligonucleotide targeting Survivin expression induces apoptosis and sensitizes lung cancer cells to chemotherapy" CANCER RESEARCH, AMERICAN ASSOCIATION FOR CANCER RESEARCH, BALTIMORE, MD, US, vol. 60, no. 11, 1 June 2000 (2000-06-01), pages 2805-2809, XP002210584 ISSN: 0008-5472
- CHUNYAO XIA ET AL.: "Induction of apoptosis in mesothelioma cells by antisurvivin oligonucleotides" MOLECULAR CANCER THERAPEUTICS, vol. 1, July 2002 (2002-07), pages 687-694, XP002308516
- GROSSMAN D. ET AL.: "Expression and targeting of the inhibitor, Survivin, in human melanoma" JOURNAL OF INVESTIGATIVE DERMATOLOGY, NEW YORK, NY, US, vol. 113, no. 6, December 1999 (1999-12), XP001041399 ISSN: 0022-202X
- KURRECK J. ET AL.: "Design of antisense oligonucleotides stabilized by locked nucleic acids" NUCLEIC ACIDS RESEARCH, vol. 30, no. 9, 1 May 2002 (2002-05-01), pages 1911-1918, XP002281375 ISSN: 0305-1048
- KOJI MORITA ET AL.: "2'-O,4'-C-ethylene-bridged nucleic acid (ENA): highly nuclease resistant and thermodinamically stable oligonucleotides for antisense drug" BIOORGANIC AND MEDICINAL CHEMISTRY LETTERS, vol. 12, 2002, pages 73-76, XP002290399
- ALTMANN K.-H. ET AL.: "NOVEL CHEMISTRY" 1998, APPLIED ANTISENSE OLIGONUCLEOTIDE TECHNOLOGY; STEIN C.A. AND KRIEG A.M. EDS. WILEY-LISS INC., US , XP002119324 ISBN: 0-471-17279-0 page 73 - page 107
- RAIT A.S. ET AL.: "Inhibitory effects of the combination of HER-2 antisense oligonucleotide and chemotherapeutic agents used for the treatment of human breast cancer" CANCER GENE THERAPY, vol. 8, no. 10, 2001, pages 728-739, XP008005112 ISSN: 0929-1903
- MIRIAM FRIEDEN ET AL: 'Expanding the design horizon of anitsense oligonucleotides with alpha-l-LNA' NUCLEIC ACIDS RESEARCH, 2003 vol. 31, no. 21, SANTARIS PHARMA A/S DK, pages 6365 - 6372
- HANSEN ET AL.: 'SPC3042: a proapoptotic survivin inhibitor' MOL CANCER THER vol. 7, no. 9, 2008, 2008 AMERICAN ASSOCIATION FOR CANCER RESEARCH, pages 2736 - 2745

## Description

### FIELD OF THE INVENTION

The present invention provides compositions and methods for modulating the expression of survivin. In particular, this invention relates to oligomeric compounds and preferred such compounds are oligonucleotides, which are specifically hybridisable with nucleic acids encoding survivin. The oligonucleotide compounds have been shown to modulate the expression of survivin and pharmaceutical preparations thereof and their use as treatment of cancer diseases are disclosed.

### BACKGROUND OF THE INVENTION

Cancer, a leading cause of death worldwide, comprises a group of diseases, which are caused by genetic disorders resulting from genomic instability. It has been postulated that all cancer cells harbour defects in several regulatory pathways, which govern normal cell proliferation and homeostasis. Theses defects result in acquirement of various cancer cell specific hallmark capabilities (Hanahan and Weinberg, 2000, Cell 100, 57-70). One of these hallmarks of cancer is evasion of apoptosis or programmed cell death, an evolutionary conserved program of cellular suicide (Hengartner, 2000, Nature 407, 770-776.). Apoptosis is essential in fetal development by removal of cells not needed any longer, and maintenance of homeostasis of adult tissues by balancing cell production and cell elimination. Additionally, cells exhibiting aberrant features like mutations or genomic damages induced by infectious agents or drugs are removed in this way. In malignant cells this cellular surveillance is missing due to inhibition of apoptosis, which results in extended cell viability increasing the risk of cellular transformation, accelerated disease progression and resistance to therapy (Evan and Vousden, 2001, Nature 411, 342-348. Therefore, manipulation of apoptosis has emerged as a new therapeutic strategy for treatment of cancer (Nicholson DW, 2000, Nature 407, 810-816).

Two signaling pathways leading to induction of apoptosis are known, the intrinsic or mitochodrial pathway, induced by environmental stress and chemotherapeutics, and the extrinsic or death receptor pathway, induced by effector cells of the immune system (Ashkenazi and Dixit, 1998 Science 281, 1305-1308; Green and Reed, 1998, Science 281, 1309-1312). Both pathways culminate with the activation of caspases, a family of intracellular cystein proteases, which within minutes dismantle the cell's structures leading to rapid cell death (Cohen, 1997, Biochem J 326, 1-16). Both, apoptosis promoting as well as inhibiting proteins are known. The Bcl-2 protein family comprises both, pro- and anti-apoptotic proteins. Among the inhibitors of apoptosis, the evolutionary highly conserved inhibitor of apoptosis protein (IAP) familly comprises eight proteins in humans. One of them, survivin, has only regency been identified (Ambrosini et al., 1997, Nat. Med. 3, 917-921). Survivin. Inhibits apoptosis downstream of Bcl-2 by directly or indirectly inhibiting the effector caspase -3 and -7 intracellular proteases responsible or apoptosis (Shin et al., 2001, Biochemistry 40, 1117-1123) Recent evidence suggests that survivin directly controls the activation of the upstream acting caspase 9. A survivin Thr³⁴-Ala dominant negative mutant fails to induce apoptosis in mouse embryonic fibroblasts deficient in apoptosome components Apaf-1 or caspase 9 (Blanc-Brude et al., 2003, Clin. Cancer Res. 9, 2683-2692) The hepatitis B X-interacting protein (HBXIP) operates as a cofactor for phosphorylated survivin allowing It to bind and suppress activation of pro-taspase 9 (Marusawa et al., 2003, EMBO J. 22, 2729-2740). Other modes of action are discussed, too (Beltrami et al., 2004, J. Biol. Chem. 279, 2077-2084).

Survivin has attracted great invention as novel therapeutic target, because it is selectively expressed in cancer cells and it is required for their viability. Survivin is normally expressed during embryogenesis. Apart from the thyme, CD34+ bone-marrow-derived stem cells, placenta and the basal colonic epithelium, survivin is not detectable in normal adult tissues, but is basically overexpressed in all transformed cells by their independently mitotic status. Expression is generally regulated in a cell-cyde dependent manner peaking at mitosis (U et al. 1998, Nature 396, 580-584). Upregulation In G2/M phase compared to interphase can be more than 40-fold. Also, increased protein stability due to phosphorylation of Thr 34 by CDC2-cyclin-B1 may account for elevated survivin levels ,at mitosis. In the interphase, the protein level declines due to ubiquitin dependent proteolysis (Zhao et al., 2000, J Cell Sci. 113, 4363-71) to basal levels. It has been suggested that overexpression of survivin in cancer cells counteracts a default induction of apoptosis, overcomes the G2/M checkpoint and thus enforces progression of cells through mitosis (Li et al., 1998, Nature 396, 580-584).

In cell culture systems, inhibition of cell death by overexpression of survivin is well established (Ambrosini et al. 1997, Nat. Med. 3, 917-921; Tamm et al. 1998, Cancer Res. 58, 5315-5320; Mahotka et al., 1999, Cancer Res. 59, 6097-6102). *In vivo,* survivin's role as inhibitor of apoptosis has been demonstrated In transgenic mice expressing survivin in the skin, which inhibited UVB induced apoptosis of the keratinocytes (Grossman at al., 2001, J. Clin. Invest. 108,991-999). Apart from its role in cellular apoptosis, survivin plays a critical role in various aspects of mitosis. For example, knocking out Survivin in homozygous survivin knock-out mice leads to 100% lethality (Uren et al. 2000, Curr. Biol. 10, 1319-1328; Conway et al., 2002, Gastroenteroigy 123, 619-631). Survivin has been found to be associated with various components of the mitotic apparatus, such as centrosomes, mictrotubules and the remnants of the spindle apparatus - the midbodies. Microtubule association is essential for survivin's anti-apoptotic action.

Survivin's dual role as apoptosis inhibitor and essential factor in cell division was demonstrated by targeted downregulation of survivin by transfecting Hela cells with EPR-1 cDNA, which is complementary to survivin. Downregualtion of survivin by Err-1 antisense resulted in increased apoptosis and inhibition of cell proliferation (Ambrosini et al., 1998, J. Biol. Chem. 273, 11177-11182). Other hallmarks of survivin ablation are mitotic arrest, polyploidy, defect centrosome replication, microtubule nucleation and mitotic spindle assembly/stability and inhibition of cell division. Most of these effects are exacerbated In a p53^{-/-}, mutant background (Beltrami et al., 2004, J. Biol. Chem. 279, 2077-2084; Carvalho et al, 2003, J. Cell. Sci. 116, 2987-2998; Lens et al., 2003, EMBO J. 22, 2934-2947). The pivotal role of survivin in mitosis is underscored by its association with the mitotic apparatus, including microtubules of the metaphase and anaphase spindle, and kinetochores of metaphase chromosomes (Beltrami et al., 2004, J. Biol. Chem. 279, 2077-2084). Survivin colocalizes with other chromosomal passenger proteins such as INCENP and Aurora B (Carvalho et al, 2003, J. Cell. Sd. 116, 2987-2998; Lens et al., 2003, EMBO J. 22, 2934-2947). Kinase activity of Aurora B is dependent upon interaction with surviving (Chen et al., 2003, J. Biol. Chem. 278, 486490). It has been suggested that Aurora B kinase activity is essential to cytokinesis providing a mechanistic link between survivin and cell division (Chen et al., 2003, J. Biol. Chem. 278, 486-490). Two reports demonstrate that survivin is required for sustained Δ-checkpoint arrest in response to lack of tension on kinetochores of sister chromatides. Survivin appears to be essential for the maintenance of checkpoint protein BubR1 at the kinetochores and mitotic arrest under protracted conditions of lack of tension at the kinetochores. Taxol, a microtubule stabilizing agent, allows attachment of microtubules to kinetochores, but prevents generation of tension. Normally taxol treated cells arrest in mitosis due to checkpoint activation mediated by kinetochore associated BubR1. Taxol treated Survivin depleted cells however manage to complete mitosis after a delay during which BubR1 is lost from the kinetochore. Nocodazol also prevents tension forming at the kinetochores but by preventing microtubule attachment. However depletion of Survivin has no effect on checkpoint activation and mitotic arrest induced by Nocodazol. Survivin is apparently not involved in monitoring kinetochore occupancy by microtubuli but instead required for checkpoint monitoring spindle tension excerted on the kinetochore. (Carvalho et al, 2003, J. Cell. Sd. 116, 2987-2998; Lens et al., 2003, EMBO J. 22, 2934-2947). Moreover it has been suggested that survivin serves as a crucial p53 dependent mitotic checkpoint protein required for genomic integrity and cytoprotection (Beltrami et al., 2004, J. Biol. Chem. 279, 2077-2084). Survivin may therefore be an important link between cell death and the regulation of cell division. Due to its dual role as inhibitor of apoptosis and promoter of mitosis survivin is an important factor in onset and progression of cancer as well as resistance to chemotherapeutic agents.

Its clinical role in cancer has been emphasized by detection of high levels of survivin in almost all tumour types. Elevated expression of survivin in tumours is associated with poor prognosis, increased cancer recurrence and resistance to therapy (Kawasaki et al., 1998, Cancer Res. 58, 5071-5074; Adida et al., 1998, Lancet 351, 882-883). Interestingly, lung and breast tumours express the highest levels of survivin. These tumours are generally associated with unfavourable prognosis due to early metastasizing and development of resistance to a number of mechanistically unrelated chemotherapeutic agents. Downregulation of survivin has been shown to sensitize tumor cells to DNA damaging agents such as etoposide (Li et al., 1999, Nature Cell Biology 1, 461-466; Olie et al., 2000, Cancer Res. 60, 2805-2809; Jiang et al., 2001, J. Cell. Biochem. 83, 342-354), cisplatin (Pennati et al., 2004, Oncogene 23, 386-394; ), doxorubicin (Zhou et al., 2002, J. Pharmacol. Exp. Ther. 303, 124-131) and radiotherapy (Pennati et al., 2003, J. Invest. Dermatol. 120, 648-654; Asanuma et al., 2002, Jpn. J. Cancer Res. 93, 1057-1062). Survivin depleted cells are particularly senetive to texol is also true for taxol (Zaffaroni et al., 2002, Cell. Mol. Life Sci. 59, 1406-1412; Ling et al., 2004, J. Biol. Chem. Epub ahead of print). Resistance to taxol and radiotherapy has been shown to correlate with the expression level of survivin (Zaffaroni et al., 2002, Cell. Mol. Life Sci. 59, 1406-1412; Rodel et al., 2003, Int. J. Radiat. Oncol. Biol. Phys. 55, 1341-1347) and sublethal concentrations of taxol has been shown to upregulate survivin expression significantly in MCF-7 breast cancer cells (Ling et al., 2004, J. Biol. Chem. Epub ahead of print). Survivin appears to be required for the function of the spindle checkpoint in response to taxol treatment (Carvalho et al, 2003, J. Cell. Sci. 116, 2987-2998; Lens et al., 2003, EMBO J. 22, 2934-2947). In the absence of survivin cells are therefore deprived of one of their natural resistance mechanisms that allows repair of the adverse effects of taxol on mitosis.

Interestingly, survivin also plays a critical role in angiogenesis. Survivin was found upregulated in angiogenically stimulated endothelium in vitro and in vivo (O'Connor et al., 2000, Am. J. Pathol. 156, 393-398; Tran et al., 1999, Biochem. Biophys. Res. Commun. 264, 781-788). Antisense trageting of survivin caused endothelial apoptosis and rapid involution of capillary-like vessels in vitro (Mesri et al., 2001a, Am. J. Pathol. 158, 1757-1765). Injection into human breast cancer xenografts of an adenovirus expressing a dominant negative version of survivin inhibited growth of established tumors. This was associated with apoptosis of both tumor cells and endothelial cells and a significant reduction in tumor derived blood vessels (Blanc-Brude et al., 2003, Clin. Cancer Res. 9; 2683-2692). Chemotherapy and radiotherapy targets both tumor cells and the proliferating endothelial cells of the tumor vasculature. Vascular endothelial growth factor (VEGF) has been shown to significantly reduce the proapoptotic potency of chemotherapy on vascular endothelial cells. This cytoprotection to drug toxicity has been linked to a VEGF mediated upregulation of survivin expression. Supression of survivin activity abrogates the cytoprotective effect of VEGF to drugs that interfere with microtubule dynamics (Taxol) and damage DNA as well as protection against tumor necrosis facor α (Tran et al., 2002, Proc. Natl. Acad. Sci. USA 99, 4349-4354; Mesri et al., 2001a, Am. J. Pathol. 158, 1757-1765).In addition expression of a dominant negative survivin (T34A) protein in endothelial cells (HUVECC and DMVEC) resulted in massive induction of apoptosis (Blanc-Brude et al., 2003, Clin. Cancer Res. 9, 2683-2692) .

Targeting survivin is increasingly being mentioned as having a dual anticancer activity by inducing tumor cell apoptosis and suppression of tumor associated angiogenesis (Altieri DC, 2003, Oncogene 22, 8581-8591).

Several therapeutic approaches using survivin as target have been initiated. The most promising ones comprise vaccination strategies, use of mutant survivin as dominant-negative antagonists, and application of survivin specific antisense oligonucleotides. Application of a replication deficient adenovirus expressing a dominant negative survivin mutant protein (Thr34 - Ala) caused inhibited tumour growth in three distinct breast cancer xenograft models in mice. This adenovirus has shown in vivo efficacy in a breast cancer xenograft models and induced expression of survivin (T34A) in melanoma cells inhibited tumor growth in a melanoma xenograft model (Blanc-Brude et al., 2003, Clin. Cancer Res. 9, 2683-2692; Grossman et al., 2001 Proc. Natl. Sci. USA 98; 635-640). In cell cultures apoptosis was increased by binding of mutant survivin to CDC2-cyclin-B1 and thus preventing phosphorylation of wildtype survivin (Mesri et 61., 2001b, J. Clin. Invest 108, 981-990). Some CDC2 antagonists like purvalanol A and flavopiridol, preventing survivin phospholylation, are currently being tested in clinical trials in combination with taxol (Schwartz et al., 2002, J. Clin. Oncol. 20, 2157-2170).

Several approaches using antisense oligonucleotides have shown that anti-survivin antisense oligonucleotides downregulate survivin in cell cultures, induce apoptosis and sensitize lung cancer cells and HeLa cells to the chemotherapeutic agent etoposide (Li et al., 1999, Nature Cell Biology 1, 461-466; Olie et al., 2000, Cancer Res. 60, 2805-2809; Jiang et al., 2001, J. Cell. Biochem. 83, 342-354). Inhibition of several cell lines with antisense oligo ISIS 28599, a mixed backbone 2'-O- MOE wingmer, resulted in multinucleated cells and induction of apoptosis (Chen at al., 2000, Neoplasia 2, 235-241). In a mouse liver regeneration model survivin mRNA was reduced 90% by the antisense oligonucleotide ISIS 114926 (Proceedings of the American Association for Cancer Research, vol. 42, 2001, abstract #2468). Intratumoral injection of antisense oligonucleotide ISIS 23722 reduced had limited effect on the growth rate of aggressive non-Hodgkin's lymphoma xengraft tumors in mice (Ansell et al., 2004, Leukemia - Epub ahead of print).

There are currently no therapeutic agents, which effectively inihibit the synthesis of survivin. Therefore, there is a longfelt need for agents inhibiting tumor cell growth by reducing survivin expression. In WO9822589 methods of modulating apoptosis with agents, that modulates the amount or activity of survivin and methods for reducing the severity of a pathological state mediated by survivin with such agents are disclosed. Such an agent is a construct encoding the EPR-1 coding strand, which is complementary to survivin but no specific antisense oligos are disclosed. W00164741 discloses a "tet-off" promoter system regulating a survivin antisense mRNA transcript. However, this application does not disclose any antisense oligonucleotides.

Most of the oligonucleotides currently in clinical trials are based on the phosphorothioate chemistry from 1988, which was the first useful antisense chemistry to be developed. However, as it has become clear in recent years this chemistry has serious shortcomings that limit its clinical use. These include low affinity for their target mRNA, which negatively affects potency and puts restrictions on how small active oligonucleotides can be thus complicating manufacture and increasing treatment costs. Also, their low affinity translate into poor accessibility to the target mRNA thus complicating identification of active compounds. Finally, phosphorothioate oligonucleotides suffer from a range of side effects that narrow their therapeutic window.

To deal with these and other problems, much effort has been invested in creating novel analogues with improved properties. As depicted in the scheme 1 below, these include wholly artificial analogues such as PNA and Morpholino and more conventional DNA analogues such as boranosphosphates, N3'-P5'phosphoroamidates and several 2' modified analogues, such as 2'-F, 2'-O-Me, 2'-O-methoxyethyl (MOE) and 2'-O- (3-aminopropyl)(AP). More recently hexitol nucleic acid (HNA), 2'-F-arabino nucleic acid (2'-F-ANA) and D-cyclohexenyl nucleoside (CeNA) have been introduced.

Many of these analogues exhibit improved binding to complementary nucleic acids, improvements in bio-stability or they retain the ability to recruit a cellular enzyme, RNAseH, which is involved in the mode-of-action of many antisense compounds. None of them, however, combine all of these advantages and in many cases improvements in one of the properties compromise one or more of the other properties. Also, in many cases new complications have been noted which seriously limits the commercial value of some of the analogues. These include low solubility, complex oligomerisation chemistries, very low cellular up-take, incompatibility with other chemistries, etc.

Antisense oligonucleotides for modulation of survivin expression for treatment of diseases are disclosed in W00018781 and W00157059. These oligonucleotides are all between 18-20 bp in length and designed with the phosphorothioate or the MOE chemistry.

W0014655 discloses one single antisense oligonucleotide targeting Survivin and it is a fully modified phosphorthioate with some MOE nucleosides. The MOE chemistry has several limitations. It has only modest affinity, which only manifests when several MOE's are inserted *en block* into the oligo. MOE belongs to the family of 2'-modifications and it is well known, for this group of compound, that the antisense activity is directly correlated with RNA binding affinity *in vitro.* A MOE 20 bp gapmer (5MOE/PO-10PS-5MOE/PO) targeting c-raf has been reported to have an IC₅₀ of about 20 nm in T24 cells and an MOE gapmer targeting PKC-a has been reported to have an IC₅₀ of 25 nm in A549 cells. In comparison, phosphorthioate compounds used in antisense experiments typically exhibit IC₅₀ in the 150 nm range. (Stein, Kreig, Applied Antisense Oligonucleotide Technology, Wiley-Liss, 1988, p 87-90)

WO03027244, filed subsequent to the present invention, discloses a 20-mer phosphorthioate antisense oligonucleotides targeting survivin which show down regulation at very high concentrations (for example compound 903 showed 51% protein reduction at 200 nM).

US 2002/137708 discloses antisense oligonucleotides targeting Survivin. Among the oligonucleotides disclosed are phosphorothioate oligodeoxynucleotides and chimeric phosphorothioate oligonucleotides having 2'MOE wings and a deoxy gap, as well as conjugates comprising the oligonucleotides and their therapeutic application against cancer.

It is a principal object of the present invention to provide novel oligomeric compounds, against the survivin mRNA. The compounds of the invention have been found to exhibit an decreased IC₅₀ (thus increased activity), thereby facilitating an effective treatment of a variety of cancer diseases in which the expression of survivin is implied as a causative or related agent. As explained in the following, this objective is best achieved through the utilisation of a super high affinity chemistry termed LNA (Locked Nucleic Acid).

The present invention is directed to oligomeric compounds, particularly LNA antisense oligonucleotides, which are targeted to a nucleic acid encoding survivin and which modulate the expression of the survivin. This modulation was particularly a very potent down regulation survivin mRNA as well as elicitation of apoptotic response. The ULNA-containing oligomeric compounds are highly active as a 16-mers, which is considerably shorter than the reported antisense compounds targeting survivin. These 16-mer oligomeric compounds, have an IC₅₀ in the sub-nanomofar range. The invention enables a considerable shortening of the usual length of an antisense oligomers (from 20-25 mers to 16 mers) without compromising the affinity required for pharmacological activity. As the *intrisic specificity* of an oligo is inversely correlated to its length, such a shortening Will significantly increase the specificity of the antisense compound towards its RNA target. Furthermore, it is anticipated that shorter oligomeric compounds have as higher blostability and cell permeability than longer oligomeric compounds. For at least these reasons, the present invention is a considerable contribution to the art.

### SUMMARY OF THE INVENTION

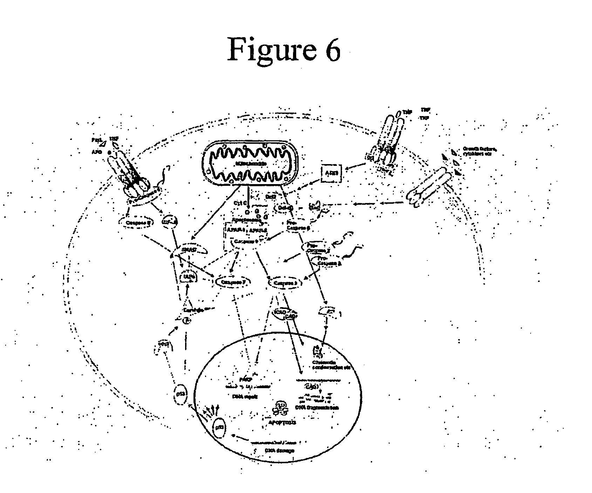

Survivin is essential to cell proliferation and Involved in multiple phases of mitosis. It is involved in several checkpoints linking mitosis with cell division and apoptosis. Survivin is a member of the inhibitor pf apoptosis (IAP) gene family that suppresses programmed cell death (apoptosis) (see figure 6). Increased survivin expression is observed in most common human neoplasms, including colorectal cancer, bladder cancer; lung carcinoma, breast cancer, malignant gloma and haematological cancers. Expression of survivin correlates with advanced grade and invasiveness in several cancers. Survivin is undetectable or present at very low levels in normal differentiated tissues, making survivin a preferred target in several human cancers. A central aspect of the invention to provide a compound capable of modulating the expression of survivin wherein said compound is selected from the group consisting of
CₛTₛCₛAₛaₛtₛcₛcₛaₛtₛgₛgₛCₛAₛGₛC (SEQ ID NO: 130A),
CₛTₛCₛAₛaₛtₛcₛcₛaₛtₛgₛgₛCₛAₛGₛc (SEQ ID NO: 130B), and
C_{O}T_{O}C_{O}A_{O}aₛtₛcₛcₛaₛtₛgₛgₛC_{O}A_{O}G_{O}C (SEQ ID NO: 130C);
wherein
capital letters indicate beta-D-oxy-LNA,
small letters indicate DNA,
subscript O indicates a phosphate linkage,
subscript s indicates a phosphorothioate linkage, and
wherein each C is 5'-methyl-cytosine.

One embodiments of the invention is, since the sequence of the humane genome is available and the annotation of its genes rapidly progressing, to identify the shortest possible, unique sequences In the target mRNA. LNA containing oligomeric compounds have also been compared to a number of 18-mers containing LNA and/or phosphorthioates which are iso-sequential to the antisense oligomer the ISIS 23722. A comparison to the ISIS 23722 (being a 18-mer 4 MOE 10 phosphorthioate followed by 4 MOE) has also been performed.

Pharmaceutical and other compositions comprising the oligomeric compounds of the invention are also provided. Further provided are methods of modulating the expression of survivin in cells or tissues comprising contacting said cells or tissues with one or more of the oligomeric compounds or compositions of the invention. Also disclosed are methods of treating an animal or a human, suspected of having or being prone to a disease or condition, associated with expression of survivin by administering a therapeutically or prophylactically effective amount of one or more of the oligomeric compounds or compositions of the invention. Further, methods of using oligomeric compounds for the inhibition of expression of survivin and for treatment of diseases associated with survivin activity are provided. Examples of such diseases are different types of cancer, such as for instance lung, breast, colon, prostate, pancreas, lung, liver, thyroid, kidney, brain, testes, stomach, intestine, bowel, spinal cord, sinuses, bladder, urinary tract or ovaries.

### BRIEF DESCRIPTION OF THE FIGURES

Figure 1. Illustration of the different designs of the invention: Gapmers, Head- and Tailmers and Mixmers of different composition. For the mixmer, the numbers designate the alternate contiguous stretch of DNA, β-D-oxy-LNA or α-L-LNA. In the drawing, the line is DNA, the gray shadow corresponds to α-L-LNA residues and the rectangle is β-D-oxy-LNA.
Figure 2 Survivin mRNA downregulation by LNA antisense oligomeric compound. Northern blot of total RNA from 15PC3 that have been treated with 0.2, 1, 5, 25 nM compound 2A, 6A, 9A, 15A respectively. All compounds were effective inhibitors at low concentrations.
Figure 3A Survivin mRNA downregulation by LNA antisense oligomeric compound. Northern Blot of total RNA from SW480 (upper panel) and A549 (lower panel) that have been treated with 0.2, 1, 5, 25 nM compound 2A and 15A respectively. Cells were transfected with oligo nucleotide and cultured for 24 h.
Figure 4 General scheme of the synthesis of thio-LNA.

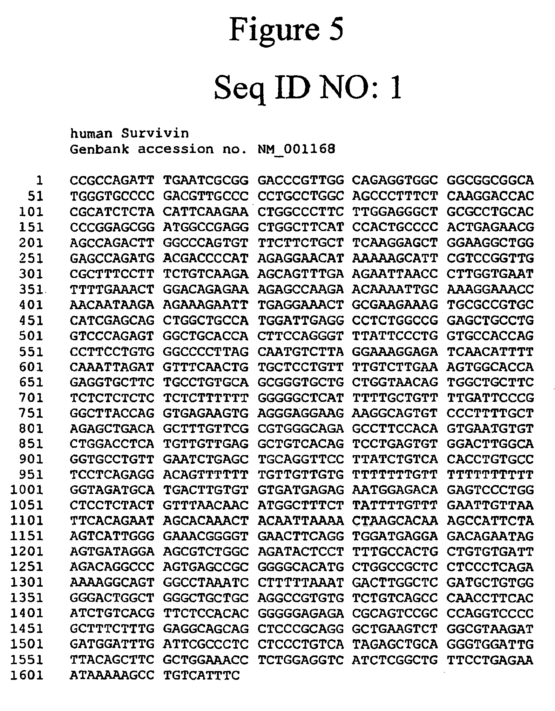

Figure 5 SEQ ID No 1 GenBank accession number NM_001168 human survivin mRNA sequence.
Figure 6 Schematic way of Survivin in the apoptotic pathway.
Figure 7 Survivin mRNA downregulation by LNA antisense oligonucleotides. Cells were transfected with oligonucleotide and culutured for 24 h. Total RNA was extracted and expression of Survivin mRNA were detected by either Realtime PCR in 15PC3 and MCF-7. Survivin expression id presented normalised to GAPDH transcript stady state.
Figure 8 Induction of apoptosis by LNA containing antisense oligo nucleotides. 15PC3 cells transfected with the oligos and concentrations Indicated in 96 well. 24h following transfection Caspase 3/7-Glo reagens were added as described and the induction of luminescence (luciferase activity) were recorded in a Luminoskan Ascent Instrument from Thermo Labsystems. The luciferase activity is measured as Relative Light Units per seconds (RLU/s).

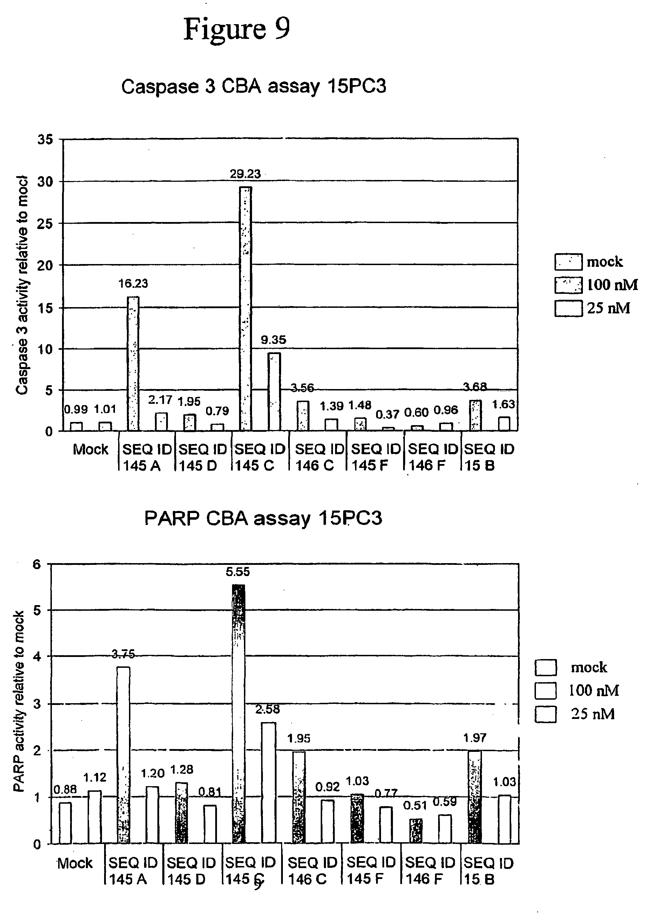

Figure 9 shows that the LNA containing compounds (145A and 145C) improve induction of apoptosis compared to the iso-sequential MOE compound ISIS27322 (here 145F) and the iso-sequential phosphorthioate compound (145D). Mismatch controls of a LNA compound (146C) and the MOE compound (146F) as well as the LNA compound 158 were also included in the study. The targeted downregulation of Survivin mRNA using LNA antisense oligomeric compound results in increased apoptosis in 15PC3 cells. Activation of apoptosis is measured by cytometric bead array. Fold induction is presented relative to mock treated cells.
Figure 10 Using immunohistochemistry detection, active Caspase 3 was detected in 15PC3 cells treated with LNA antisense oligonucleotides 15A targeting Survivin.
Figure 11 LNA antisense inhibition of Survivin in proliferating cancer cells. For example, compound 6A is particularly potent.

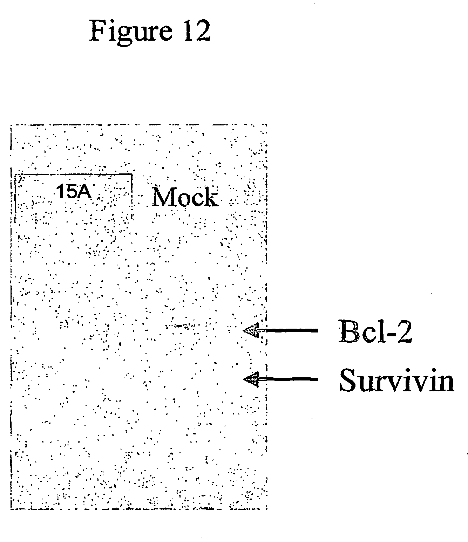

Figure 12 Down regulation of Survivin in 15PC3 cells transfected with compound 15A analyzed by western blotting at 100nM.

### DESCRIPTION OF THE INVENTION

The present invention employs oligomeric compounds, particularly antisense oligonucleotides, for use in modulating the function of nucleic add molecules encoding survivin. The modulation is ultimately a change in the amount of survivin produced. In one embodiment this is accomplished by providing antisense compounds, which specifically hybridise with nucleic adds encoding survivin. The modulation is preferably an inhibition of the expression of survivin, which leads to a decrease in the number of functional proteins produced.

Antisense and other oligomeric compounds of the invention, which modulate expression of the target, are identified through experimentation or though rational design based on sequence information on the target and know-how on how best to design an oligomeric compound against a desired target. The sequences of these compounds are preferred embodiments of the invention. Likewise, the sequence motifs in the target to which these preferred oligomeric compounds are complementary (referred to as "hot spots") are preferred sites for targeting.

The invention is directed to a compound capable of modulating the expression of survivin wherein said compound is selected from the group consisting of
CₛTₛCₛAₛaₛtₛcₛcₛaₛtₛgₛgₛCₛAₛGₛC (SEQ ID NO: 130A),
CₛTₛCₛAₛaₛtₛcₛcₛaₛtₛgₛgₛCₛAₛGₛc (SEQ ID NO: 130B), and
C_{O}T_{O}C_{O}A_{O}aₛtₛcₛcₛaₛtₛgₛgₛC_{O}A_{O}G_{O}C (SEQ ID NO: 130C);
wherein
capital letters indicate beta-D-oxy-LNA,
small letters indicate DNA,
subscript O indicates a phosphate linkage,
subscript s indicates a phosphorothioate linkage, and
wherein each C is 5'-methyl-cytosine.

In the present context, the term "nucleoside" is used in its normal meaning, i.e. it contains a 2-deoxyribose unit or a ribose unit which is bonded through its number one carbon atom to one of the nitrogenous bases adenine (A), cytosine (C), thymine (T), uracil (U) or guanine (G).

In a similar way, the term "nucleotide" means a 2-deoxyribose unit or RNA unit which is bonded through its number one carbon atom to one of the nitrogenous bases adenine (A), cytosine (C), thymine (T) or guanine (G), uradl (U) and which is bonded through its number five carbon atom to an internucleoside phosphate group, or to a terminal group.

When used herein, the term "nucleotide analogue" refers to a non-naturaly occurring nucleotide wherein either the ribose unit is different from 2-deoxyribose or RNA and/or the nitrogenous base is different from A, C, T and G and/or the Internucleoside phosphate linkage group is different. Specific examples of nucleoside analogues are described by e.g. Freier & Altmann; Nucl. Acid Res., 1997, 25, 4429-4443 and Uhlmann; Curr. Opinion In Drug Development, 2000, 3(2), 293-213.

The terms "corresponding nucleoside analogue" and "corresponding nucleoside" are intended to indicate that the nucleobase in the nucleoside analogue and the nucleoside is identical. For example, when the 2-deoxyribose unit of the nucleotide is linked to an adenine, the "corresponding nucleoside analogue" contains a pentose unit (different from 2-deoxyribose) linked to an adenine.

The term "nucleic acid" is defined as a molecule formed by covalent linkage of two or more nucleotides. The terms "nucleic acid" and "polynucleotide" are used interchangeable herein

The term "nucleic acid analogue" refers to a non-natural nucleic acid binding compound.

Nucleotide analogues and nucleic acid analogues are described in e.g. Freier & Altmann (Nucl. Acid Res., 1997, 25, 4429-4443) and Uhlmann (Curr. Opinion in Drug & Development (2000, 3(2): 293-213). Scheme 1 illustrates selected examples of nucleotide analogues suitable for making nucleic acids.

The term "LNA" refers to a nucleotide containing one bicyclic nucleoside analogue, also referred to as a LNA monomer, or an oligonucleotide containing one or more bicyclic nucleoside analogues. LNA monomers are described in WO 9914226 and subsequent applications, WO0056746, W00056748, W00066604, WO00125248, W00228875, WO2002094250 and WO03/006475. One particular example of a thymidine LNA monomer is the (1S, 3R, 4R 7S)-7-hydroxy-1-hydroxymethyl-5-methyl-3-(thymin-1-yl)-2,5-dioxa-bicyclo[2:2:1]heptane.

The term "oligonucleotide" refers, in the context of the present invention, to an oligomer (also called oligo) or nucleic acid polymer (e.g. ribonucleic acid (RNA) or deoxyribonucleic acid (DNA)) or nucleic acid analogue of those known in the art, preferably Locked Nucleic Acid (LNA), or a mixture thereof. This term includes oligonucleotides composed of naturally occurring nucleobases, sugars and internucleoside (backbone) linkages as well as oligonucleotides having non-naturally-occurring portions which function similarly or with specific improved functions. A fully or partly modified or substituted oligonucleotides are often preferred over native forms because of several desirable properties of such oligonucleotides such as for instance, the ability to penetrate a cell membrane, good resistance to extra- and intracellular nucleases, high affinity and specificity for the nucleic acid target. The LNA analogue is particularly preferred exhibiting the above-mentioned properties.

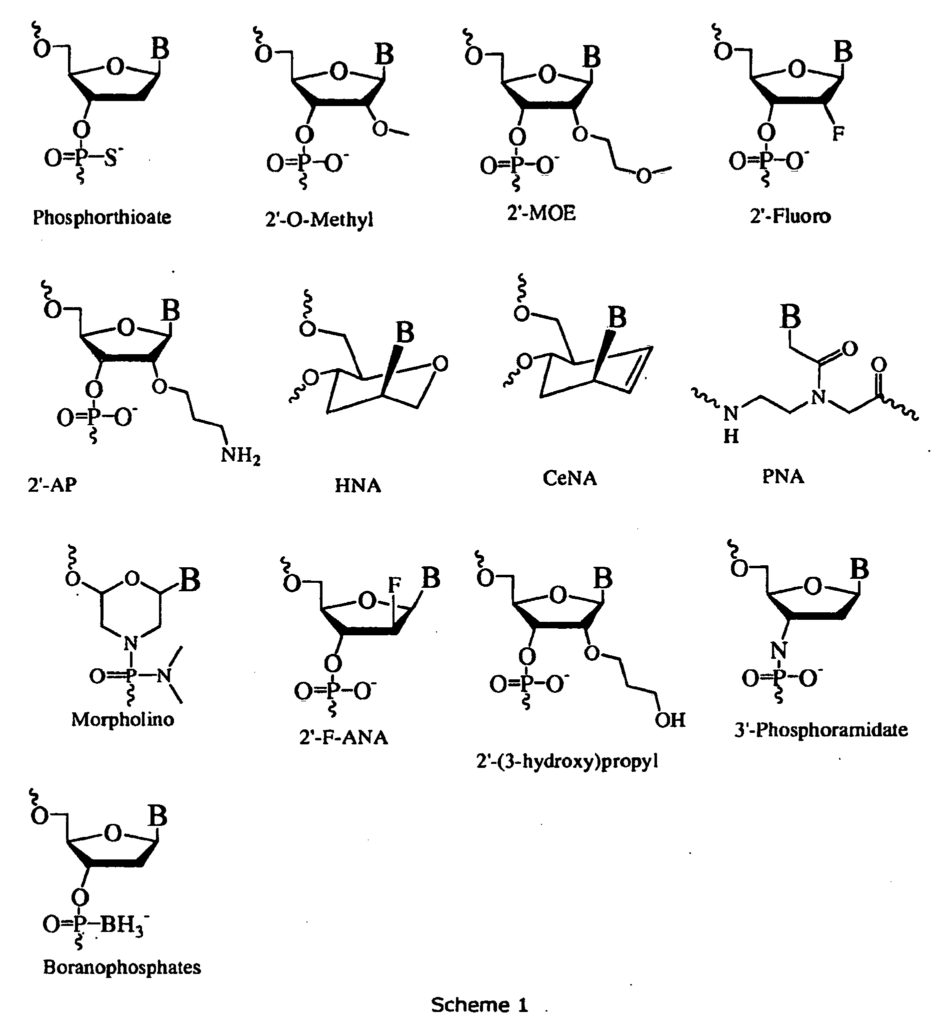

By the term "unit" is understood a monomer.

The term "at least one" comprises the integers larger than or equal to 1, such as 1, 2, 3, 4, 5, 6, 7, 8, 9, 10, 11, 12, 13, 14, 15, 16, 17 and so forth.

The term "thio-LNA" comprises a locked nucleotide in which at least one of X or Y in Scheme 2 is selected from S or -CH₂-S-. Thio-LNA can be in both beta-D and alpha-L-configuration.

The term "amino-LNA" comprises a locked nucleotide in which at least one of X or Y in Scheme 2 -N(H)-, N(R)-, CH₂-N(H)-, -CH₂-N(R)- where R is selected form hydrogen and C₁₋₄-alkyl. Amino-LNA can be in both beta-D and alpha-L-configuration.

The term "oxy-LNA" comprises a locked nucleotide in which at least one of X or Y in Scheme 2 represents -O- or -CH₂-O-. Oxy-LNA can be in both beta-D and alpha-L-configuration.

The term "ena-LNA" comprises a locked nucleotide in which Y in Scheme 2 is -CH₂-O-.

By the term "alpha-L-LNA" comprises a locked nucleotide represented as shown in Scheme 3 (structure to the right).

By the term "LNA derivatives" comprises all locked nucleotide in Scheme 2 except beta-D-methylene LNA e.g. thio-LNA, amino-LNA, alpha-L-oxy-LNA and ena-LNA.

The term "linkage group" is intended to mean a group capable of covalently coupling together two nucleosides, two nucleoside analogues, a nucleoside and a nucleoside analogue, etc. Specific and preferred examples include phosphate groups and phosphorothioate groups.

In the present context the term "conjugate" is intended to indicate a heterogenous molecule formed by the covalent attachment of a compound as described herein (i.e. a compound comprising a sequence of nucleosides or nucleoside analogues) to one or more non-nucleotide or non-polynucleotide moieties. Examples of non-nucleotide or non-polynucleotide moieties include macromolecular agents such as proteins, fatty acid chains, sugar residues, glycoproteins, polymers, or combinations thereof. Typically proteins may be antibodies for a target protein. Typical polymers may be polyethelene glycol.

The term "carcinoma" is intended to indicate a malignant tumor of epithelial origin. Epithelial tissue covers or lines the body surfaces inside and outside the body. Examples of epithelial tissue are the skin and the mucosa and serosa that line the body cavities and internal organs, such as intestines, urinary bladder, uterus, etc. Epithelial tissue may also extend into deeper tissue layers to from glands, such as mucus-secreting glands.

The term "sarcoma" is intended to indicate a malignant tumor growing from connective tissue, such as cartilage, fat, muscles, tendons and bones.

The term "glioma", when used herein, is intended to cover a malignant tumor originating from glial cells

The term "a" as used about a nucleoside, a nucleoside analogue, a SEQ ID NO, etc. is intended to mean one or more. In particular, the expression "a component (such as a nucleoside, a nucleoside analogue, a SEQ ID NO or the like) selected from the group consisting of ..." is intended to mean that one or more of the cited components may be selected. Thus, expressions like "a component selected from the group consisting of A, B and C" is intended to include all combinations of A, B and C, i.e. A, B, C, A+B, A+C, B+C and A+B+C.

In the present context, the term "C₁₋₄-alkyl" is intended to mean a linear or branched saturated hydrocarbon chain wherein the chain has from one to four carbon atoms, such as methyl, ethyl, n-propyl, isopropyl, n-butyl, isobutyl, sec-butyl and tert-butyl.

As used herein, the terms "target nucleic acid" encompass DNA encoding the survivin, RNA (including pre-mRNA and mRNA) transcribed from such DNA, and also cDNA derived from such RNA.

As used herein, the term "gene" means the gene including exons, introns, non-coding 5'and 3' regions and regulatory elements and all currently known variants thereof and any further variants, which may be elucidated.

As used herein, the terms "oligomeric compound" refers to an oligonucleotide which can induce a desired therapeutic effect in humans through for example binding by hydrogen bonding to either a target gene "Chimeraplast" and "TFO", to the RNA transcript(s) of the target gene "antisense inhibitors", "siRNA", "ribozymes" and oligozymes" or to the protein(s) encoding by the target gene "aptamer", spiegelmer" or "decoy".

As used herein, the term "mRNA" means the presently known mRNA transcript(s) of a targeted gene, and any further transcripts, which may be identified.

As used herein, the term "modulation" means either an increase (stimulation) or a decrease (inhibition) in the expression of a gene. In the present invention, inhibition is the preferred form of modulation of gene expression and mRNA is a preferred target.

As used herein, the term "targeting" an antisense compound to a particular target nucleic acid means providing the antisense oligonucleotide to the cell, animal or human in such a way that the antisense compound is able to bind to and modulate the function of its intended target.

As used herein, "hybridisation" means hydrogen bonding, which may be Watson-Crick, Holstein, reversed Holstein hydrogen bonding, etc. between complementary nucleoside or nucleotide bases. Watson and Crick showed approximately fifty years ago that deoxyribo nucleic add (DNA) is composed of two strands which are held together in a helical configuration by hydrogen bonds formed between opposing complementary nucleobases in the two strands. The four nudeobases, commonly found in DNA are guanine (G), adenine (A), thymine (T) and cytosine (C) of which the G nucleobase pairs with C, and the A nucleobase pairs with T. In RNA the nucleobase thymine is replaced by the nucleobase uracil (U), which similarly to the T nucleobase pairs with A. The chemical groups in the nucleobases that participate in standard duplex formation constitute the Watson-Crick face. Hoogsteen showed a couple of years later that the purine nucleobases (G and A) in addition to their Watson-Crick face have a Hoogsteen face that can be recognised from the outside of a duplex, and used to bind pyrimidine oligonucleotides via hydrogen bonding, thereby forming a triple helix structure.

In the context of the present invention "complementary" refers to the capacity for precise pairing between two nucleotides or nucleoside sequences with one another. For example, if a nucleotide at a certain position of an oligonucleotide is capable of hydrogen bonding with a nucleotide at the corresponding position of as DNA or RNA molecule, then the oligonucleotide and the DNA or RNA are considered to be complementary to each other at that position. The DNA or RNA and the oligonucleotide are considered complimentary to each other when a sufficient number of nucleotides in the oligonucleotide can form hydrogen bonds with corresponding nucleotides in the target DNA or RNA to enable the formation of a stable complex. To be stable *in vitro* or *in vivo* the sequence of an antisense compound need not be 100% complementary to its target nucleic add. The terms "complementary" and "specifically hybridisable" thus Imply that the antisense compound binds sufficiently strongly and specifically to the target molecule to provide the desired interference with the normal function of the target whilst leaving the function of non-target mRNAs unaffected.

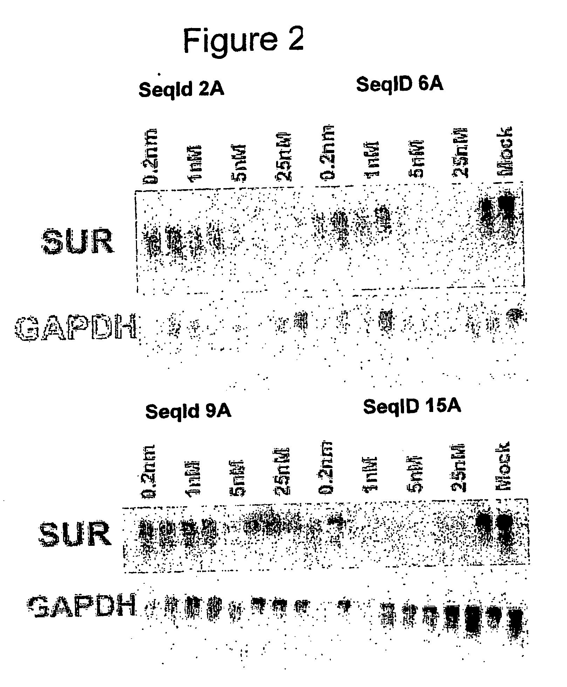

The oligomeric compounds according to the invention are potent modulators of the target. In vitro inhibition of the target is shown in Table 1 measured by Real time PCR. Figure 2 shows in vitro potency of oligomeric compounds measured by Northern Blot. Very low IC₅₀ values of oligomeric compounds are shown in Table 3 which shows that the compounds according to the invention can constitute the active compound in a pharmaceutical composition.

The nucleotides are typically linked to each other by means of a linkage group selected from the group consisting of a phosphate group, a phosphorothioate group and a boranophosphate group. Suitably, some or all of the nucleotides are linked to each other by means of a phosphate group. Suitably, all nucleotides are linked to each other by means of a phosphate group.

Similarly, the nucleotides of the invention are typically linked to each other by means of a linkage group selected from the group consisting of a phosphate group and a phosphorothioate group.

Oligomeric compounds according to the invention are SEQ ID NO 130.

In another embodiment of the invention, said nucleotides are linked to each other by means of a phosphorothioate group, such as all nucleotides being linked to each other by means of a phosphorothioate group. An interesting embodiment of the invention is directed to compounds of SEQ NO 130 wherein each linkage group within the compound is a phosphorothioate group. Such modifications is denoted by the subscript S. Alternatively stated, one aspect of the invention is directed to compound of SEQ NO 130_{A}.

A further aspect of the invention is directed to compound of SEQ NO 130_{B}.

A further aspect of the invention is directed to a compound of SEQ NO 130_{C}.

A preferred nucleotide analogue of the invention is LNA.

A further preferred nucleotide analogue of the invention is wherein the internucleoside phosphate linkage is a phosphorothioate.

A still further preferred nucleotide analogue is wherein the nucleotide is LNA with an internucleoside phosphorothioate linkage.

In a particularly interesting embodiment, the compound comprises at least one of nucleotide analogues, wherein said nucleotide analogue is a locked nucleic acid (LNA) of the formula 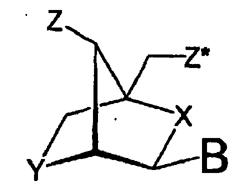 wherein Z and Z* are independently absent, selected among an internucleoside linkage, a terminal group or a protecting group; wherein X and Y are independently selected from the group consisting of O, S, NR, CH₂, CH, (if part of a double bond), CH₂-O, CH₂-S, CH₂-NR, CH₂CH₂, CH₂-CH (if part of a double bond) and CH=CH, where R is hydrogen or C₁₋₄-alkyl. The bonds represent connection to the linkage group. Typically, X is O and Y are independently selected from the group consisting of O, S and NR, where R is hydrogen or C₁₋₄-alkyl. More typically, X is O and Y is selected from the group consisting of O, S and NH. Most typically, X is O and Y is O**.** In embodiments wherein at least one of the LNA nucleotides is at the 3'-end, at said position Z is a terminal group and Z* is an internucleoside linkage. In embodiments wherein at least one of the LNA nucleotides is at the 5'-end, at said position Z is absent and Z* is a terminal group. Within the nucleotide sequence, Z is absent and Z* is an internucleoside linkage

In a suitable embodiment of the invention comprising LNA as the nucleotide analogues, said LNA is in the β-D form.

In embodiments of the invention comprising at least one LNA as the nucleotide analogues, such as 7 or 8 LNA nucleotide analogues said nucleotides and/or nucleotide analogues are linked to each other by means of a linkage group selected from the group consisting of a phosphate group and a phosphorothioate group. In a suitable embodiment of the invention comprising LNA nucleotide analogues, said nucleotides and/or nucleotide analogues are linked to each other by means of a phosphate group. In a preferred embodiment of the invention comprising LNA nucleotide analogues said nucleotides and/or nucleotide analogues are linked to each other by means of a phosphorothioate group.

In a combination of interesting embodiment, in embodiments of the invention comprising LNA nucleotide analogues said nucleotide and/or nucleotide analogues are linked to each other by means of a phosphorothioate group, wherein X is O and Y is O, and said LNA is in the β-D form.

Subseqences comprising a stretch of LNAs, followed by a stretch of nucleotides, followed by a stretch of LNAs are known as gapmers.

Suitably, said subsequence comprises a stretch of 4 LNAs, as defined herein, followed by a stretch of 8 nucleotides, which is followed by a stretch of 4 LNAs as defined herein.

In embodiments of the compound of the invention comprises a stretch of 4 LNAs as defined herein, followed by a stretch of 8 nucleotides, which is followed by a stretch of 3 LNAs as defined herein, which is followed by a single nuceloside at the 3' end of the oligomer.

In embodiments of the compound of the invention said compound comprises a stretch of LNAs, followed by a stretch of nucleotides, which is followed by a stretch of LNAs as defined herein as gapmers, said nucleotides and/or LNAs are linked to each other by means of a linkage group selected from the group consisting of a phosphate group and a phosphorothioate group.

Suitably, said nucleotides and/or said LNAs are linked together by means of phosphate groups. Typically, said nucleotides and/or said LNAs are linked together by means of phosphorothioate groups.

In embodiments of the compound of the invention consists of a stretch of 4 LNAs, as defined herein, a stretch of 8 nucleotides, and a stretch of 4 LNAs, as defined herein, so as to make a total of 16 nucleotides and nucleotide analogues in said subsequence, said nucleotides and said LNAs are linked together by means of phosphorothioate groups.

In a suitable embodiment, the compound is SEQ ID NO: 130a.

In a further suitable embodiment, the compound of the invention has a sequence SEQ ID NO: 130 and wherein said sequence consisting of a stretch of 4 LNAs, as defined herein, a stretch of 8 nucleotides, and a stretch of 4 LNAs, as defined herein, so as to make a total of 16 nucleotides and nucleotide analogues in said compound, said nucleotides and said LNAs-being linked together by means of phosphorothioate groups.

In a suitable embodiment, the compound consists of SEQ ID NO: 130a. In the immediately aforementioned individual suitable embodiments wherein the compound is SEQ ID NO: 130a, the 3' end. LNA of the compound may suitably be replaced by the corresponding nucleotide.

A further aspect of the invention relates to a conjugate comprising the compound as defined herein at least one non-nucleotide or non-polynucletide moiety covalently attached to said compound.

In a related aspect of the invention, the compound of the invention is linked to ligands so as to form a conjugate. Said ligands are intended to increase the cellular uptake of the conjugate relative to the antisense oligonucleotides this conjugation can take place at the terminal positions 5'/3'-OH but the ligands may also take place at the sugars and/or the bases. In particular, the growth factor to which the antisense oligonucleotide may be conjugated, may comprise transferrin or folate. Transferrin-polylysine-oligonucleotide complexes or folate-polylysine-oligonucleotide complexes may be prepared for uptake by cells expressing high levels of transferrin or folate receptor. Other examples of conjugates/lingands are cholesterol moieties, duplex intercalators such as acridine, poly-L-lysine, "end-capping" with one or more nuclease-resistant linkage groups such as phosphoromonothioate, and the like.

The preparation of transferrin complexes as carriers of oligonucleotide into cells is described by Wagner et al ., Proc. Natl. Acad. Sci. USA 87, 3410-3414,(1390). Cellular delivery of folate-macromolecule conjugates via folate receptor endocytosis, including delivery of an antisense oligonucleotide, is described by Low et al., U.S. Patent 5,108,921. Also see, Leamon et al., Proc. Natl. Acad. Sci. 88, 5572 (1991).

The compounds or conjugates of the invention may also be conjugated or further conjugated to active drug substance, for example, aspirin, Ibuprofen, a sulfa drug, an antidiabetic, an antibacterial agent, a chemotherapeutic agent or an antibiotic.

A particularly interesting aspect of the invention is directed to a pharmaceutical composition comprising a compound as defined herein or a conjugate as defined herein, and a pharmaceutically acceptable diluent, carrier or adjuvant.

It should be understood that the present invention also particularly relevant for a pharmaceutical composition, which comprises a least one antisense oligonucleotide construct of the invention as an active ingredient. It should be understood that the pharmaceutical composition according to the invention optionally comprises a pharmaceutical carrier, and that the pharmaceutical composition optionally comprises further antisense compounds, chemotherapeutic agents, anti-inflammatory compounds, antiviral compounds and/or immuno-modulating compounds.

As stated, the pharmaceutical composition of the invention may further comprise at least one chemotherapeutic agent. The chemotherapeutic compound is typically selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BICNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine).

The oligomeric compound or conjugate comprised in this invention can be employed in a variety of pharmaceutically acceptable salts. As used herein, the term refers to salts that retain the desired biological activity of the herein identified compounds and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, N,N-dibenzylethylene-diamine, *D*-glucosamine, tetraethylammonium, or ethylenediamine; or (c) combinations of (a) and (b); e.g., a zinc tannate salt or the like.

In one embodiment of the invention the oligomeric compound of conjugate may be in the form of a pro-drug. Oligonucleotides are by virtue negatively charged ions. Due to the lipophilic nature of cell membranes the cellular uptake of oligonucleotides are reduced compared to neutral or lipophilic equivalents. This polarity "hindrance" can be avoided by using the pro-drug approach (see e.g. Crooke, R. M. (1998) in Crooke, S. T. Antisense research and Application. Springer-Verlag, Berlin, Germany, vol. 131, pp. 103-140). In this approach the oligonucleotides are prepared in a protected manner so that the oligo is neutral when it is administered. These protection groups are designed in such a way that so they can be removed then the oligo is taken up be the cells. Examples of such protection groups are S-acetylthioethyl (SATE) or S-pivaloylthioethyl (t-butyl-SATE). These protection groups are nuclease resistant and are selectively removed intracellulary.

The invention also includes the formulation of one or more oligonucleotide compound or conjugate as disclosed herein. Pharmaceutically acceptable binding agents and adjuvants may comprise part of the formulated drug. Capsules, Tablets and pills etc. may contain for example the following compounds: microcrystalline cellulose, gum or gelatin as binders; starch or lactose as excipients; stearates as lubricants; various sweetening or flavouring agents. For capsules the dosage unit may contain a liquid carrier like fatty oils. Likewise coatings of sugar or enteric agents may be part of the dosage unit. The oligonucleotide formulations may also be emulsions of the active pharmaceutical ingredients and a lipid forming a micellular emulsion.

An oligonucleotide of the invention may be mixed with any material that do not impair the desired action, or with material that supplement the desired action. These could include other drugs including other nucleotide compounds.

For parenteral, subcutaneous, intradermal or topical administration the formulation may include a sterile diluent, buffers, regulators of tonicity and antibacterials. The active compound may be prepared with carriers that protect against degradation or immediate elimination from the body, including implants or microcapsules with controlled release properties. For intravenous administration the preferred carriers are physiological saline or phosphate buffered saline.

Preferably, an oligomeric compound is included in a unit formulation such as in a pharmaceutically accepTable carrier or diluent in an amount sufficient to deliver to a patient a therapeutically effective amount without causing serious side effects in the treated patient.

The pharmaceutical compositions of the present invention may be administered in a number of ways depending upon whether local or systemic treatment is desired and upon the area to be treated. Administration may be (a) oral (b) pulmonary, e.g., by inhalation or insufflation of powders or aerosols, including by nebulizer; intratracheal, intranasal, (c) topical including epidermal, transdermal, ophthalmic and to mucous membranes including vaginal and rectal delivery; or (d) parenteral including intravenous, intraarterial, subcutaneous, intraperitoneal or intramuscular injection or infusion; or intracranial, e.g., intrathecal or intraventricular, administration. In one embodiment the active oligo is administered IV, IP, orally, topically or as a bolus injection or administered directly in to the target organ.

Pharmaceutical compositions and formulations for topical administration may include transdermal patches, ointments, lotions, creams, gels, drops, sprays, suppositories, liquids and powders. Conventional pharmaceutical carriers, aqueous, powder or oily bases, thickeners and the like may be necessary or desirable. Coated condoms, gloves and the like may also be useful. Preferred topical formulations include those in which the oligonucleotides of the invention are in admixture with a topical delivery agent such as lipids, liposomes, fatty acids, fatty acid esters, steroids, chelating agents and surfactants. Compositions and formulations for oral administration include but is not restricted to powders or granules, microparticulates, nanoparticulates, suspensions or solutions in water or non-aqueous media, capsules, gel capsules, sachets, Tablets or minitablets. Compositions and formulations for parenteral, intrathecal or intraventricular administration may include sterile aqueous solutions which may also contain buffers, diluents and other suiTable additives such as, but not limited to, penetration enhancers, carrier compounds and other pharmaceutically accepTable carriers or excipients.

Pharmaceutical compositions of the present invention include, but are not limited to, solutions, emulsions, and liposome-containing formulations. These compositions may be generated from a variety of components that include, but are not limited to, preformed liquids, self- emulsifying solids and self-emulsifying semisolids. Delivery of drug to tumour tissue may be enhanced by carrier-mediated delivery including, but not limited to, cationic liposomes, cyclodextrins, porphyrin derivatives, branched chain dendrimers, polyethylenimine polymers, nanoparticies and microspheres (Dass CR. J Pharm Pharmacol 2002; 54(1):3-27).

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both; and then, if necessary, shaping the product.

The compositions of the present invention may be formulated into any of many possible dosage forms such as, but not limited to, tablets, capsules, gel capsules, liquid syrups, soft gels and suppositories. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

LNA containing oligomeric compounds are useful for a number of therapeutic applications as indicated above. In general, therapeutic methods of the invention include administration of a therapeutically effective amount of an LNA-moditied oligonucleotide to a mammal, particularly a human.

In a certain embodiment, the present invention provides pharmaceutical compositions containing (a) one or more antisense compounds and (b) one or more other chemotherapeutic agents which function by a non-antisense mechanism. When used with the compounds of the invention, such chemotherapeutic agents may be used individually (e.g. mithramydn and oligonucleotide), sequentially (e.g. mithramycin and oligonucleotide for a period of time followed by another agent and oligonucleotide), or in combination with one or more other such chemotherapeutic agents or in combinations with radiotherapy. All chemotherapeutic agents known to a person skilled in the art are here incorporated as combination treatments with compound according to the invention.

Anti-inflammatory drugs, including but not limited to nonsteroidal anti- inflammatory drugs and corticosteroids, antiviral drugs, and lmmuno-modulating drugs may also be combined in compositions of the invention. Two or more combined compounds may be used together or sequentially.

In another embodiments, compositions of the invention may contain one or more antisense compounds, particularly oligonucleotides, targeted to a first nucleic acid and one or more additional antisense compounds targeted to a second nucleic acid target. Two or more combined compounds may be used together or sequentially.

Dosing is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient.

Optimum dosages may vary depending on the relative potency of individual oligonucleotides. Generally it can be estimated based on EC50s found to be effective in *in vitro* and *in vivo* animal models. In general, dosage is from 0.01 µg to 1 g per kg of body weight, and may be given once or more daily, weekly, monthly or yearly, or even once every 2 to 10 years or by continuous infusion for hours up to several months. The repetition rates for dosing can be estimated based on measured residence times and concentrations of the drug in bodily fluids or tissues. Following successful treatment, it may be desirable to have the patient undergo maintenance therapy to prevent the recurrence of the disease state.

As stated, in an interesting embodiment of the invention, the oligomeric compounds contain at least one unit of chemistry termed LNA (Locked Nucleic Acid).

LNA monomer typically refers to a bicyclic nucleotide analogue wherein the nucleoside moiety is an analogue as described in the International Patent Application WO 99/14226 and subsequent applications, WO0056746, WO0056748, WO0066604, WO00125248, WO0228875, WO2002094250 and WO3006475. Preferred LNA nucleotide structures for forming a compound of the invention are exemplified in Scheme 2 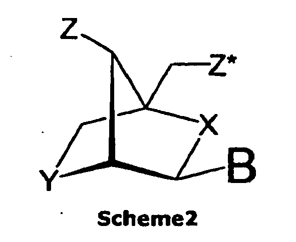 wherein X and Y are independently selected among the groups -O-, -S-, -N(H)-, N(R)-, -CH₂- or -CH- (if part of a double bond), -CH₂-O-, -CH₂-S-, -CH₂-N(H)-, -CH₂-N(R)-, -CH₂-CH₂- or -CH₂-CH- (if part of a double bond), -CH=CH-, where R is selected form hydrogen and C₁₋₄-alky; wherein Z and Z* are independently absent, selected among an internucleoside linkage, a terminal group or a protecting group. In embodiments wherein at least one of the LNA nucleotides is at the 3'-end, at said position Z is a terminal group and Z* is an internucleoside linkage. In embodiments wherein at least one of the LNA nucleotides is at the 5'-end, at said position Z is absent and Z* is a terminal group. Within the nucleotide sequence, Z is absent and Z* is an internucleoside linkage. The asymmetric groups may be found in either orientation. In Scheme 2, the 4 chiral centers are shown in a fixed configuration. However, the configuarations in Scheme 2 are not necessarily fixed. Also comprised in this invention are compounds of the general Scheme 2 in which the chiral centers are found in different configurations, such as those represented in Scheme 3 or 4. Thus, the intention in the illustration of Scheme 2 is not to limit the configuration of the chiral centre. Each chiral center in Scheme 2 can exist in either R or S configuration. The definition of R (rectus) and S (sinister) are described in the IUPAC 1974 Recommendations, Section E, Fundamental Stereochemistry: The rules can be found in Pure Appl. Chem. 45, 13-30, (1976) and in "Nomenclature of organic Chemistry" pergamon, New York, 1979.

Z and Z* serve for forming an internucleoside linkage, are a terminal group or a protecting group, depending on the position of the LNA within the compound, namely within the subsequence or at the 3' end of the subsequence or compound.

The internucleoside linkage may be -O-P(O)₂-O-, -O-P(O,S)-O-, -O-P(S)₂-O-, -S-P(O)₂-O-, -S-P(O,S)-O-, -S-P(S)₂-O-, -O-P(O)₂₋S-, -O-P(O,S)-S-, -S-P(O)₂-S-, -O-PO(R^{H})-O-, O PO(OCH₃)-O-, -O-PO(NR^{H})-O-, -O-PO(OCH₂CH₂S-R^{H})-O-, -O-PO(BH₃)-O-, -O-PO(NHR^{H})-O-; -O-P(O)₂-NR^{H}-,-NR^{H}-P(O)₂-O-, -NR^{H}-CO-O-, -NR^{H}-CO-NR^{H}-, -O-CO-O-, -O-CO-NR^{H}-, -NR^{H}-CO-CH₂-, -O-CH₂-CO-NR^{H} -, -O-CH₂-CH₂-NR_{H}-, -CO-NR^{H}-CH₂-, -CH₂-NR^{H}-CO-, -O-CH₂-CH₂-S-, -S-CH₂-CH₂-O-, -S-CH₂-CH₂-S-, -CH₂-SO₂-CH₂-, -CH₂-CO-NR^{H}-, -O-CH₂-CH₂-NR^{H}-CO -, -CH₂-NCH₃-O-CH₂-, where R^{H} is selected form hydrogen and C₁₋₄-alkyl,

The terminal groups are selected independently among from hydrogen, azido, halogen, cyano, nitro, hydroxy, Prot-O-, Act-O-, mercapto, Prot-S-, Act-S-, C₁-₆-alkylthio, amino, Prot-N(R^{H})-, Act-N(R^{H})-, mono- or di(C₁-₆-alkyl)amino, optionally substituted C₁-₆-alkoxy, optionally substituted C₁-₆-alkyl, optionally substituted C₂-₆-alkenyl, optionally substituted C₂-₆-alkenyloxy, optionally substituted C₂-₆-alkynyl, optionally substituted C₂-₆-alkynyloxy, monophosphate, monothiophosphate, diphosphate, dithiophosphate, triphosphate, trithiophosphate, DNA intercalators, photochemically active groups, thermochemically active groups, chelating groups, reporter groups, ligands, carboxy, sulphono, hydroxymethyl, Prot-O-CH₂-, Act-O-CH₂-, aminomethyl, Prot-N(R^{H}) -CH₂-, Act-N(R^{H})-CH₂-, carboxymethyl, sulphonomethyl, where Prot is a protection group for -OH, -SH, and -NH(R^{H}), respectively, Act is an activation group for -OH, -SH, and -NH(R^{H}), respectively, and R^{H} is selected from hydrogen and C₁₋₆-alkyl;

The protection groups of hydroxy substituents comprises substituted trityl, such as 4,4'-dimethoxytrityloxy (DMT), 4-monomethoxytrityloxy (MMT), and trityloxy, optionally substituted 9-(9-phenyl)xanthenpoxy (pixyl), optionally substituted methoxytetrahydropyranyloxy (mthp), silyloxy such as trimethylsilyloxy (TMS), triisopropylsilytoxy (TIPS), *tert*-butyldimethylsilyloxy (TBDMS), triethylsilyloxy, and phenyldimethylsilyloxy, *tert-*butylethers, acetals. (including two hydroxy groups), acyloxy such as acetyl or halogen substituted acetyls, *e.g.* chloroactyloxy or fluoroacetyloxy, isobutyryloxy, pivaloyloxy, benzoyloxy and substituted benzoyls, methoxymethyloxy (MOM), benzyl ethers or substituted benzyl ethers such as 2,6-dichlorobenzyloxy.(2,6-Cl₂Bzl). Alternatively when Z or Z* is hydroxyl they may be protected by attachment to a solid support optionally through a linker.

When Z or Z* are amino groups, illustrative examples of the amino protection group are fluorenylmethoxycarbonyl (Fmoc), *tert*-butyloxycarbonylamino (BOC), trifluoroacetylamino, allyloxycarbonylamino (alloc, AOC), Z benzyloxycarbonylamino ( Cbz), substituted benzyloxycarbonylaminos such as 2-chloro benzyloxycarbonylamino (2-ClZ), monomethoxytritylamino (MMT), dimethoxytritylamino (DMT), phthaloylamino, and 9-(9-phenyl)xanthenylamino (pixyl).

In the embodiment above, Act designates an activation group, for -OH, -SH, and -NH(R^{H}), respectively. Such activation groups are, *e.g*., selected from optionally substituted O-phosphoramidite, optionally substituted O-phosphortriester, optionally substituted O-phosphordiester, optionally substituted H-phosphonate, and optionally substituted O-phosphonate.

In the present context, the term "phosphoramidite" means a group of the formula -P(OR*)-N(R^{y})₂, wherein R^{x} designates an optionally substituted alkyl group, *e.g*, methyl, 2-cyanoethyl, or benzyl; and each of R^{y} designate optionally substituted alkyl groups, *e.g*. ethyl or isopropyl, or the group -N(R^{y})₂ forms a morpholino group (-N(CH₂CH₂)₂O). R^{x} preferably designates 2-cyanoethyl and the two R^{y} are preferably identical and designate isopropyl. Thus, an especially relevant phosphoramidite is N,N-diisopropyl-*O*-(2cyanoethyl)phosphoramidite.

B constitutes a natural or non-reatural nucleobase selected among adenine, cytosine, 5-methylcytosine, isocytosine, pseudoisocytosine, guanine, thymine, uracil, 5-bromduracil, 5-propynyluracil, 5-propyny-6-fluoroluracil, 5-methylthiazoleuracil, 6-aminopurine, 2-aminopurine, inosine, 2,6- diaminopurine, -7-propyne-7-deazaadenine, 7-propyne-7-deazaguanine, 2-chloro-6-aminopurine.

Particularly preferred bicyclic structures are shown in Scheme 3 below: 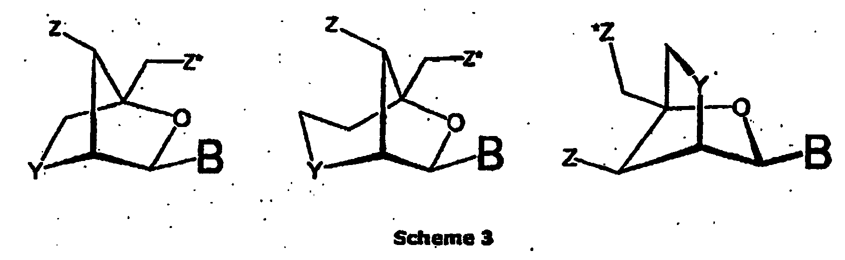 Where Y is -O-, -S-, -NH-, or N(R^{H}),
Z and Z* are independently absent, selected among an internucleoside linkage, a terminal group or a protecting group. In embodiments wherein at least one of the LNA nucleotides is at the 3'-end, at said position Z is a terminal group and Z* is an internucleoside linkage. In embodiments wherein at least one of the LNA nucleotides is at the 5'-end, at said portion Z is absent and Z* is a terminal group. Within the nucleotide sequence, Z is absent and Z* is an internucleoside linkage.

The internucleotide linkage may be -O-P(O)₂-O-, -O-P(O,S)-O-, -O-P(S)₂-O-, -S-O(O)₂-O-, -S-P(O,S)-O-, -S-P(S)₂-O-, -O-P(O)-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, where R^{H} is selected form hydrogen and C₁₋₄-alkyl.

The terminal groups are selected independently from hydrogen, azido, halogen, cyano, nitro, hydroxy, Prot-O-, Act-O-, mercapto, Prot-S-, Act-S-, C₁₋₆-alkylthio, amino, Prot-N(R^{H})-, Act-N(R^{H})-, mono- or di(C₁₋₆-alkyl)amino, optionally substituted C₁₋₆-alkoxy, optionally substituted C₁₋₆-alkyl, optionally substituted monophosphate, monothiophosphate, diphosphate, dithiophosphate triphosphate, trithiophosphate, where Prot is a protection group for -OH, -SH, and -NH(R^{H}), respectively, Act is an activation group for -OH, -SH, and -NH(R^{H}), respectively, and R^{H} is selected from hydrogen and C₁₋₆-alkyl.

The protection groups of hydroxy substituents comprises substituted trityl, such as 4,4'-dimethoxytrityloxy (DMT), 4-monomethoxytrityloxy (MMT), optionally substituted 9-(9-phenyl)xanthenyloxy (pixyl), optionally substituted methoxytetrahydropyranyloxy (mthp), silyloxy such as trimethylsilyloxy (TMS), triisopropylsilyloxy (TIPS), *tert*-butyldimethylsilyloxy (TBDMS), triethylsilyloxy, and phenyldimethylsilyloxy, *tert*-butylethers, acetals (including two hydroxy groups), acyloxy such as acetyl Alternatively when Z or Z* is hydroxyl they may be protected by attachment to a solid support optionally through a linker.

Specifically preferred LNA units are shown in scheme 4.

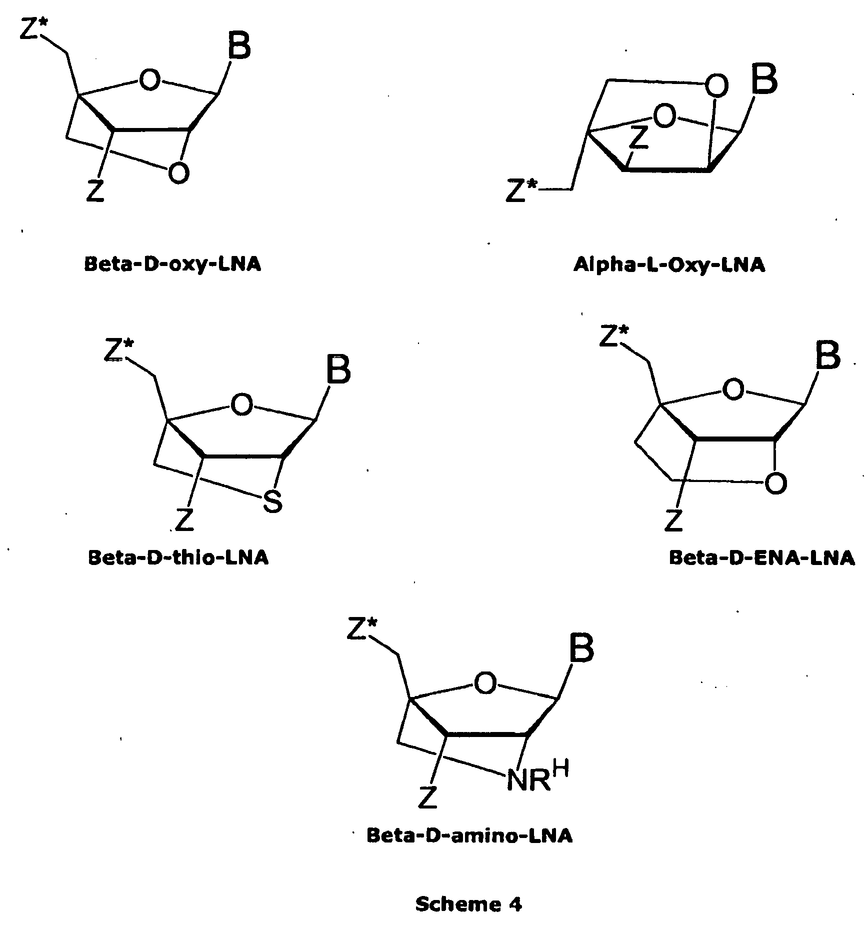

When Z or Z* is amino groups, illustrative examples of the amino protection protections are fluorenylmethoxycarbonylamino (Fmoc), *tert*-butyloxycarbonylamino (BOC), trifluoroacetylamino, allyloxycarbonylamino (alloc, AOC), monomethoxytritylamino (MMT), dimethoxytritylamino (DMT), phthaloylamino.

In the embodiments above, Act designates an activation group for -OH, -SH, and -NH(R^{H}), respectively. Such activation groups are, *e.g*., selected from optionally substituted O-phosphoramidite, optionally substituted O-phosphortriester, optionally substituted O-phosphordiester, optionally substituted H-phosphonate, and optionally substituted O-phosphonate.

In the present context, the term "phosphoramidite" means a group of the formula -P(OR^{x})-N(R^{y})₂, wherein R^{x} designates an optionally substituted alkyl group, *e.g.* methyl, 2-cyanoethyl, and each of R^{y} designate optionally substituted alkyl groups, R^{x} preferably designates 2-cyanoethyl and the two R^{y} are preferably identical and designate isopropyl. Thus, an especially relevant phosphoramidite is N,N-diisopropyl-*O*-(2-cyanoethyl)-phosphoramidite.

B, in the context of Schemes 3 and 4, constitutes a natural or non-natural nucleobase and selected among adenine, cytosine, 5-methylcytosine, isocytosine, pseudoisocytosine, guanine, thymine, uracil, 5-bromouracil, 5-propynyluracil, 6-aminopurine, 2-aminopurine, inosine, diaminopurine, 2-chloro-6-aminopurine.

A person skilled in the art will appreciate that oligomeric compounds containing LNA can be used to combat survivin linked diseases by many different principles, which thus falls within the spirit of the present invention.

For instance, LNA oligomeric compounds may be designed as antisense inhibitors, which are single stranded nucleic acids that prevent the production of a disease causing protein, by intervention at the mRNA level. Also, they may be designed as Ribozymes or Oligozymes which are antisense oligonucleotides which in addition to the target binding domain(s) comprise a catalytic activity that degrades the target mRNA (ribozymes) or comprise an external guide sequence (EGS) that recruit an endogenous enzyme (RNase P) which degrades the target mRNA (oligozymes)

Equally well, the LNA oligomeric compounds may be designed as siRNA's which are small double stranded RNA molecules that are used by cells to silence specific endogenous or exogenous genes by an as yet poorly understood "antisense-like" mechanism.

LNA oligomeric compounds may also be designed as Aptamers (and a variation thereof, termed Spiegelmers) which are nucleic acids that through intra-molecular hydrogen bonding adopt three-dimensional structures that enable them to bind to and block their biological targets with high affinity and specificity. Also, LNA oligomeric compounds may be designed as Decoys, which are small double-stranded nucleic acids that prevent cellular transcription factors from transactivating their target genes by selectively blocking their DNA binding site.

Furthermore, LNA oligomeric compounds may be designed as Chimeraplasts, which are small single stranded nucleic acids that are able to specifically pair with and alter a target gene sequence. LNA containing oligomeric compounds exploiting this principle therefore may be particularly useful for treating survivin linked diseases that are caused by a mutation in the survivin gene.

Dictated in part by the therapeutic principle by which the oligonucleotide is intended to operate, the LNA oligomeric compounds in accordance with this invention are antisense compounds comprising 16 nucleobases. The compouns shown in Table 1 and 2 are all 16-mers.

Referring to the above principles by which an LNA oligomeric compound can elicit its therapeutic action the target of an oligonucleotide may be the survivin gene, the mRNA or the protein. LNA oligomeric compounds may be designed as an antisense inhibitor directed against the survivin pre-mRNA or survivin mRNA. The oligonucleotides may hybridize to any site along the survivin pre-mRNA or mRNA such as sites in the 5' untranslated leader, exons, introns and 3'untranslated tail.

The oligonucleotide may hybridize to a portion of the human survivin pre-mRNA or mRNA that comprises the translation-initiation site. The survivin oligonucleotide may comprise a CAT sequence, which is complementary to the AUG initiation sequence of the survivin pre-mRNA or RNA. The survivin oligonucleotide may hybridize to a portion of the splice donor site of the human survivin pre-mRNA. The survivin oligonucleotide may hybridize to a portion of the splice acceptor site of the human survivin pre-mRNA. The survivin oligonucleotide may hybridize to portions of the human survivin pre-mRNA or mRNA involved in polyadenylation, transport or degradation.
The skilled person will appreciate that preferred oligonucleotides are those that hybridize to a portion of the survivin pre-mRNA or mRNA whose sequence does not commonly occur in transcripts from unrelated genes so as to maintain treatment specificity.
The oligomeric compound of the invention are designed to be sufficiently complementary to the target to provide the desired clinical response e.g. the oligomeric compound must bind with sufficient strength and specificity to its target to give the desired effect. In one embodiment, said compound modulating survivin is designed so as to also modulate other specific nucleic acids which do not encode survivin.

It is preferred that the oligomeric compound according to the invention is designed so that intra- and intermolecular oligonucleotide hybridisation is avoided.

In many cases the identification of an LNA oligomeric compound effective in modulating survivin activity in vivo or clinically is based on sequence information on the target gene: However, one of ordinary skill in the art will appreciate that such oligomeric compounds can also be identified by empirical testing. Such survivin oligomeric compounds having, for example, less sequence homology, greater or fewer modified nucleotides, or longer or shorter lengths, compared to those of the preferred embodiments, may nevertheless demonstrate responses in clinical treatments.

In one embodiment of the invention the oligomeric compounds are suitable antisense drugs. The design of a potent and safe antisense drug requires the fine-tuning of diverse parameters such as affinity/specificity, stability in biological fluids, cellular uptake, mode of action, pharmacokinetic properties and toxicity.

Affinity & specificity: LNA with an oxymethylene 2'-O, 4'-C linkage (β-D-oxy-LNA), exhibits unprecedented binding properties towards DNA and RNA target sequences. Likewise LNA derivatives, such as amino-, thio- and α-L-oxy-LNA display unprecedented affinities towards complementary RNA and DNA and in the case of thio-LNA the affinity towards RNA is even better than with the β-D-oxy-LNA.

In addition to these remarkable hybridization properties, LNA monomers can be mixed and act cooperatively with DNA and RNA monomers, and with other nucleic acid analogues, such as 2'-O-alkyl modified RNA monomers. As such, the oligonucleotides of the present invention are composed of β-D-oxy-LNA in any combination with DNA. The unprecedented binding affinity of LNA towards DNA or RNA target sequences and its ability to mix freely with DNA, RNA and a range of contemporary nucleic acid analogues has a range of important consequences according to the invention for the development of effective and safe antisense compounds.

Firstly, in one embodiment of the invention it enables a considerable shortening of the usual length of an antisense oligo (from 20-25 mers to, 16 mers) without compromising the affinity required for pharmacological activity. As the *intrinsic specificity* of an oligo is inversely correlated to its length, such a shortening will significantly increase the specificity of the antisense compound towards its RNA target. One embodiment of the invention is to, due to the sequence of the humane genome is available and the annotation of its genes rapidly progressing, identify the shortest possible, unique sequences in the target mRNA.

In another embodiment, the use of LNA to reduce the size of oligos significantly eases the process and prize of manufacture thus providing the basis for antisense therapy to become a commercially competitive treatment offer for a diversity of diseases.

In another embodiment, the unprecedented affinity of LNA can be used to substantially enhance the ability of an antisense oligo to hybridize to its target mRNA *in-vivo* thus significantly reducing the time and effort required for identifying an active compound as compared to the situation with other chemistries.

In another embodiment, the unprecedented affinity of LNA is used to enhance the potency of antisense oligonucleotides thus enabling the development of compounds with more favorable therapeutic windows than those currently in clinical trials.

When designed as an antisense inhibitor, the oligonucleotides of the invention bind to the target nucleic acid and modulate the expression of its cognate protein. Preferably, such modulation produces an inhibition of expression of at least 10% or 20% compared to the normal expression level, more preferably at least a 30%, 40%, 50%, 60%, 70%, 80%, or 90% inhibition compared to the normal expression level.

The LNA oligonucleotides of the invention contain other residues than β-D-oxy-LNA such as native DNA monomers.

Stability in biological fluids: LNA monomers may be incorporated into a standard DNA or RNA oligonucleotide to increase the stability of the resulting oligomeric compound in biological fluids e.g. through the increase of resistance towards nucleases (endonucleases and exonucleases). The extent of stability will depend on the number of LNA monomers used, their position in the oligonucleotide and the type of LNA monomer used. Compared to DNA and phosphorothioates the following order of ability to stabilize an oligonucleotide against nucleolytic degradation can be established: DNA << phosphorothioates ∼ oxy-LNA < α-L-LNA < amino-LNA < thio-LNA.

Given the fact that LNA is compatible with standard DNA synthesis and mixes freely with many contemporary nucleic acid analogues nuclease resistance of LNA- oligomeric compounds can be further enhanced by either incorporating other analogues that display increased nuclease stability or by exploiting nuclease-resistant internucleoside linkages e.g. phosphoromonothioate, phosphorodithioate, and methylphosphonate linkages, etc.

Mode of action: Antisense compounds according to the invention may elicit their therapeutic action via a variety of mechanisms and may be able to combine several of these in the same compound. In one scenario, binding of the oligonucleotide to its target (pre-mRNA or mRNA) acts to prevent binding of other factors (proteins, other nucleic acids, etc.) needed for the proper function of the target i.e. operate by steric hindrance. For instance, the antisense oligonucleotide may bind to sequence motifs in either the pre-mRNA or mRNA that are important for recognition and binding of transacting factors involved in splicing, poly-adenylation, cellular transport, post-transcriptional modifications of nucleosides in the RNA, capping of the 5'-end, translation, etc. In the case of pre-mRNA splicing, the outcome of the interaction between the oligonucleotide and its target may be either suppression of expression of an undesired protein, generation of alternative spliced mRNA encoding a desired protein or both.

In another embodiment, binding of the oligonucleotide to its target disables the translation process by creating a physical block to the ribosomal machinery, i.e. tranlational arrest.

In yet another embodiment, binding of the oligonucleotide to its target interferes with the RNAs ability to adopt secondary and higher order structures that are important for its proper function, i.e. structural interference. For instance, the oligonucleotide may interfere with the formation of stem-loop structures that play crucial roles in different functions, such as providing additional stability to the RNA or adopting essential recognition motifs for different proteins.

In still another embodiment, binding of the oligonucleotide inactivates the target toward further cellular metabolic processes by recruiting cellular enzymes that degrades the mRNA. For instance, the oligonucleotide may comprise a segment of nucleosides that have the ability to recruit ribonuclease H (RNaseH) that degrades the RNA part of a DNA/RNA duplex. Likewise, the oligonucleotide may comprise a segment which recruits double stranded RNAses, such as for instance RNAseIII or it may comprise an external guide sequence (EGS) that recruit an endogenous enzyme ( RNase P) which degrades the target mRNA Also, the oligonucleotide may comprise a sequence motif which exhibit RNAse catalytic activity or moieties may be attached to the oligonucleotides which when brought into proximity with the target by the hybridization event disables the target from further metabolic activities.

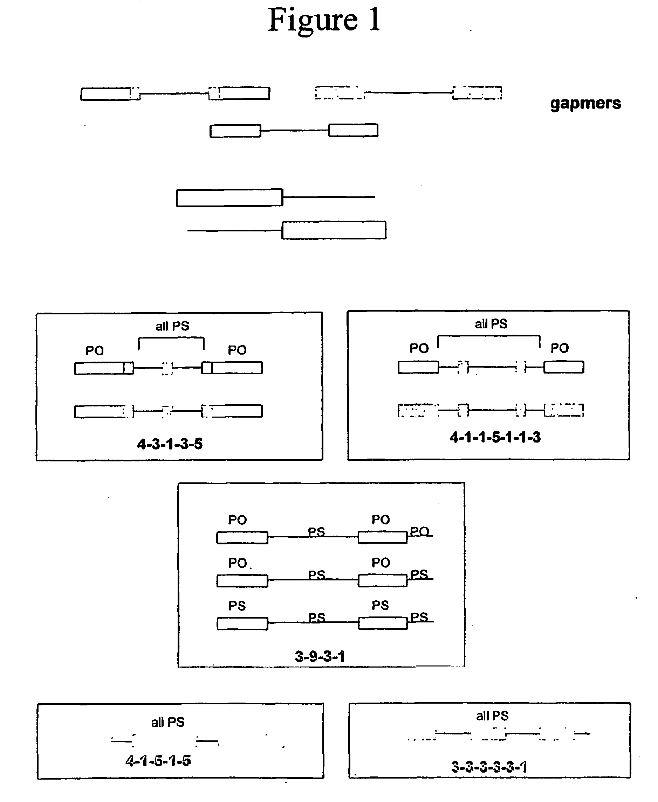

It has been shown that β-D-oxy-LNA does not support RNaseH activity. However, this can be changed according to the invention by creating chimeric oligonucleotides composed of β-D-oxy-LNA and DNA, called gapmers. A gapmer is based on a central stretch of 4-12 nt DNA or modified monomers recognizable and cleavable by the RNaseH (the gap) typically flanked by 1 to 6 residues of β-D-oxy-LNA (the flanks). The flanks can also be constructed with LNA derivatives. There are other chimeric constructs according to the invention that are able to act via an RNaseH mediated mechanism. A header is defined by a contiguous stretch of β-Doxy-LNA or LNA derivatives at the 5'-end followed by a contiguous stretch of DNA or modified monomers recognizable and cleavable by the RNaseH towards the 3'-end, and a talimer is defined by a contiguous stretch of DNA or modified monomers recognizable and cleavable by the RNaseH at the 5'-end followed by a contiguous stretch of β-D-oxy-LNA or LNA derivatives towards the 3'-end. Other chimeras according to the invention, called mixmers consisting of an alternate composition of DNA or modified monomers recognizable and cleavable by RNaseH and β-D-oxy-LNA and/or LNA derivatives might also be able to mediate RNaseH binding and cleavage. Since α-L-LNA recruits RNaseH activity to a certain extent, smaller gaps of DNA or modified monomers recognizable and, cleavable by the RNaseH for the gapmer construct might be required, and more flexibility in the mixmer construction might be introduced. Figure 1 shows an outline of different designs.

The clinical effectiveness of antisense oligonucleotides depends to a significant extent on their pharmacokinetics e.g. absorption, distribution, cellular uptake, metabolism and excretion. In turn these parameters are guided significantly by the underlying chemistry and the size and three-dimensional structure of the oligonucleotide.

As mentioned earlier, LNA is not a single, but several related chemistries, which although molecularly different all exhibit stunning affinity towards complementary DNA and RNA. Thus, the LNA family of chemistries is uniquely suited for development of oligos according to the invention with tailored pharmacokinetic properties exploiting either the high affinity of LNA to modulate the size of the active compounds or exploiting different LNA chemistries to modulate the exact molecular composition of the active compounds. In the latter case, the use of for instance amino-LNA rather than oxy-LNA will change the overall charge of the oligo and affect uptake and distribution behavior-Likewise the use of thio-LNA instead of oxy-LNA will increase the lipophilicity of the oligonucleotide and thus influence its ability to pass through lipophilic barriers such as for instance the cell membrane.

Modulating the pharmacokinetic properties of an LNA oligonucleotide according to the invention may further be achieved through attachment of a variety of different moieties. For instance, the ability of oligonucleotides to pass the cell membrane may be enhanced by attaching for instance lipid moieties such as a cholesterol moiety, a thioether, an aliphatic chain, a phospholipid or a polyamine to the oligonucleotide. Likewise, uptake of LNA oligonucleotides into cells may be enhanced by conjugating moieties to the oligonucleotide that interacts with molecules in the membrane, which mediates transport into the cytoplasm.

The pharmacodynamic properties can according to the invention be enhanced with groups that improve oligomer uptake, enhance biostability such as enhance oligomer resistance to degradation, and/or increase the specificity and affinity of oligonucleotides hybridisation characteristics with target sequence e.g. a mRMA sequence.

There are basically two types of toxicity associated with antisense oligos: sequence-dependant toxicity, involving the base sequence, and sequence-independent, class-related toxicity. With the exception of the issues related to immunostimulation by native CpG sequence motifs, the toxicities that have been the most prominent in the development of antisense oligonucleotide are independent of the sequence, e.g. related to the chemistry of the oligonucleotide and dose, mode, frequency and duration of administration. The phosphorothioates class of oligonucleotides have been particularly well characterized and found to elicit a number of adverse effects such as complement activation, prolonged PTT (partial thromboplastin time), thrombocytopenia, hepatotoxicity (elevation of liver enzymes), cardiotoxicity, splenomegaly and hyperplasia of reticuloendothelial cells.

As mentioned earlier, the LNA family of chemistries provides unprecedented affinity, very high bio-stablity and the ability to modulate the exact molecular composition of the oligonucleotide. In one embodiment of the invention, LNA containing compounds enable the development of oligonucleotides which combine high potency with little- if any-phosphorothioate linkages and which are therefore likely to display better efficacy and safety than contemporary antisense compounds.

Oligo- and polynucleotides of the invention may be produced using the polymerisation techniques of nucleic acid chemistry well known to a person of ordinary skill in the art of organic chemistry. Generally, standard oligomerisation cycles of the phosphoramidite wapproach (S. L. Beaucage and R. P. Iyer, Tetrahedron, 1993, 49, 6123; S. L. Beaucage and R. P. Iyer, Tetrahedron, 1992, 48, 2223) is used, but *e.g.* H-phosphonate chemistry, phosphortriester chemistry can also be used.

For some monomers, described in the Examples longer coupling time, and/or repeated couplings with fresh reagents, and/or use of more concentrated coupling reagents were used. The phosphoramidites employed coupled with satisfactory >95% step-wise coupling yields. Thiolation of the phosphate is performed by exchanging the normal, *e.g.* iodine/pyridine/H₂O, oxidation used for synthesis of phosphordiester oligomers with an oxidation using Beaucage's reagent (commercially available) other sulfurisation reagents are also comprised. The phosphorthioate LNA oligomers were efficiently synthesised with stepwise coupling yields >= 98 %.

The β-D-amino-LNA, β-D-thio-LNA oligonucleotides, α-L-LNA and β-D-methylamino-LNA oligonucleotides were also efficiently synthesised with step-wise coupling yields ≥ 98% using the phosphoramidite procedures.

Purification of LNA oligomeric compounds was done using disposable reversed phase purification cartridges and/or reversed phase HPLC and/or precipitation from ethanol or butanol. Capillary gel electrophoresis, reversed phase HPLC, MALDI-MS, and ESI-MS was used to verify the purity of the synthesized oligonucleotides. Furthermore, solid support materials having immobilised thereto an optionally nucleobase protected and optionally 5'-OH protected LNA are especially interesting as material for the synthesis of LNA containing oligomeric compounds where an LNA monomer is included in at the 3' end. In this instance, the solid support material is preferable CPG, *e.g.* a readily (commercially) available CPG material or polystyrene onto which a 3'-functionalised, optionally nucleobase protected and optionally 5'-OH protected LNA is linked using the conditions stated by the supplier for that particular material.

As it must be clear by now, an interesting aspect of the invention is directed to a compound of the invention or a conjugate of the invention for use as a medicament. As it must also be unambiguous by now, the use of a compound of the invention or as conjugate of the invention for the manufacture of a medicament for the treatment of cancer is a particularly interesting aspect of the invention.

The pharmaceutical composition according to the invention can be used for the treatment of many different diseases. For example survivin has been found to be overexpressed in human tumours of lung (Monzo et al.,1999, J. Clin. Oncol 17, 2100-2104), breast (Tanaka et al., 2000, Clin. Cancer Res. 6, 127-134; Nasu et al., 2002, Anticancer Res. 22, 1839-1844), colon/rectum (Kawasaki et al., 1998, Cancer Res. 58, 5071-5074; Rödel et al., 2002, Strahlenther. Onkol. 8, 426-434), stomach (Lu et al., 1998, Cancer Res. 58, 1808-1812; Tsuburaya et al., 2002, Hepatogastroenterology 49, 1150-1152), oesophagus (Kato et al., 2001, Int. J. Cancer 95, 92-95; Ikeguchi and Kaibara, 2002, Br. J. Cancer 87, 883-887), pancreas (Satoh et al., 2001, Cancer 92, 271-278; Sarela et al., 2002, Br. J. Cancer 86, 886-892), liver (Ikeguchi et al., 2002, Clin. Cancer Res. 8, 3131-3136), uterus (Saitoh et al., 1999, Int. J. Oncol. 15, 137-141; Takai et al., 2002, Cancer Lett. 184, 105-116), ovaries (Yoshoda et al., 2001, Int. J. Oncol. 19, 537-542; Takai et al., 2002, Int. J. Mol. Med. 10, 211-216), Hodgkin's disease (Garcia et al., 2003, Blood 101, 681-689), non-Hodgkin's lymphoma (Adida et al., 2000, Blood 96, 1921-1925.; Kuttler et al., 2002, Leukemia 16, 726-735), leukemias (Adida et al., 2000, Br. J. Haematol. 111, 196-203.; Kamihira et al., 2001, Br. J. Haematol. 114, 63-69; Mori et al., 2001, Int. J. Haematol. 75, 161-165), neuroblastoma (Islam et al., 2000, Oncogene 19, 617-623; Adida et al., 1998, Lancet 351, 882-883), phaeochromocytoma (Koch et al., 2002, Eur. J. Endocrinol. 146, 381-388), soft tissue sarcomas (Würl et al., 2002, Lancet 359, 943-945), gliomas (Chakravarti et al. 2002, J. Clin. Oncol. 20, 1063-1068), melanoma (Grossman et al., 1999, J. Invest. Dermatol. 113, 1076-1081), bladder (Swana et al., 1999, New Engl. J. Med. 341, 452-453; Smith et al., 2001, JAMA 285, 324-328), cervix (Kim et al., 2002, Anticancer Res. 22, 805-808; Yoshida et al., 2003, Oncol. Rep. 10, 45-49), prostate (Ambrosini et al., 1997, Nat. Med. 3, 917-921). Like cancer cells proliferating vascular endothelial cells are sensitive to downregulation of survivin expression. The pharmaceutical composition according to the invention can therefore be used in the treatmens of diseases characterized by abnormal disease causing angiogenesis. Examples of such diseases are cancers in general and artherosclerosis, psoriasis, diabetic retinopathy, rheumatoid arthritis, asthma, warts, allergic dermatitis and Karposis sarcoma. Furthermore, survivin may be actively involved in regulating cell viability during HIV-1 infection (Zhu et al., 2003, Apoptosis 8, 71-79). Survivin is essential to the correct execution of mitosis and completion of cell division. Downregulation of survivin should therefore be relevant in the treatment of any disease characterized by uncontrolled or abnormal cell growth.

Generally stated, one aspect the component of the invention is used in a method of treating a mammal suffering from or susceptible to a disease caused by abnormal angiogenesis, comprising administering to the mammal an therapeutically effective amount of an oligonucleotide targeted to survivin that comprises one or more LNA units.

An interesting aspect of the invention is directed to the use of a compound as defined herein or as conjugate as defined herein for the preperation of a medicament for the treatment of artherosclerosis, psoriasis, diabetic retinopathy, rheumatoid arthritis, asthma, warts and allergic dermatitis.

The compounds of the invention are preferably employed for treatment or prophylaxis against diseases caused by cancer, particularly for treatment of cancer as may occur in tissue such as lung, breast, colon, prostate, pancreas, liver, brain, testes, stomach, intestine, bowel, spinal cord, sinuses, urinary tract or ovaries cancer.

Furthermore, the compounds of the invention described herein may be used in a method of preventing or treating cancer comprising a therapeutically effective amount of a survivin modulating oligomeric compound, including but not limited to high doses of the oligomer, to a human in need of such therapy. The compounds of the invention further may be used for a short period of administration of a survivin modulating oligomeric compound. Normal, non-cancerous cells divide at a frequency characteristic for the particular cell type. When a cell has been transformed into a cancerous state, uncontrolled cell proliferation and reduced cell death results, and therefore, promiscuous cell division or cell growth is a hallmark of a cancerous cell type. Examples of types of cancer, include, but are not limited to, non-Hodgkin's lymphoma, Hodgkin's lymphoma, leukemia (e.g., acute leukemia such as acute lymphocytic leukemia, acute myelocytic leukemia, chronic myeloid leukemia, chronic lymphocytic leukemia, multiple myeloma), colon carcinoma, rectal carcinoma, pancreatic cancer, breast cancer, ovarian cancer, prostate cancer, renal cell carcinoma, hepatoma, bile duct carcinoma, choriocarcinoma, cervical cancer, testicular cancer, lung carcinoma, bladder carcinoma, melanoma, head and neck cancer, brain cancer, cancers of unknown primary site, neoplasms, cancers of the peripheral nervous system, cancers of the central nervous system, tumors (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, seminoma, embryonal carcinoma, Wilms' tumor, small cell lung carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, and retinoblastoma), heavy chain disease, metastases, or any disease or disorder characterized by uncontrolled or abnormal cell growth.

In the use of a compound of the invention or as conjugate of the invention for the manufacture of a medicament for the treatment of cancer, said cancer may suitably be in the form of a solid tumor. Furthermore, said cancer is also suitably a carcinoma. The carcinoma is typically in the from selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumors. More typically, said carcinoma is selected from the group consisting of malignant melanoma, non-small cell lung cancer, breast carcinoma, colon carcinoma and renal cell carcinoma. The malignant melanoma is typically selected from the group consisting of superficial spreading melanoma, nodular melanoma, lentigo maligna melanoma, acral melagnoma, amelanotic melanoma and desmoplastic melanoma.

Alternatively, the cancer may suitably be a sarcoma. The sarcoma is typically in the form selected from the group consisting of osteosarcoma, Ewing's sarcoma, chondrosarcoma, malignant fibrous histiocytoma, fibrosarcoma and Kaposi's sarcoma.

Alternatively, the cancer may suitably be a glioma.

It should be understood that the invention also relates to a pharmaceutical composition, which comprises a least one antisense oligonucleotide construct of the invention as an active ingredient. It should be understood that the pharmaceutical composition according to the invention optionally comprises a pharmaceutical carrier, and that the pharmaceutical composition optionally comprises further antisense compounds, chemotherapeutic compounds, anti-inflammatory compounds, antiviral compounds and/or immuno-modulating compounds.

The oligomeric compound comprised in this invention can be employed in a variety of pharmaceutically acceptable salts. As used herein, the term refers to salts that retain the desired biological activity of the herein identified compounds and exhibit minimal undesired toxicological effects. Non-limiting examples of such salts can be formed with organic amino acid and base addition salts formed with metal cations such as zinc, calcium, bismuth, barium, magnesium, aluminum, copper, cobalt, nickel, cadmium, sodium, potassium, and the like, or with a cation formed from ammonia, *N,N*-dibenzylethylene-diamine, *D-*glucosamine, tetraethylammonium, or ethylenediamine; or (c) combinations of (a) and (b); e.g., a zinc tannate salt or the like.

In one embodiment of the invention the oligomeric compound may be in the form of a pro-drug. Oligonucleotides are by virtue negatively charged ions. Due to the lipophilic nature of cell membranes the cellular uptake of oligonucleotides are reduced compared to neutral or lipophilic equivalents. This polarity "hindrance" can be avoided by using the pro-drug approach (see e.g. Crooke, R. M. (1998) in Crooke, S. T. Antisense research and Application. Springer-Verlag, Berlin, Germany, vol. 131, pp. 103-140). In this approach the oligonucleotides are prepared in a protected manner so that the oligo is neutral when it is administered. These protection groups are designed in such a way that so they can be removed then the oligo is taken up be the cells. Examples of such protection groups are S-acetylthioethyl (SATE) or S-pivaloylthioethyl (t-butyl-SATE). These protection groups are nuclease resistant and are selectively removed intracellulary.

In one embodiment of the invention the oligomeric compound is linked to ligands/conjugates. It is way to increase the cellular uptake of antisense oligonucleotides. This conjugation can take place at the terminal positions 5'/3'-OH but the ligands may also take place at the sugars and/or the bases. Other examples of conjugates/lingands are cholesterol moieties, duplex intercalators such as acridine, poly-L-lysine, "end-capping" with one or more nuclease-resistant linkage groups.

The invention also includes the formulation of one or more oligonucleotide compound as disclosed herein. Pharmaceutically acceptable binding agents and adjuvants may comprise part of the formulated drug. Capsules, tablets and pills etc. may contain for example the following compounds: microcrystalline cellulose, gum or gelatin as binders; starch or lactose as excipients; stearates as lubricants; various sweetening or flavouring agents. For capsules the dosage unit may contain a liquid carrier like fatty oils. Likewise coatings of sugar or enteric agents may be part of the dosage unit. The oligonucleotide formulations may also be emulsions of the active pharmaceutical ingredients and a lipid forming a micellular emulsion. An oligonucleotide of the invention may be mixed with any material that do not impair the desired action, or with material that supplement the desired action. These could include other drugs including other nucleotide compounds. For parenteral, subcutaneous, intradermal or topical administration the formulation may include a sterile diluent, buffers, regulators of tonicity and antibacterials. The active compound may be prepared with carriers that protect against degradation or immediate elimination from the body, including implants or microcapsules with controlled release properties. For intravenous administration the preferred carriers are physiological saline or phosphate buffered saline. Preferably, an oligomeric compound is included in a unit formulation such as in a pharmaceutically acceptable carrier or diluent in an amount sufficient to deliver to a patient a therapeutically effective amount without causing serious side effects in the treated patient.

The pharmaceutical compositions of the present invention may be administered in a number of ways depending upon whether local or systemic treatment is desired and upon the area to be treated. Administration may be (a) oral (b) pulmonary, e.g., by inhalation or insufflation of powders or aerosols, including by nebulizer; intratracheal, intranasal, (c) topical including epidermal, transdermal, ophthalmic and to mucous membranes including vaginal and rectal delivery; or (d) parenteral including intravenous, intraarterial, subcutaneous, intraperitoneal or intramuscular injection or infusion; or intracranial, e.g., intrathecal or intraventricular, administration. In one embodiment the active oligo is administered IV, IP, orally, topically or as a bolus injection or administered directly in to the target organ. Pharmaceutical compositions and formulations for topical administration may include transdermal patches, ointments, lotions, creams, gels, drops, sprays, suppositories, liquids and powders. Conventional pharmaceutical carriers, aqueous, powder or oily bases, thickeners and the like may be necessary or desirable. Coated condoms, gloves and the like may also be useful. Preferred topical formulations include those in which the oligonucleotides of the invention are in admixture with a topical delivery agent such as lipids, liposomes, fatty acids, fatty acid esters, steroids, chelating agents and surfactants. Compositions and formulations for oral administration include but is not restricted to powders or granules, microparticulates, nanoparticulates, suspensions or solutions in water or non-aqueous media, capsules, gel capsules, sachets, tablets or minitablets. Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self- emulsifying solids and self-emulsifying semisolids. Delivery of drug to tumour tissue may be enhanced by carrier-mediated delivery including, but not limited to, cationic liposomes, cyclodextrins, porphyrin derivatives, branched chain dendrimers, polyethylenimine polymers, nanoparticles and microspheres (Dass CR. J Pharm Pharmacol 2002; 54(1):3-27). The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product. The compositions of the present invention may be formulated into any of many possible dosage forms such as, but not limited to, tablets, capsules, gel capsules, liquid syrups, soft gels and suppositories. The compositions of the present invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers. Oligomeric compounds of the invention may also be conjugated to active drug substances, for example, aspirin, ibuprofen, a sulfa drug, an antidiabetic, an antibacterial or an antibiotic.

LNA containing oligomeric compound are useful for a number of therapeutic applications as indicated above. In general, therapeutic methods of the invention include administration of a therapeutically effective amount of an LNA-modified oligonucleotide to a mammal, particularly a human. In a certain embodiment, the present invention provides pharmaceutical compositions containing (a) one or more antisense compounds and (b) one or more other chemotherapeutic agents which function by a non-antisense mechanism. When used with the compounds of the invention, such chemotherapeutic agents may be used individually (e.g. mithramycin and oligonucleotide), sequentially (e.g. mithramycin and oligonucleotide for a period of time followed by another agent and oligonucleotide), or in combination with one or more other such chemotherapeutic agents or in combination with radiotherapy. All chemotherapeutic agents known to a person skilled in the art are here incorporated as combination treatments with compound according to the invention. Anti-inflammatory drugs, including but not limited to nonsteroidal anti- inflammatory drugs and corticosteroids, antiviral drugs, and immuno-modulating drugs may also be combined in compositions of the invention. Two or more combined compounds may be used together or sequentially.

Accordingly, a further aspect of the invention is directed to the use of a compound as defined herein or as conjugate as defined herein for the manufacture of a medicament for the treatment of cancer, wherein said medicament further comprises a chemotherapeutic agent selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine). Suitably, the further chemotherapeutic agent is selected from taxanes such as Taxol, Paclitaxel or Docetaxel.

Similarly, the invention is further directed to the use of a compound as defined herein or as conjugate as defined herein for the manufacture of a medicament for the treatment of cancer, wherein said treatment further comprises the administration of a further chemotherapeutic agent selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine). Suitably, said treatment further comprises the administration of a further chemotherapeutic agent selected from taxanes, such as Taxol, Paclitaxel or Docetaxel.

Alternatively stated, the compound of the invention may be used in a method for treating cancer, said method comprising administering a compound as defined herein, or a conjugate as defined herein or a pharmaceutical composition as defined herein to a patient in need thereof and further comprising the administration of a a further chemotherapeutic agent. Said further administration may be such that the further chemotherapeutic agent is conjugated to the compound of the invention, is present in the pharmaceutical composition, or is administered in a separate formulation.

In another embodiment, compositions of the invention may contain one or more antisense compounds, particularly oligonucleotides, targeted to a first nucleic acid and one or more additional antisense compounds targeted to a second nucleic acid target. Two or more combined compounds may be used together or sequentially.

In a preferred embodiment the present invention provides pharmaceutical compositions containing (a) one or more antisense compounds and (b) one or more other chemotherapeutic agents which stabilize microtubules or dampen microtubule dynamic - and therby prevent tension forming at the kinetochores of sister chromatids. Such chemotherapeutic agents includes taxanes, in particular Taxol, Paclitaxel and Docetaxel. When used with the compounds of the invention, such chemotherapeutic agents should be used sequentially initiating with oligonucleotide treatment for a period of time which sensitises the target cells to subsequent co-treatment with the chemotherapeutic agent by reducing the level of survivin protein in tumor cells and proliferating endothelial cells of the tumor vasculature.

In another preferred embodiment the present invention provides pharmaceutical compositions containing (a) one or more antisense compounds and (b) radiation therapy. When used with the compounds of the invention, radiation therapy should be used sequentially initiating with oligonucleotide treatment for a period of time which sensitises the target cells to subsequent additional radiotherapy by reducing the level of survivin protein in tumor cells and proliferating endothelial cells of the tumor vasculature.

Dosing is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient. Optimum dosages may vary depending on the relative potency of individual oligonucleotides.

Generally it can be estimated based on EC50s found to be effective in *in vitro* and *in vivo* animal models. In general, dosage is from 0.01 µg to 1 g per kg of body weight, and may be given once or more daily, weekly, monthly or yearly, or even once every 2 to 10 years or by continuous infusion for hours up to several months. The repetition rates for dosing can be estimated based on measured residence times and concentrations of the drug in bodily fluids or tissues. Following successful treatment, it may be desirable to have the patient undergo maintenance therapy to prevent the recurrence of the disease state.

The LNA containing oligomeric compounds of the present invention can also be utilized for as research reagents for diagnostics, therapeutics and prophylaxis. In research, the antisense oligonucleotides may be used to specifically inhibit the synthesis of survivin genes in cells and experimental animals thereby facilitating functional analysis of the target or an appraisal of its usefulness as a target for therapeutic intervention. In diagnostics the antisense oligonucleotides may be used to detect and quantitate survivin expression in cell and tissues by Northern blotting, in-situ hybridisation or similar techniques. For therapeutics, an animal or a human, suspected of having a disease or disorder, which can be treated by modulating the expression of survivin is treated by administering antisense compounds in accordance with this invention. Further provided are methods of treating an animal particular mouse and rat and treating a human, suspected of having or being prone to a disease or condition, associated with expression of survivin by administering a therapeutically or prophylactically effective amount of one or more of the antisense compounds or compositions of the invention.

A further aspect of the invention is directed to a method of preventing or limiting apoptosis comprising the adminisration of a compound as herein, a conjugate as defined herein or a pharmaceutical composition as defined herein. The prevention of apoptosis may be in vitro. The prevention may be done on a cellular assay or within a tissue sample.

A related aspect of the invention is directed method of preventing cellular proliferation comprising the adminisration of a compound as defined herein, a conjugate as defined herein or a pharmaceutical composition as defined herein. The prevention of proliferation may be in vitro. The prevention may be done on a cellular assay or within a tissue sample.

The invention is further illustrated in a non-limiting manner by the following examples.

### EXAMPLES (Examples referring to compounds other than SEQ ID No 130A, SEQ ID No 130B and SEQ ID No 130C are for reference only)

### Example 1: Monomer synthesis

The LNA monomer building blocks and derivatives thereof were prepared following published procedures and references cited therein, see:
- WO 03/095467 A1
- D. S. Pedersen, C. Rosenbohm, T. Koch (2002) Preparation of LNA Phosphoramidites, Synthesis 6, 802-808.
- M. D. Sorensen, L. Kværnø, T. Bryld, A. E. Håkansson, B. Verbeure, G. Gaubert, P. Herdewijn, J. Wengel (2002) α-L-ribo-configured Locked Nucleic Acid (α-I-LNA): Synthesis and Properties, J. Am. Chem. Soc., 124, 2164-2176.
- S. K. Singh, R. Kumar, J. Wengel (1998) Synthesis of Novel Bicyclo[2.2.1] Ribonucleosides: 2'-Amino- and 2'-Thio-LNA Monomeric Nucleosides, J. Org. Chem. 1998, 63, 6078-6079.
- C. Rosenbohm, S. M. Christensen, M. D. Sørensen, D. S. Pedersen, L. E. Larsen, J. Wengel, T. Koch (2003) Synthesis of 2'-amino-LNA: a new strategy, Org. Biomol. Chem. 1, 655-663.

Synthesis of the 2'-thio-LNA ribothymidine phosphoramidite. *Reagents and conditions:* i) Pd/C, H₂, acetone, MeOH; ii) BzCl, pyridine, DMF; iii) 0.25 M H₂SO₄ (aq), DMF, 80°C (79% from **4**; 3 steps); iv) Tf₂O, DMAP, CH₂Cl₂, 0°C; v) Na₂S, DMF (72% from **7**; 2 steps); vi) NaOBz, DMF, 100°C (81%); vii) NH₃, MeOH (76%); viii) DMT-Cl, pyridine (88%); ix) P(OCH₂CH₂CN)(N(*ⁱ*Pr)₂)₂, 4,5-dicyanoimidazole, CH₂Cl₂ (99%). DMT= 4,4'-dimethoxytrityl, PN₂= 2-cyanoethoxy(diisopropylamino)phosphinoyl.

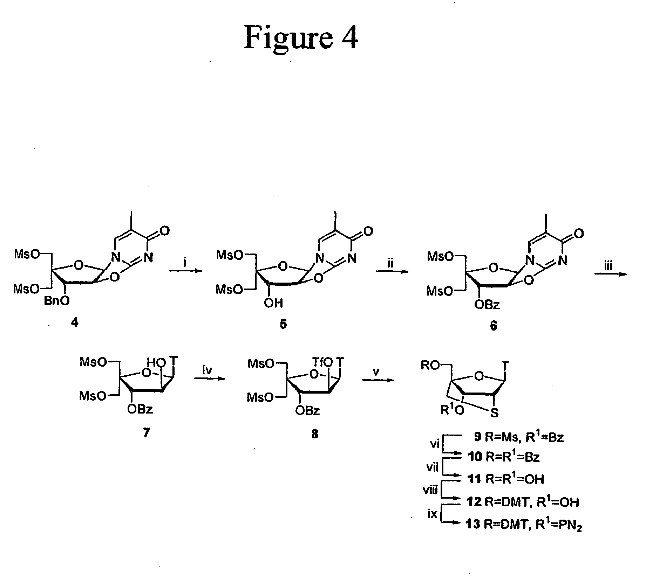

### 1-(3-O-Benzoyl-5-O-methanesulfonyl-4-C-methanesulfonyloxymethyl-β-D-threo-pentofuranosyl)thymine (7, Figure 4)

Anhydro-nucleoside **4** (C. Rosenbohm, S. M. Christensen, M. D. Sørensen, D. S. Pedersen, L. E. Larsen, J. Wengel, T. Koch (2003) Synthesis of 2'-amino-LNA: a new strategy, Org. Biomol. Chem. 1, 655-663) (30.0 g, 58.1 mmol) was heated to 70°C in a mixture of methanol (1000 cm³) and acetone (1000 cm³) until a clear solution was obtained and the solution was allowed to reach room temperature. The reaction flask was flushed with argon and Pd/C (10 wt.% Pd on carbon, 6.2 g, 5.8 mmol) was added. The mixture was stirred vigorously under an atmosphere of hydrogen gas (balloon). After 23 h the slurry was filtered through a pad of celite. The catalyst was recovered from the celite and refluxed in DMF (1000 cm³) for 1 h. The hot DMF slurry was filtered through a pad of celite and the organic layers combined and evaporated *in vacuo* to give nucleoside **5** as a yellow powder. Residual solvents were removed on a high vacuum pump overnight.

The crude nucleoside **5** (23 g) was heated to 70°C in DMF (300 cm³) to give a clear yellow solution that was allowed to cool to room temperature. Benzoyl chloride (81.7 g, 581 mmol, 67.4 cm³) was added followed by pyridine (70 cm³). After 18 h the reaction was quenched with methanol (200 cm³) and excess methanol was removed *in vacuo.*
To the dark brown solution of nucleoside **6** aqueous H₂SO₄ (0.25 M, 400 cm³) was added. The solution was heated to 80°C on an oil bath (At approx 50°C precipitation occurs. The solution becomes clear again at 80 °C). After 22 h at 80°C the solution was allowed to cool to room temperature. The reaction mixture was transferred to a separatory funnel with ethyl acetate (1000 cm³). The organic layer was washed with sat. aq NaHCO₃ (2 x 1000 cm³). The combined aqueous layers were extracted with ethyl acetate (1000 + 500 cm³). The organic layers were combined and washed with sat. aq NaHCO₃ (1000 cm³), dried (Na₂SO₄), filtered and evaporated *in vacuo* to give a yellow liquid. Residual solvents were removed on a high vacuum pump overnight to give a yellow syrup. The product was purified by Dry Column Vacuum Chromatography (id 10 cm; 100 cm³ fractions; 50-100% EtOAc in n-heptane (v/v) - 10% increments; 2-24% MeOH in EtOAc (v/v) - 2% increments). Fractions containing the product were combined and evaporated *in vacuo* giving nucleoside **7** (25.1 g, 79%) as a white foam.
R_{f} = 0.54 (5% MeOH in EtOAc, v/v);
ESI-MS m/z found 549.0 ([MH]⁺, calcd 549.1);
¹H NMR (DMSO-d₆) δ 11.39 (br s, 1H, NH), 8.10-8.08 (m, 2H, Ph), 7.74-7.70 (m, 1H, Ph), 7.60-7.56 (m, 2H, Ph), 7.51 (d, *J* = 1.1 Hz, 1H, H6), 6.35 (d, *J* = 4.9 Hz, 1H, H1'), 6.32 (d, *J* = 5.3 Hz, 1H, 2'-OH), 5.61 (d, *J* = 4.0 Hz, 1H, H3'), 4.69 (d, *J* = 10.8 Hz, 1H), 4.59 (m, 1H, H2'), 4.55 (d, *J* = 10.8 Hz, 1H), 4.52 (d, *J* = 10.8 Hz, 1H), 4.46 (d, *J* = 10.6 Hz, 1H) (H5' and H1"), 3.28 (s, 3H, Ms), 3.23 (s, 3H, Ms), 1.81 (s, 3H, CH₃);
¹³C NMR (DMSO-*d*₆) δ 164.5, 163.6 (C4, PhC(O)), 150.3 (C2), 137.7 (C6), 133.8, 129.6, 128.7, 128.6 (Ph), 108.1 (C5), 84.8 (C1'), 81.1 (C4'), 78.0 (C3'), 73.2 (C2'), 68.0, 67.1 (C5', C1"), 36.7, 36.6 (2 x Ms), 11.9 (CH₃);
Elemental anal. calcd for C₂₀H₂₄N₂O₁₂S₂·0.33 H₂O (%): C, 44.34; H, 4.65; N, 4.85. Found: C, 44.32; H, 4.58; N, 4.77.

### (1R,3R,4R,7R)-7-Benzoyloxy-1-methansulfonyloxymethyl-3-(thymin-1-yl)-2-oxa-5-thiabicyclo[2:2:1]heptane (9)

1-(3-*O*-Benzoyl-5-*O*-methanesulfonyl-4-*C*-methanesulfonyloxymethyl-β-D-*threo*pentofuranosyl)thymine (**7**) (10.00 g, 18.23 mmol) was dissolved in dichloromethane (500 cm³) and cooled to 0 °C. Pyridine (15 cm³) and DMAP (8.91 g, 72.9 mmol) was added followed by dropwise addition of trifluoromethanesulfonic anhydride (10.30 g, 36.5 mmol, 6.0 cm³). After 1 h the reaction was quenched with sat. aq NaHCO₃ (500 cm³) and transferred to a separatory funnel. The organic layer was washed with 1.0 M aq HCl (500 cm³), sat. aq NaHCO₃ (500 cm³) and brine (500 cm³). The organic layer was evaporated *in vacuo* with toluene (100 cm³) to give 1-(3-*O*-benzoyl-5-*O*-methanesulfonyl-4-*C-*methanesulfonyloxymethyl-2-*O*-trifluoromethanesulfonyl-β-D-*threo-*pentofuranosyl)thymine (**8**) as a yellow powder.
The crude nucleoside **8** was dissolved in DMF (250 cm³) and Na₂S (1.57 g, 20.1 mmol) was added to give a dark green slurry. After 3 h the reaction was quenched with half sat. aq NaHCO₃ (500 cm³) and extracted with dichloromethane (500 + 2 × 250 cm³). The combined organic layers were washed with brine (500 cm³), dried (Na₂SO₄), filtered and concentrated *in vacuo* to give a yellow liquid. Residual solvent was removed overnight on a high vacuum pump to give a yellow gum that was purified by Dry Column Vacuum Chromatography (id 6 cm: 50 cm³ fractions; 50-100% EtOAc in *n*-heptane (v/v) - 10% increments; 2-20% MeOH in EtOAc (v/v) - 2% increments) to give nucleoside **9** (6.15 g, 72%) as a yellow foam.
R_{f} = 0.27 (20% n-heptane in EtOAc, v/v);
ESI-MS *m*/*z* found 469.0 ([MH]⁺, calcd 469.1);
¹H NMR (CDCl₃) δ 8.70 (br s, 1H, NH), 8.01-7.99 (m, 2H, Ph), 7.67 (d, *J*= 1.1 Hz, 1H, H6), 7.65-7.61 (m, 1H, Ph), 7.50-7.46 (m, 2H, Ph), 5.98 (s, 1H, H1'), 5.34 (d, *J* = 2.4 Hz, 1H, H3'), 4.66 (d, *J* = 11.7 Hz, 1H, H5'a), 4.53 (d, J = 11.5 Hz, 1H, H5'b), 4.12 (m (overlapping with residual EtOAc), 1H, H2'), 3.15-3.13 (m, 4H, H1"a and Ms), 3.06 (d, *J* = 10.6 Hz, 1H, H1"b), 1.98 (d, *J* = 1.1 Hz, 3H, CH₃);
¹³C NMR (CDCl₃) δ 165.2, 163.5 (C4, PhC(O)), 149.9 (C2), 134.1, 133.9, 129.8, 128.7, 128.3 (C6, Ph), 110.7 (C5), 91.1 (C1'), 86.8 (C4'), 72.6 (C3'), 65.8 (C5'), 50.5 (C2'), 37.9 (Ms), 35.1 (C1"), 12.5 (CH₃);
Elemental anal. calcd for C₁₉H₂₀N₂O₈S₂·0.33 EtOAc (%): C, 49.21; H, 4.72; N, 5.47. Found: C, 49.25; H, 4.64; N, 5.48.

### (1R,3R,4R,7R)-7-Benzoyloxy-1-benzoyloxymethyl-3-(thymin-1-yl)-2-oxa-5-thiabicyclo[2:2:1]heptane (10)

Nucleoside **9** (1.92 g, 4.1 mmol) was dissolved in DMF (110 cm³). Sodium benzoate (1.2 g, 8.2 mmol) was added and the mixture was heated to 100°C for 24 h. The reaction mixture was transferred to a separatory funnel with half sat. brine (200 cm³) and extracted with ethyl acetate (3 × 100 cm³). The combined organic layers were dried (Na₂SO₄), filtered and evaporated *in vacuo* to give a brown liquid. The product was put on a high vacuum pump to remove residual solvent. The resulting brown gum was purified by Dry Column Vacuum Chromatography (id 4 cm; 50 cm³ fractions; 0-100% EtOAc in n-heptane (v/v) - 10% increments; 2-10% MeOH in EtOAc (v/v) - 2% increments) to give nucleoside **10** (1.64 g, 81%) as a slightly yellow foam.
R_{f} = 0.57 (20% n-heptane in EtOAc, v/v);
ESI-MS m/z found 495.1 ([MH]⁺, calcd 495.1);
¹H NMR (CDCl₃) δ 9.02 (br s, 1H, NH), 8.07-7.99 (m, 4H, Ph), 7.62-7.58 (m, 2H, Ph), 7.47-7.42 (m, 5H, Ph and H6), 5.95 (s, 1H, H1'), 5.46 (d, *J* = 2.2 Hz, 1H, H3'), 4.93 (d, *J* = 12.8 Hz, 1H, H5'a), 4.60 (d, *J* = 12.8 Hz, 1H, H5'b), 4.17 (d, *J* = 2.2 Hz, 1H, H2'), 3.27 (d, *J* = 10.6 Hz, 1H, H1"a), 3.16 (d, *J* = 10.6 Hz, 1H, H1"b), 1.55 (d, *J* = 1.1 Hz, 3H, CH₃);
¹³C NMR (CDCl₃) δ 165.8, 165.1, 163.7 (C4, 2 x PhC(O)), 150.0 (C2), 133.9, 133.7, 133.6, 129.8, 129.6, 129.0, 128.8, 128.6, 128.5 (C6, 2 x Ph), 110.3 (C5), 91.3 (C1'), 87.5 (C4'), 72.9 (C3'), 61.3 (C5'), 50.6 (C2'), 35.6 (C1"), 12.3 (CH₃);
Elemental anal. calcd for C₂₅H₂₂N₂O₇S (%): C, 60.72; H, 4.48; N, 5.66. Found: C, 60.34; H, 4.49; N, 5.35.

### (1R,3R,4R,7R)-7-Hydroxy-1-hydroxymethyl-3-(thymin-1-yl)-2-oxa-5-thiabicyclo[2:2:1]heptane (11)

Nucleoside **10** (1.50 g, 3.0 mmol) was dissolved in methanol saturated with ammonia (50 cm³). The reaction flask was sealed and stirred at ambient temperature for 20 h. The reaction mixture was concentrated *in vacuo* to give a yellow gum that was purified by Dry Column Vacuum Chromatography (id 4 cm; 50 cm³ fractions; 0-16% MeOH in EtOAc (v/v) - 1% increments) giving nucleoside **11** (0.65 g, 76%) as clear needles.
R_{f} = 0.31 (10% MeOH in EtOAc, v/v);
ESI-MS m/z found 287.1 ([MH]⁺, calcd 287.1);
¹H NMR (DMSO-*d₆*) δ 11.32 (br s, 1H, NH), 7.96 (d, *J* = 1.1 Hz, 1H, H6), 5.95 (s, 1H, H6), 5.70 (d, *J* = 4.2 Hz, 1H, 3'-OH), 5.62 (s, 1H, H1'), 4.49 (t, *J* = 5.3 Hz, 1H, 5'-OH), 4.20 (dd, *J* = 4.1 and 2.1 Hz, 1H, H3'), 3.77-3.67 (m, 2H, H5'), 3.42 (d, J = 2.0 Hz, 1H, H2'), 2.83 (d, *J* = 10.1 Hz, 1H, H1"a), 2.64 (d, *J* = 10.1 Hz, 1H, H1"b), 1.75 (d, *J* = 1.1 Hz, 3H, CH₃);
¹³C NMR (DMSO-*d₆*) δ 163.8 (C4), 150.0 (C2), 135.3 (C6), 107.5 (C5), 90.2, 89.6 (C1' and C4'), 69.4 (C3'), 58.0 (C5'), 52.1 (C2'), 34.6 (C1"), 12.4 (CH₃);
Elemental anal. calcd for C₁₁H₁₄N₂O₅S (%): C, 46.15; H, 4.93; N, 9.78. Found: C, 46.35; H, 4.91; N, 9.54.

### (1R,3R,4R,7R)-1-(4,4'-Dimethoxytrityloxymethyl)-7-hydroxy-5-methyl-3-(thymin-1-yl)-2-oxa-5-thiabiryclo[2:2:1]heptane (12)

Nucleoside **11** (0.60 g, 2.1 mmol) was dissolved in pyridine (10 cm³). 4,4'-Dimethoxytrityl chloride (0.88 g, 2.6 mmol) was added and the reaction was stirred at ambient temperature for 3 h. The reaction mixture was transferred to a separatory funnel with water (100 cm³) and extracted with ethyl acetate (100 + 2 x 50 cm³). The combined organic layers were washed with sat. aq NaHCO₃ (100 cm³), brine (100 cm³) and evaporated to dryness *in vacuo* to give a viscous yellow liquid. The product was redissolved in toluene (50 cm³) and concentrated *in vacuo* to give a yellow foam. The foam was dried on a high vacuum pump overnight and purified by Dry Column Vacuum Chromatography (id 4 cm: 50 cm³ fractions; 10-100% EtOAc in n-heptane (v/v) - 10% increments) giving nucleoside **12** (1.08 g, 88%) as a white foam.
R_{f} = 0.24 (20% n-heptane in EtOAc, v/v);
ESI-MS m/z found 587.1 ([M-H]⁺, calcd 587.2);
¹H NMR (CDCl₃) δ 8.96 (br s, 1H, NH), 7.74 (d, *J* = 1.1 Hz, 1H, H6), 7.46-7.44 (m, 2H, Ph), 7.35-7.22 (m; 9H, Ph), 7.19-7.15 (m, 2H, Ph), 6.86-6.80 (m, 2H, Ph), 5.82 (s, 1H, H1'), 4.55 (dd, *J* = 9.3 and 2.1 Hz, 1H, H3'), 3.79 (s, 6H, OCH₃), 3.71 (d, *J* = 2.0 Hz, 1H, H2'), 3.50 (s, 2H, H5'), 2.81 (d, *J* = 10.8 Hz, 1H, H1"a), 2.77 (d, *J* = 10.8 Hz, 1H, H1"b), 2.69 (d, *J* = 9.2 Hz, 1H, 3'-OH), 1.42 (s, 3H, CH₃);
¹³C NMR (CDCl₃) δ 158.7 (C4), 150.1 (C2), 144.1, 135.2, 135.1, 130.1, 129.1, 128.1, 128.0, 127.1, 127.0, 113.3 (C6, 3 x Ph), 110.0 (C5), 90.2 (C(Ph)₃), 89.6 (C1'), 87.0 (C4'), 71.7 (C3'), 60.9 (C5'), 55.2 (C2'), 34.7 (C1"), 12.2 (CH₃);
Elemental anal. calcd for C₃₂H₃₂N₂O₇S·0.5 H₂O (%): C, 64.31; H, 5.57; N, 4.69. Found: C, 64.22; H, 5.67; N, 4.47.

### (1R,3R,4R,7R)-7-(2-Cyanoethoxy(diisopropylamino)phosphinoxy)-1-(4,4'-dimethoxytrityloxymethyl)-3-(thymin-1-yl)-2-oxa-5-thiabicyclo[2.2.1]heptane (13)

According to the published method (D. S. Pedersen, C. Rosenbohm, T. Koch (2002) Preparation of LNA Phosphoramidites, Synthesis, 6, 802-808) nucleoside **12** (0.78 g, 1.33 mmol) was dissolved in dichloromethane (5 cm³) and a 1.0 M solution of 4,5-dicyanoimidazole in acetonitrile (0.93 cm³, 0.93 mmol) was added followed by dropwise addition of 2-cyanoethyl-*N*,*N*,*N*',*N*'-tetraisopropylphosphorodiamidite (0.44 cm³, 1.33 mmol). After 2 h the reaction was transferred to a separatory funnel with dichloromethane (40 cm³) and washed with sat. aq NaHCO₃ (2 x 25 cm³) and brine (25 cm³). The organic layer was dried (Na₂SO₄), filtered and evaporated *in vacuo* to give nucleoside **13** (1.04 g, 99%) as a white foam. R_{f} = 0.29 and 0.37 - two diastereoisomers (20% n-heptane in EtOAc, v/v); ESI-MS m/z found 789.3 ([MH]⁺, calcd 789.3); ³¹P NMR (DMSO-d₆) δ150.39, 150.26.

### Example 2: Oligonucleotide synthesis

Oligonucleotides were synthesized using the phosphoramidite approach on an Expedite 8900/MOSS synthesizer (Multiple Oligonucleotide Synthesis System) at 1 or at 15 µmol. At the end of the synthesis (DMT-on) the oligonucleotides were cleaved from the solid support using aqueous ammonia for 1 h at room temperature, and further deprotected for 3 h at 65°C. The oligonucleotides were purified by reverse phase HPLC (RP-HPLC). After the removal of the DMT-group, the oligonucleotides were characterized by IE-HPLC or RP-HPLC. The identity of the oligonucleotides is confirmed by ESI-MS. See below for more details.

### Preparation of the LNA succinyl hemiester

5'-O-Dmt-3'-hydroxy-LNA monomer (500 mg), succinic anhydride (1.2 eq.) and DMAP (1.2 eq.) were dissolved in DCM (35 mL). The reaction was stirred at room temperature overnight. After extractions with NaH₂PO₄ 0.1 M pH 5.5 (2×) and brine (1×), the organic layer was further dried with anhydrous Na₂SO₄ filtered and evaporated. The hemiester derivative was obtained in 95 % yield and was used without any further purification.

### Preparation of the LNA-support

The above prepared hemiester derivative (90 µmol) was dissolved in a minimum amount of DMF, DIEA and pyBOP (90 µmol) were added and mixed together for 1 min. This preactivated mixture was combined with LCAA-CPG (500 Å, 80-120 mesh size, 300 mg) in a manual synthesizer and stirred. After 1.5 h at room temperature, the support was filtered off and washed with DMF, DCM and MeOH. After drying the loading was determined to be 57 µmol/g (see Tom Brown, Dorcas J.S.Brown, "Modern machine-aided methods of oligodeoxyribonucleotide synthesis", in: F.Eckstein, editor. Oligonucleotides and Analogues A Practical Approach. Oxford: IRL Press, 1991: 13-14).

### Elongation of the oligonucleotide

The coupling of phosphoramidites (A(bz), G(ibu), 5-methyl-C(bz)) or T-β-cyanoethylphosphoramidite) is performed by using a solution of 0.1 M of the 5'-O-DMT-protected amidite in acetonitrile and DCI (4,5-dicyanoimidazole) in acetonitrile (0.25 M) as activator. The thiolation is carried out by using xanthane chloride (0.01 M in acetonitrile:pyridine 10%). The rest of the reagents are the ones typically used for oligonucleotide synthesis.

### Purification by RP-HPLC:

- Column:: XTerra, RP18, 5µm, 7.8×50mm column.
- Eluent:: Eluent A: 0.1M NH₄OAc, pH: 10.
Eluent B: Acetonitrile
- Flow:: 5ml/min.

### Gradient:

| Time (min.) | Eluent A | Eluent B |
|---|---|---|
| 0,05 min. | 95% | 5% |
| 5 min. | 95% | 5% |
| 12 min. | 65% | 35% |
| 16 min. | 0% | 100% |
| 19 min. | 0% | 100% |
| 21 min | 100% | 0% |

### Analysis by IE-HPLC:

- Column:: Dionex, DNAPac PA-100, 2x250mm column.
- Eluent:: Eluent A: 20mM Tris-HCl, pH 7.6; 1mM EDTA; 10mM NaClO₄.
Eluent B: 20mM Tris-HCl, pH 7.6; 1mM EDTA; 1M NaClO₄.
- Flow:: 0.25ml/min.

### Gradient:

| Time (min.) | Eluent A | Eluent B |
|---|---|---|
| 1 min. | 95% | 5% |
| 10 min. | 65% | 35% |
| 11 min. | 0% | 100% |
| 15 min. | 0% | 100% |
| 16 min | 95% | 5% |
| 21 min. | 95% | 5% |

### Abbreviations

- DMT:: Dimethoxytrityl
- DCI:: 4,5-Dicyanoimidazole
- DMAP:: 4-Dimethylaminopyridine
- DCM:: Dichloromethane
- DMF:: Dimethylformamide
- THF:: Tetrahydrofurane
- DIEA:: *N,N*-diisopropylethylamine
- PyBOP:: Benzotriazole-1-yl-oxy-tris-pyrrolidino-phosphonium-hexafluorophosphate
- Bz:: Benzoyl
- Ibu:: Isobutyryl

### Example 3: Test of design of the oligomeric compound

It was of our interest to evaluate the antisense activity of oligonucleotides with different designs, in order to prove the importance of choosing the best design for an oligonucleotide targeting survivin. For this purpose, we set up an *in vitro* assay that would allow us to screen many different oligonucleotide designs by measuring the activity of the firefly (Photinus pyralis) luciferase after down-regulation by antisense oligonucleotides. Figure 1 contains an illustration of most the designs mentioned in the text. In a first screen, designs containing β-D-oxy-LNA, which were all targeting the same motif within the mRNA were evaluated. Designs consisting of gapmers with a different gap-size, a different load of phosphorothioate internucleoside linkages, and a different load of LNA were tested. Headmers and tailmers with a different load of β-D-oxy-LNA, a different load of phosphorothioate internucleoside linkages and a different load of DNA were prepared. Mixmers of various compositions, which means that bear an alternate number of units of β-D-oxy-LNA, α-L-LNA and DNA, were also analysed in the *in vitro* assay. Moreover, LNA derivatives were also included in different designs, and their antisense activity was assessed. The importance of a good design is reflected by the data that can be obtained in a luciferase assay. The luciferase expression levels are measured in %, and give an indication of the antisense activity of the different designs containing β-D-oxy-LNA and LNA derivatives. We can easily see that some designs are potent antisense oligonucleotides, while others give moderate to low down-regulation levels. Therefore, a close correlation between good antisense activity and optimal design of an oligonucleotide is very evident. We appreciated good levels of down-regulation with various designs. Gapmers with gaps of 7-10 nt DNA and thiolation all over the backbone or with thiolation exclusively in the gap and PO in the flanks showed good results. These designs contain β-D-oxy-LNA or LNA derivatives. Headmers of 6 nt and 8 nt β-D-oxy-LNA also presented good levels of down-regulation, when the phosphorothioate internucleoside linkages are all over the backbone or only in the DNA-segment. Different mixmers gave good antisense activity in the luciferase assay. The alternate number of units of each α-L-oxy-LNA, β-D-oxy-LNA or DNA composition defines the mixmers, see figure 1. A mixmer **3-9-3-1,** which has a deoxynucleoside residue at the 3'-end showed significant levels of down-regulation. In a mixmer **4-1-1-5-1-1-3,** we placed two α-L-oxy-LNA residues interrupting the gap, being the flanks β-D-oxy-LNA. Furthermore, we interrupted the gap with two α-L-oxy-LNA residues, and substituted both flanks with α-L-oxy-LNA. Both designs presented significant levels of down-regulation. The presence of α-L-oxy-LNA might introduce a flexible transition between the North-locked flanks (oxy-LNA) and the α-L-oxy-LNA residue by spiking in deoxynucleotide residues. It is also interesting to study design **4-3-1-3-5** where a α-L-oxy-LNA residue interrupts the DNA stretch. In addition to the α-L-oxy-LNA in the gap, we also substituted two oxy-LNA residues at the edges of the flanks with two α-L-oxy-LNA residues. The presence of just one β-D-oxy-LNA residue (design **4-3-1-3-5**) interrupting the stretch of DNAs in the gap results in a dramatic loss of down-regulation. Just by using α-L-oxy-LNA instead, the design shows significant down-regulation at 50nM oligonucleotide concentration. The placement of α-L-oxy-LNA in the junctions and one α-L-oxy-LNA in the middle of the gap also showed down-regulation.
α-L-oxy-LNA reveals to be a potent tool enabling the construction of different mixmers, which are able to present high levels of antisense activity. Other mixmers such as **4-1-5-1-5** and **3-3-3-3-3-1** can also be prepared. We can easily see that some designs are potent antisense oligonucleotides, while others give moderate to low down-regulation levels. Therefore, again a close correlation between good antisense activity and optimal design of an oligonucleotide is very evident. Other preferred designs are **(1-3-8-3-1)** where DNA residues are located in the flanks with 3 β-D-oxy-LNA monomers at each side of the gap. A further preferred design is (**4-9-3-1**) with D-oxy-LNA flanks and a 9 gap with a DNA at the 3'-end.

### Assay

X1/5 Hela cell line (ECACC Ref. No: 95051229), which was stably transfected with a "tet-off" luciferase system, was used. In the absence of tetracycline the luciferase gene is expressed constitutively. The expression can be measured as light in a luminometer, when the luciferase substrate, luciferin is added. The X1/5 Hela cell line was grown in Minimun Essential Medium Eagle (Sigma M2279) supplemented with 1x Non Essential Amino Acid (Sigma M7145), 1x Glutamax I (Invitrogen 35050-038), 10 % FBS calf serum, 25 µg/ml Gentamicin (Sigma G1397), 500 µg/ml G418 (Invitrogen 10131-027) and 300 µg/ml Hygromycin B (Invitrogen 10687-010). The X1/5 Hela cells were seeded at a density of 8000 cells per well in a white 96 well plate (Nunc 136101) the day before the transfection. Before the transfection, the cells were washed one time with OptiMEM (Invitrogen) followed by addition of 40 µl OptiMEM with 2µ/ml of Lipofectamine2000 (Invitrogen). The cells were incubated for 7 minutes before addition of the oligonucleotides. 10 µl of oligonucleotide solutions were added and the cells were incubated for 4 h at 37°C and 5 % CO₂. After the 4 h incubation, the cells were washed once in OptiMEM and growth medium was added (100 µl). The luciferase expression was measure the next day. Luciferase expression was measured with the Steady-Glo luciferase assay system from Promega. 100 µl of the Steady-Glo reagent was added to each well and the plate was shaken for 30 s at 700 rpm. The plate was read in Luminoskan Ascent instrument from ThermoLabsystems after 8minof incubation to complete total lysis of the cells. The luciferase expression is measured as Relative Light Units per seconds (RLU/s). The data was processed in the Ascent software (v2.6) and graphs were drawn in SigmaPlot2001.

### Example 4: In vitro model: Cell culture

The effect of antisense compounds on target nucleic acid expression can be tested in any of a variety of cell types provided that the target nucleic acid is present at measurable levels. Target can be expressed endogenously or by transient or stable transfection of a nucleic acid encoding said nucleic acid. The expression level of target nucleic acid can be routinely determined using, for example, Northern blot analysis, Real-Time PCR, Ribonuclease protection assays. The following cell types are provided for illustrative purposes, but other cell types can be routinely used, provided that the target is expressed in the cell type chosen.

Cells were cultured in the appropriate medium as described below and maintained at 37°C at 95-98% humidity and 5% CO₂. Cells were routinely passaged 2-3 times weekly.
15PC3: The human prostate cancer cell line 15PC3 was kindly donated by Dr. F. Baas, Neurozintuigen Laboratory, AMC, The Netherlands and was cultured in DMEM (Sigma) + 10% fetal bovine serum (FBS) + Glutamax I + gentamicin
A549: The human non-small cell lung cancer cell line A549 was purchased from ATCC, Manassas and was cultured in DMEM (Sigma) + 10% FBS + Glutamax I + gentamicin
MCF7: The human breast cancer cell line MCF7 was purchased from ATCC and was cultured in Eagle MEM (Sigma) + 10% FBS + Glutamax I + gentamicin
SW480: The human colon cancer cell line SW480 was purchased from ATCC and was cultured in L-15 Leibovitz (Sigma) + 10% FBS + Glutamax I + gentamicin
SW620: The human colon cancer cell line SW620 was purchased from ATCC and was cultured in L-15 Leibovitz (Sigma) + 10% FBS + Glutamax I + gentamicin
HT29: The human prostate cancer cell line HT29 was purchased from ATCC and was cultured in McCoy's 5a MM (Sigma) + 10% FBS + Glutamax I + gentamicin
NCI H23: The human non-small-cell lung cancer cell line was purchased from ATCC and was cultured in RPMI 1640 with Glutamax I (Gibco) + 10% FBS + HEPES + gentamicin
HCT-116: The human colon cancer cell line HCT-116 was purchased from ATCC and was cultured in McCoy's 5a MM + 10% FBS + Glutamax I + gentamicin
MDA-MB-231: The human breast cancer cell line MDA-MB-231 was purchased from ATCC and was cultured in L-15 Leibovitz + 10% FBS + Glutamax I + gentamicin
MDA-MB-435s: The human breast cancer cell line MDA-MB-435s was purchased from ATCC and was cultured in L-15 Leibovitz + 10% FBS + Glutamax I + gentamicin
DMS273: The human small-cell lung cancer cell line DMS273 was purchased from ATCC and was cultured in + 10% FBS + Glutamax +gentamicin
PC3: The human prostate cancer cell line PC3 was purchased from ATCC and was cultured in F12 Coon's with glutamine (Gibco) + 10% FBS + gentamicin
U373: The human glioblastoma astrocytoma cancer cell line U373 was purchased from ECACC and was cultured in EMEM + 10% FBS + glutamax + NEAA + sodiumpyrovate + gentamicin.

HeLa Sur-GFP: Wheately, S.P. et al, Curr. Biol. 11 446-490, 2001

HUVEC-C human umbilical vein endothelial cells were purchased from ATCC and propagated according to the manufacturers instructions.

HMVEC-d (DMVEC's- dermal human microvascular endothelial cells) were purchased from Clonetics and cultured as described by manufacturer.
HMVEC human microvascular endothelial cells were purchased from Clonetics and cultured as stated by manufacturer
Human embryonic lung fibroblasts were purchased from ATCC and cultured as described by manufacturer
HMEC-1 Human mammary epithelial cells were purchased from Clonetics and maintained as recommended by the manufacturer.

### Example 5: In vitro model: Treatment with antisense oligonucleotide

The cells were treated with oligonucleotide using the cationic liposome formulation LipofectAMINE 2000 (Gibco) as transfection vehicle.
Cells were seeded in 12-well cell culture plates (NUNC) and treated when 80-90% confluent. Oligo concentrations used ranged from 125 nM to 0,2 nM final concentration. Formulation of oligo-lipid complexes were carried out essentially as described in Dean et al. (Journal of Biological Chemistry 1994, 269, 16416-16424) using serum-free OptiMEM (Gibco) and a final lipid concentration of 10 µg/mlLipofectAMINE 2000 in 500 µl total volume..
Cells were incubated at 37°C for 4 hours and treatment was stopped by removal of oligo-containing culture medium. Cells were washed and serum-containing media was added. After oligo treatment cells were allowed to recover for 18 hours before they were harvested for RNA or protein analysis.

### Example 6: in vitro model: Extraction of RNA and cDNA synthesis

### Total RNA Isolation

Total RNA was isolated either using RNeasy mini kit (Qiagen cat. no. 74104) or using the Trizol reagent (Life technologies cat. no. 15596). For RNA isolation from cell lines, RNeasy is the preferred method and for tissue samples Trizol is the preferred method.

Total RNA was isolated from cell lines using the Qiagen RNA OPF Robot - BIO Robot 3000 according to the protocol provided by the manufacturer.
Tissue samples were homogenised using an Ultra Turrax T8 homogeniser (IKA Analysen technik) and total RNA was isolated using the Trizol reagent protocol provided by the manufacturer.

### First strand synthesis

First strand synthesis was performed using OmniScript Reverse Transcriptase kit (cat# 205113, Qiagen) according to the manufacturers instructions. For each sample 0.5 µg total RNA was adjusted to 12 µl each with RNase free H₂O and mixed with 2 µl poly (dT)₁₂₋₁₈ (2.5 µg/ml) (Life Technologies, GibcoBRL, Roskilde, DK), 2 µl dNTP mix (5 mM each dNTP), 2 µl 10x Buffer RT, 1 µl RNAguard™Rnase INHIBITOR (33.3U/ml), (cat# 27-0816-01, Amersham Pharmacia Biotech, Hørsholm, DK) and 1 µl OmniScript Reverse Transcriptase (4 U/µl) followed by incubation at 37°C for 60 minutes and heat inactivation of the enzyme at 93°C for 5 minutes.

### Example 7: in vitro model: Analysis of Oligonucleotide Inhibition of Survivin Expression by Real-time PCR

Antisense modulation of Survivin expression can be assayed in a variety of ways known in the art. For example, Survivin mRNA levels can be quantitated by, e.g., Northern blot analysis, competitive polymerase chain reaction (PCR), or real-time PCR. Real-time quantitative PCR is presently preferred. RNA analysis can be performed on total cellular RNA or mRNA.

Methods of RNA isolation and RNA analysis such as Northern blot analysis is routine in the art and is taught in, for example, Current Protocols in Molecular Biology, John Wiley and Sons.

Real-time quantitative (PCR) can be conveniently accomplished using the commercially iQ Multi-Color Real Time PCR Detection System available from BioRAD.

### Real-time Quantitative PCR Analysis of Survivin mRNA Levels

Quantitation of mRNA levels was determined by real-time quantitative PCR using the iQ Multi-Color Real Time PCR Detection System (BioRAD) according to the manufacturers instructions.
Real-time Quantitative PCR is a technique well known in the art and is taught in for example Heid et al. Real time quantitative PCR, Genome Research (1996), 6: 986-994.

Platinum Quantitative PCR SuperMix UDG 2x PCR master mix was obtained from Invitrogen cat# 11730. Primers and TaqMan® probes were obtained from MWG-Biotech AG, Ebersberg, Germany

Probes and primers to human Survivin were designed to hybridise to a human Survivin sequence, using published sequence information (GenBank accession number NM 001168, incorporated herein as SEQ ID NO:1).

For human Survivin the PCR primers were:
Assay 1
   forward primer: 5' caggtccccgctttctttg 3' (final concentration in the assay; 0.6 µM)
   reverse primer: 5' ggaggagggcgaatcaaa 3' (final concentration in the assay; 0.6 µM) and
   the PCR probe was: 5' FAM- ccatcatcttacgccagacttcagcc-TAMRA 3' (final concentration in the assay; 0.1 µM) Assay 2
   forward primer: 5' aaggaccaccgcatctctaca 3' (final concentration in the assay; 0.9 µM) reverse primer: 5' ccaagtctggctcgttctcagt 3' (final concentration in the assay; 0.6 µM) and
   the PCR probe was: 5' FAM- cgaggctggcttcatccactgcc -TAMRA 3' (final concentration in the assay; 0.1 µM)

Glyceraldehyde-3-phosphate dehydrogenase (GAPDH) mRNA quantity was used as an endogenous control for normalizing any variance in sample preparation.

The sample content of human GAPDH mRNA was quantified using the human GAPDH ABI Prism Pre-Developed TaqMan Assay Reagent (Applied Biosystems cat. no. 4310884E) according to the manufacturers instructions.

For quantification of mouse GAPDH mRNA the following primers and probes were designed: Sense primer 5'aaggctgtgggcaaggtcatc 3' (0.3 µM final concentration), antisense primer 5' gtcagatccacgacggacacatt (0.6 µM final concentration), TaqMan probe 5' FAM-gaagctcactggcatggcatggccttccgtgttc-TAMRA 3'(0.2 µM final concentration).

### Real time PCR

The cDNA from the first strand synthesis performed as described in example 6 was diluted 2-20 times, and analyzed by real time quantitative PCR. The primers and probe were mixed with 2 x Platinum Quantitative PCR SuperMix UDG (cat. # 11730, Invitrogen) and added to 3.3 µl cDNA to a final volume of 25 µl. Each sample was analysed in triplicates. Assaying 2 fold dilutions of a cDNA that had been prepared on material purified from a cell line expressing the RNA of interest generated standard curves for the assays. Sterile H₂O was used instead of cDNA for the no template control. PCR program: 50°C for 2 minutes, 95°C for 10 minutes followed by 40 cycles of 95°C, 15 seconds, 60°C, 1 minutes. Relative quantities of target mRNA sequence were determined from the calculated Threshold cycle using the iCycler iQ Real-time Detection System software.

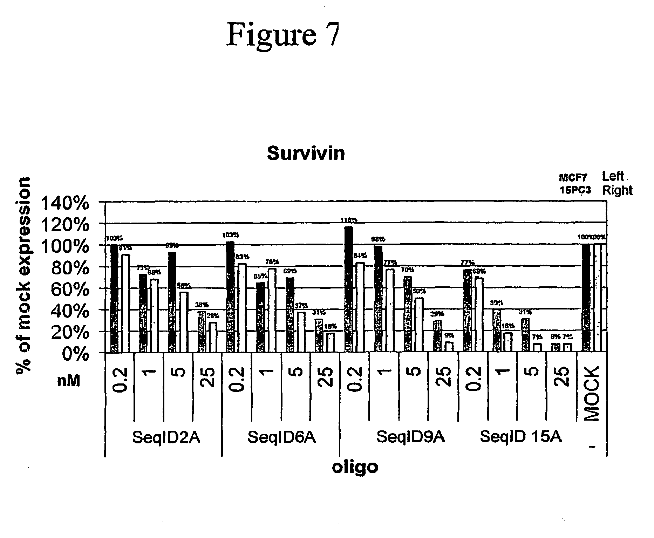

### See Figure 7 and Table 1, 2, 3, 4 and 5.

### Example 8: in vitro analysis: Northern Blot Analysis of Survivin mRNA Levels

Northern blot analysis was carried out by procedures well known in the art essentially as described in Current Protocols in Molecular Biology, John Wiley & Sons.
The hybridisation probe was obtained by PCR-amplification of a 373 bp fragment from 1 µl cDNA obtained by reverse transcription PCR. The reaction was carried out using primers 5' agcacaaagccattctaagtcattg 3' (forward) and 5' tccatcatcttacgccagacttc 3' (reverse) at 0,5 µM final concentration each, 200 nM each dNTP, 1,5 mM MgCl₂ and Platinum Taq DNA polymerase (Invitrogen cat. no. 10966-018). The DNA was amplified for 40 cycles on a Perkin Elmer 9700 thermocycler using the following program: 94°C for 2 min. then 40 cycles of 94°C for 30 sec. and 72°C for 30 sec. with a decrease of 0.5°C per cycle followed by 72°C for 7 min.

The amplified PCR product was purified using S-400 MicroSpin columns (Amersham Pharmacia Biotech cat. no. 27-5140-01) according to the manufacturers instructions and quantified by spectrophotometry.

The hybridisation probe was labelled using Redivue^{™} [α-³²P]dATP 3000 Ci/mmol (Amersham Pharmacia Biotech cat. # AA 0005) and Prime-It RmT labeling kit (Stratagene cat. no. 300392) according to the manufacturers instructions and the radioactively labeled probe was purified using S-300 MicroSpin columns (Amersham Pharmacia Biotech cat. no. 27-5130-01).
Before use, the probe was denatured at 96°C and immediately put on ice.

Samples of 2 µg of total RNA purified as described in example 6 were denatured and size separated on a 2,2 M formaldehyde/MOPS agarose gel. RNA was transferred to positively charged nylon membrane by downward capillary transfer using the TurboBlotter (Schleicher & Schuell) and the RNA was immobilised to the membrane by UV crosslinking using a Stratagene crosslinker. The membrane was prehybridised in ExpressHyb Hybridization Solution (Clontech cat. No. 8015-1) at 60°C and the probe was subsequently added for hybridisation. Hybridisation was carried out at 60°C and the blot was washed with low stringency wash buffer (2 x SSC, 0,1% SDS) at room temperature and with high stringency wash buffer (0,1 x SSC, 0,1% SDS) at 50°C.

The blot was exposed to Kodak storage phosphor screens and scanned in a BioRAD FX molecular imager. Survivin mRNA levels were quantified by Quantity One software (BioRAD)

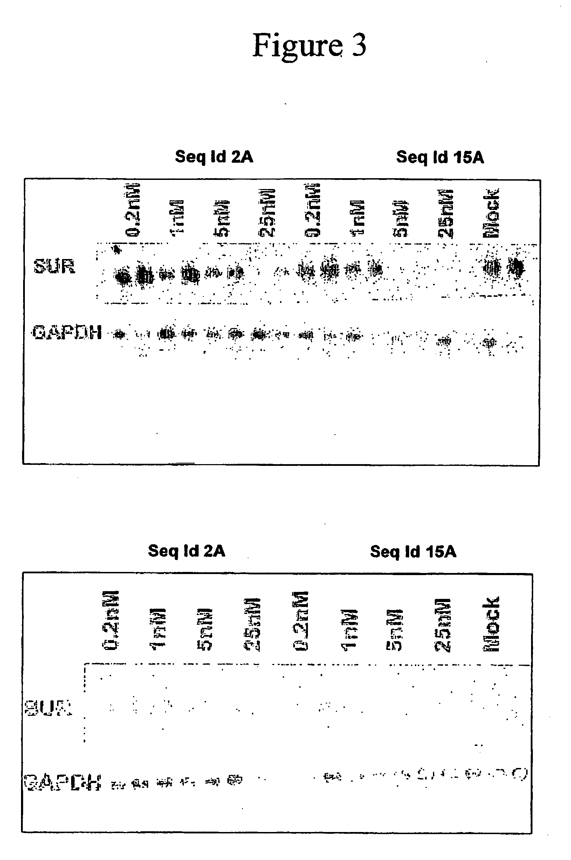

Equality of RNA sample loading was assessed by stripping the blot in 0,5% SDS in H₂O at 85°C and reprobing with a labelled GAPDH (glyceraldehyde-3-phosphate dehydrogenase) probe obtained essentially as described above using the primers 5' aacggatttggtcgtatt 3' (forward) and 5' taagcagttggtggtgca 3' (reverse). See figure 2 and 3. Intensity was monitored with phosphoimager Biorad, FX-scanher (see below). The tested oligomeric compounds are presented in Example 10.

### Percentage down regulation of mRNA estimated from Survivin Northern blotting (data is normalised to GAPDH).

| **Compound / Seq ID** | ***0,2 nM*** | ***1 nM*** | ***5 nM*** | ***25 nM*** |
|---|---|---|---|---|
| **2A** | 31 % | 34 % | 55 % | 77 % |
| **6A** | 22 % | 48 % | 71 % | 91 % |
| **9A** | 21 % | 44 % | 67 % | 64 % |
| **15A** | 45 % | 79 % | 93 % | 95 % |

### Example 9: In vitro analysis: Western blot analysis of Survivin protein levels

Protein levels of Survivin can be quantitated in a variety of ways well known in the art, such as immunoprecipitation, Western blot analysis (immunoblotting), ELISA, RIA (Radio Immuno Assay) or fluorescence-activated cell sorting (FACS). Antibodies directed to Survivin can be identified and obtained from a variety of sources, such as Upstate Biotechnologies (Lake Placid, USA), Novus Biologicals (Littleton, Colorado), Santa Cruz Biotechnology (Santa Cruz, California) or can be prepared via conventional antibody generation methods.

### Western blotting:

The *in vitro* effect of survivin oligoes on Survivin protein levels in transfected cells was determined by Western Blotting. Cells were transfected as described in example 5. Approximately 24 hours after transfection, cells were harvested, lysed in 2.5% SDS, 5 mM DTT and 6 M urea supplemented with protease inhibitor cocktail tablets (Roche). Total protein concentrations were measured using a Bradford reagent. 150 µg total proteins was loaded onto a 12% . Bis-Tris gel, run with MOPS buffer and blotted onto a PVDF membrane according to manufacture's recommendations (Invitrogen). After overnight incubation in blocking buffer (Invitrogen) the membrane was incubated two hours with rabbit anti-Survivin antibodies (AF886 from R&D or Novus 500-201 from Abcam) followed by one hour incubation in secondary antibodies. A chromogenic immunodetection kit (Invitrogen) was used to visualize Survivin. Alternatively, the membrane was incubated with HRP conjugated rabbit immunoglobulins (DAKO) followed by incubation with ECL⁺ Plus reagent (Amersham) and visualized using VersaDoc chemiluminescens detection system. (see Figure 13 The tested oligomeric compounds are presented in Example 10.)

### Example 10: In vitro analysis: Antisense Inhibition of Human Survivin Expression by oligomeric compound

In accordance with the present invention, a series of oligonucleotides were designed to target different regions of the human Survivin RNA, using published sequences (GenBank accession number NM_001168, incorporated herein as SEQ ID NO: 1). The oligonucleotides 16 nucleotides in length are shown in Table 1 and 2. "Target site" indicates the first nucleotide number on the particular target sequence to which the oligonucleotide binds. The preferred compounds are the LNA containing compounds. Table 3 shows low IC50 of four compounds.

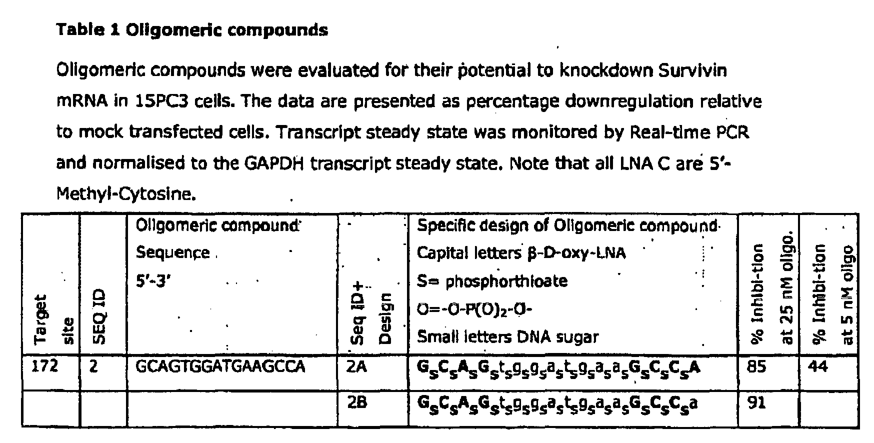

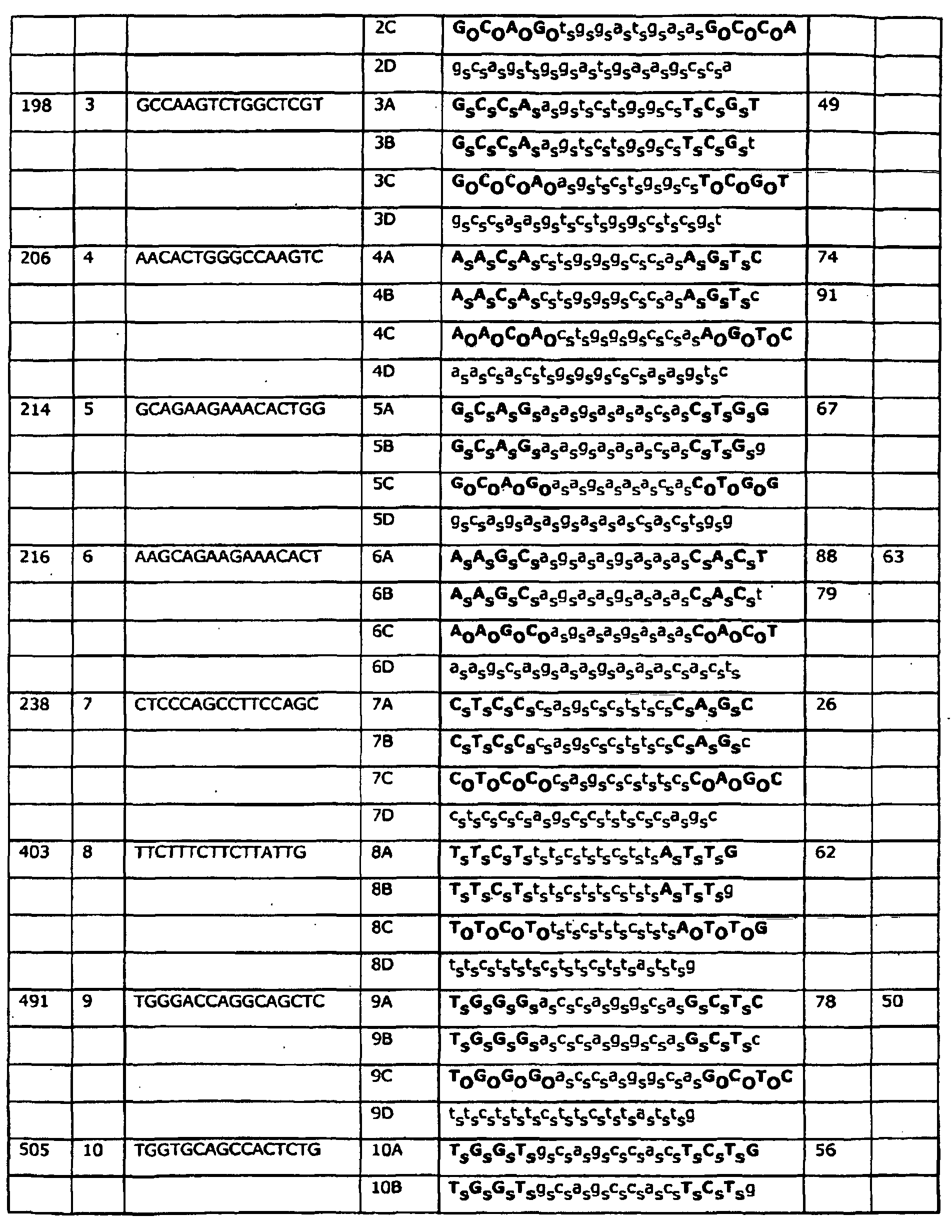

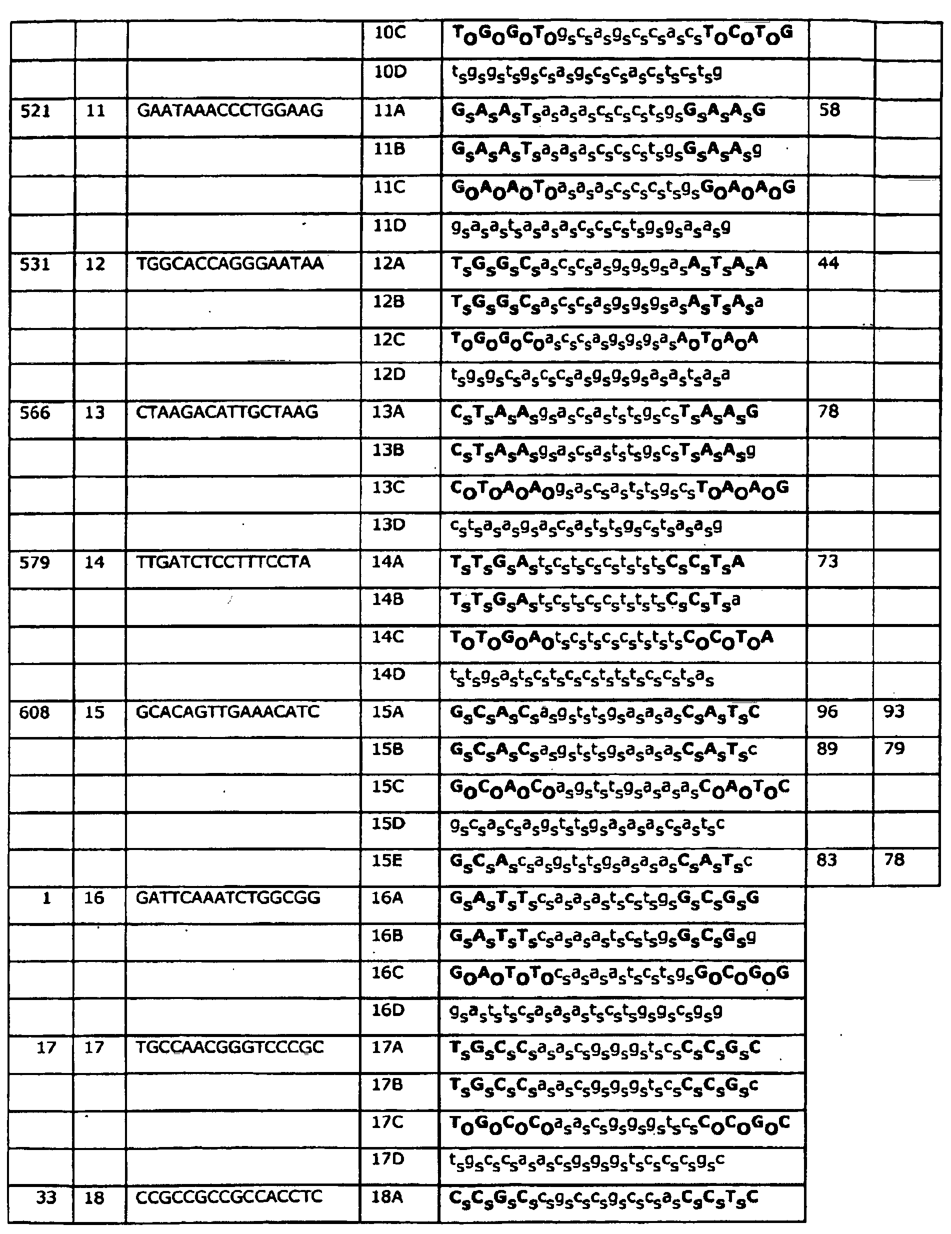

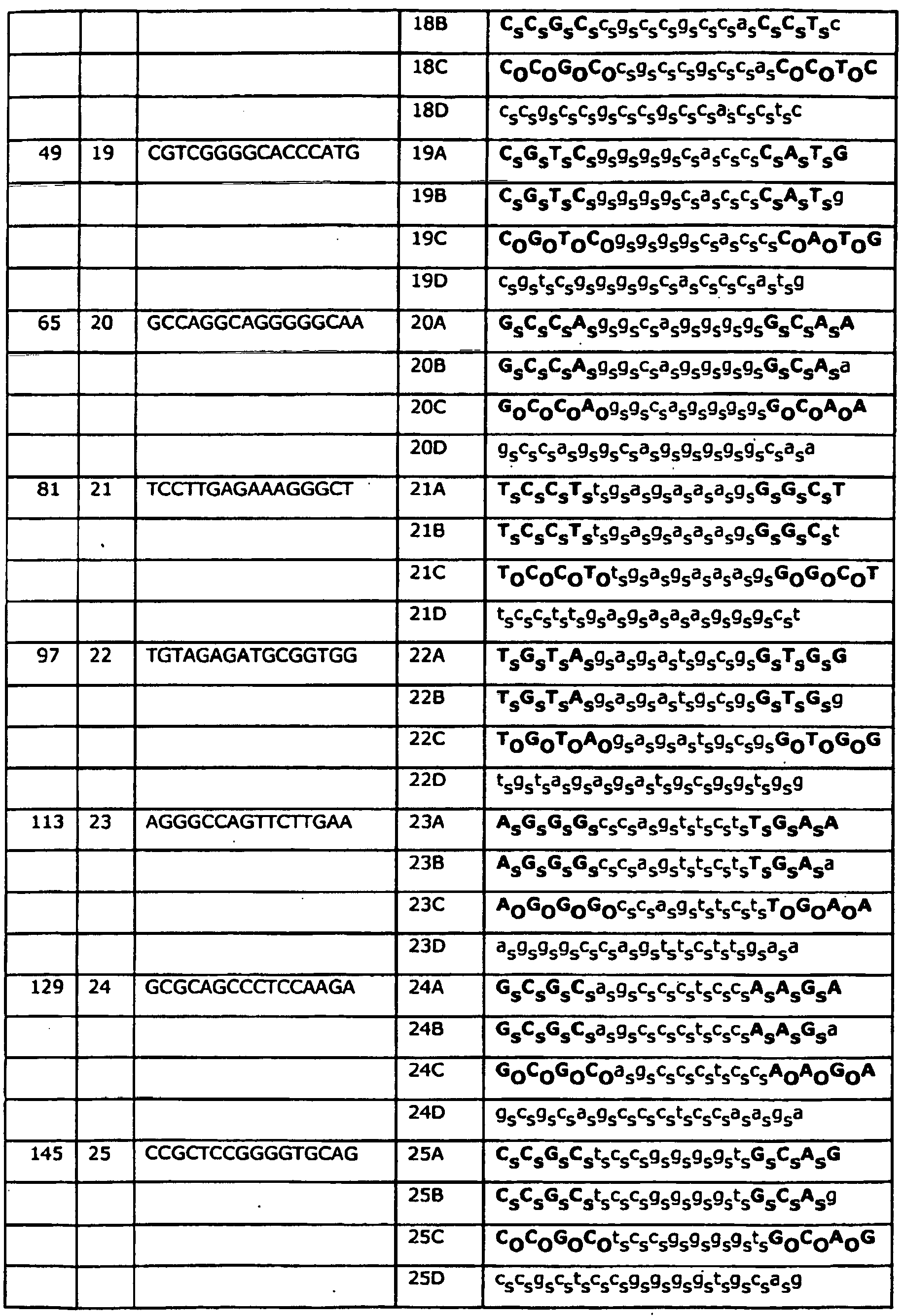

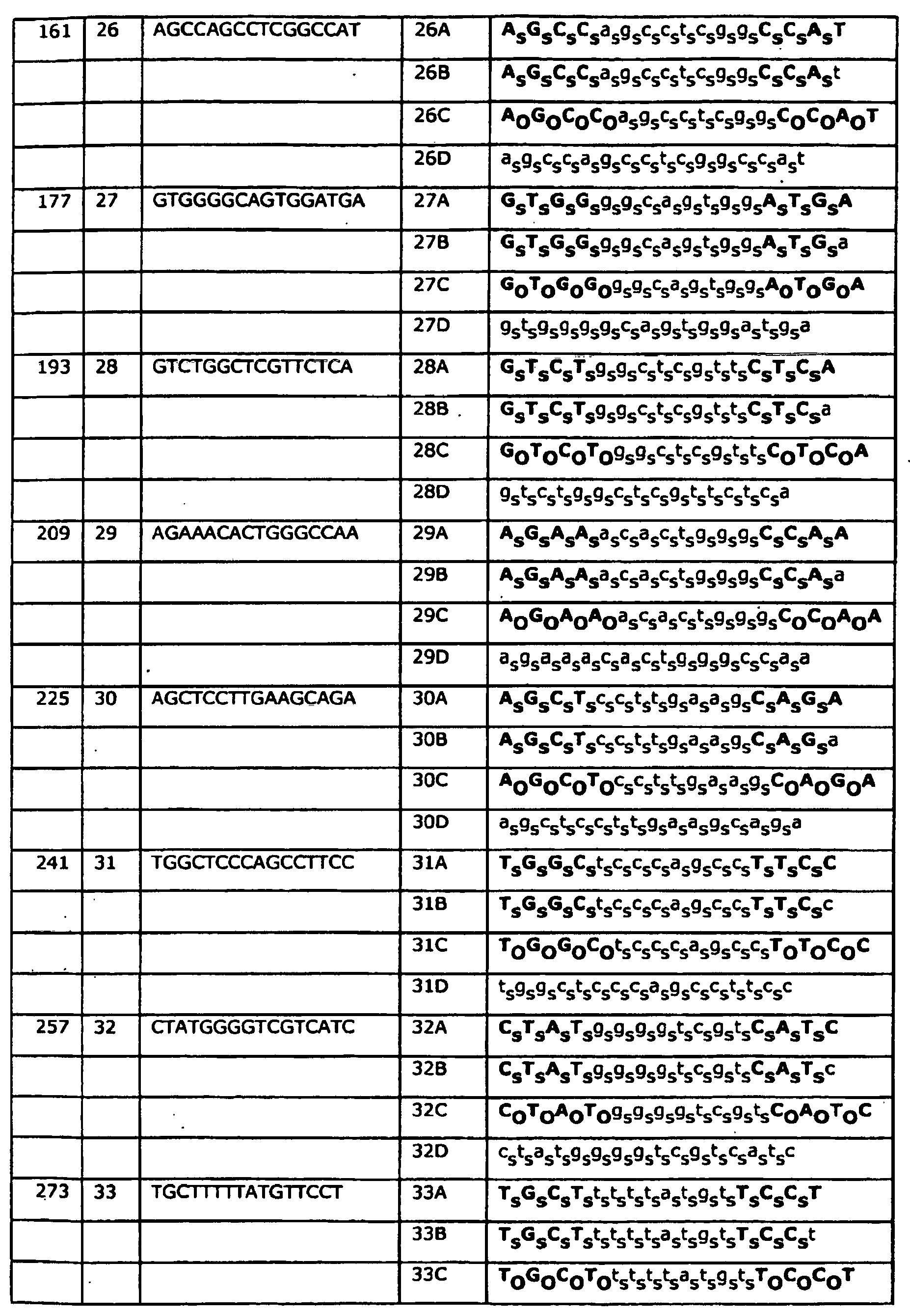

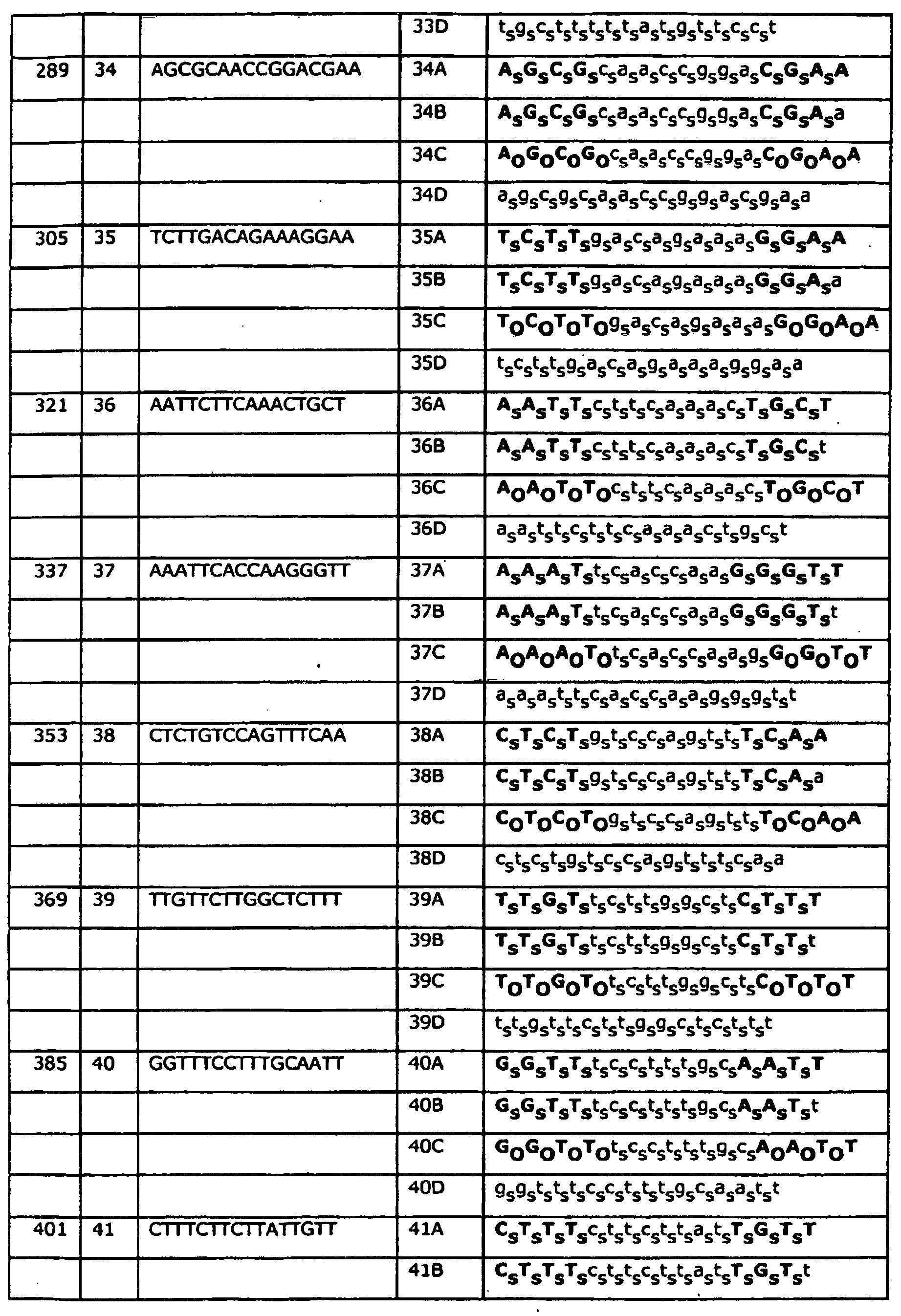

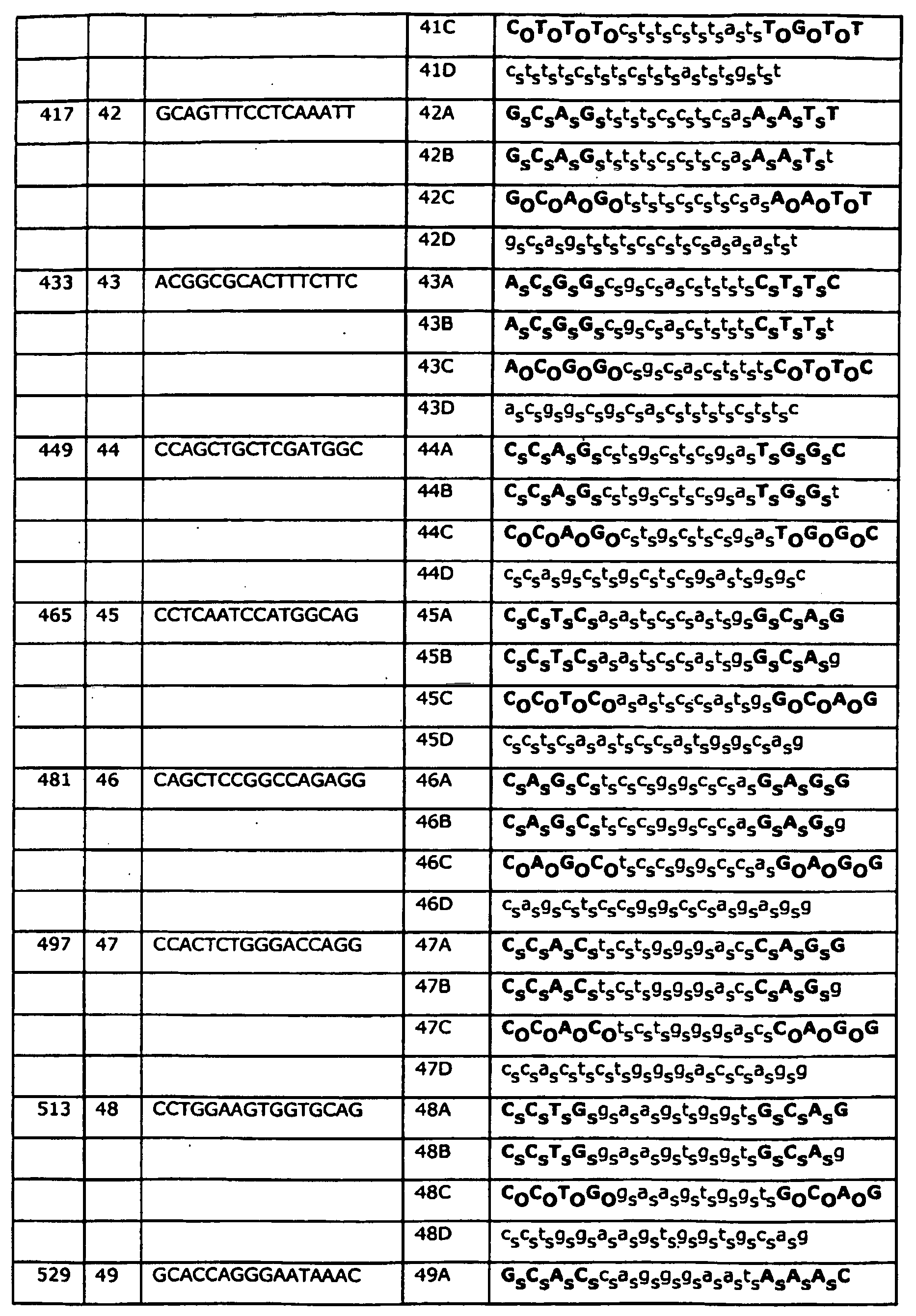

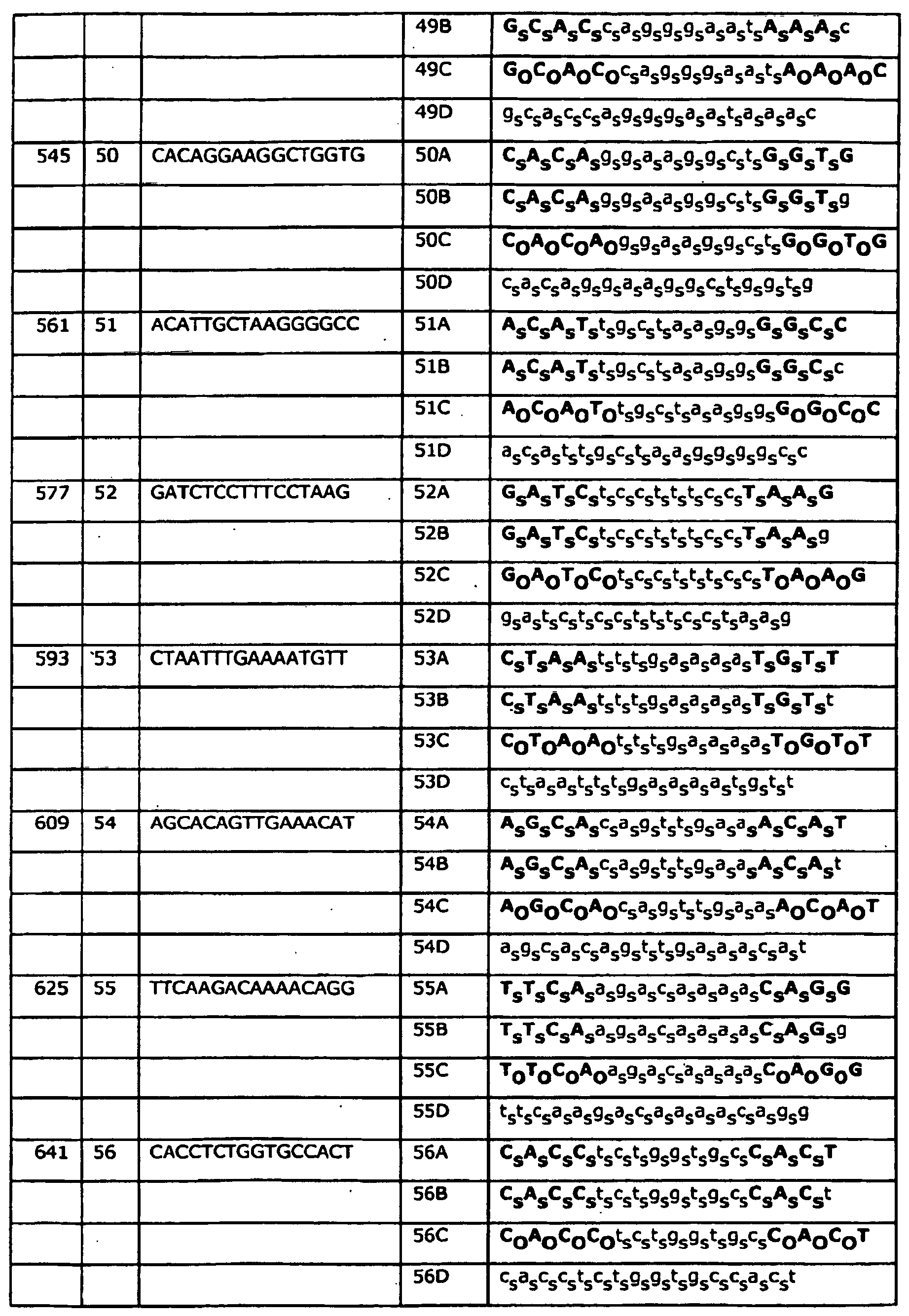

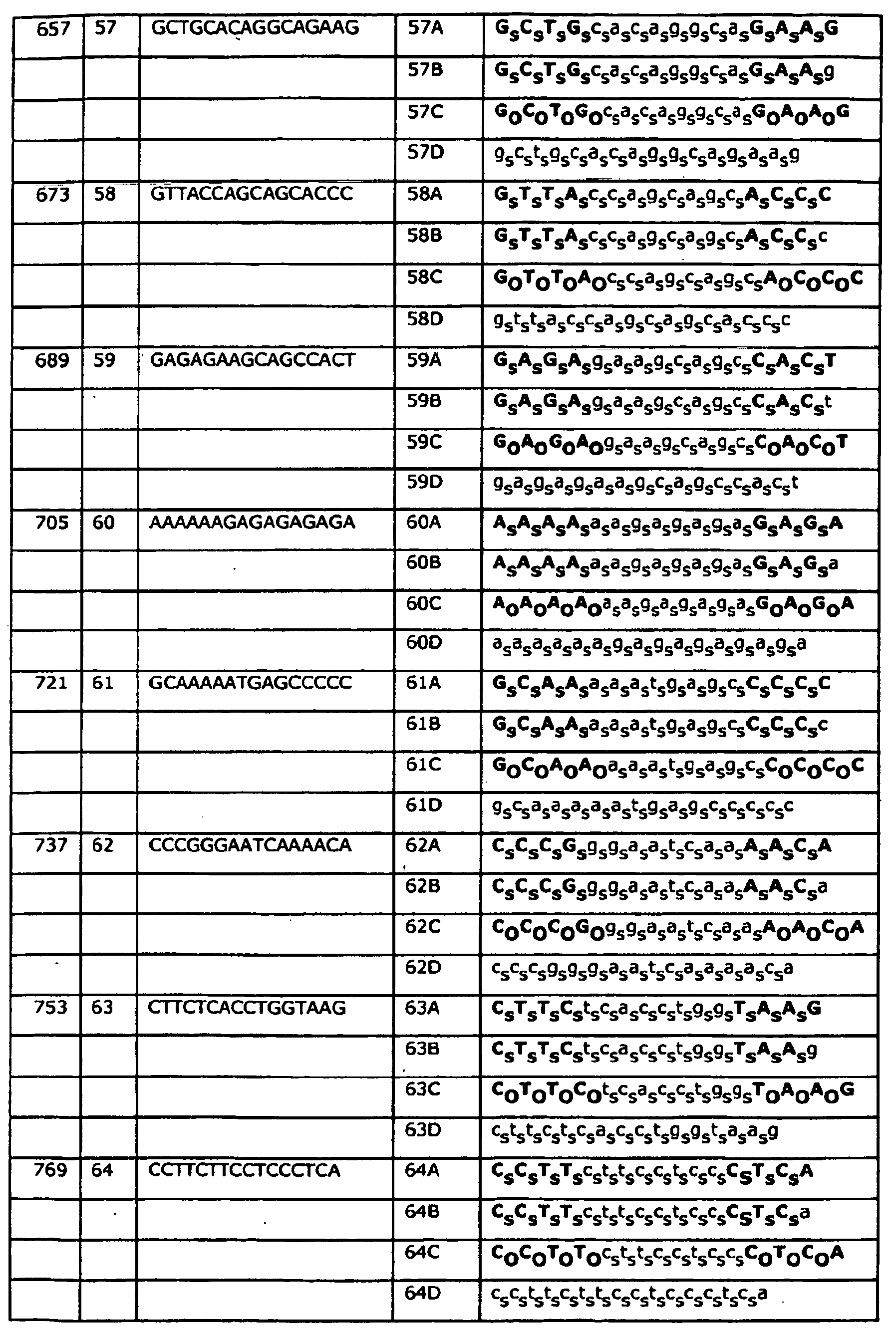

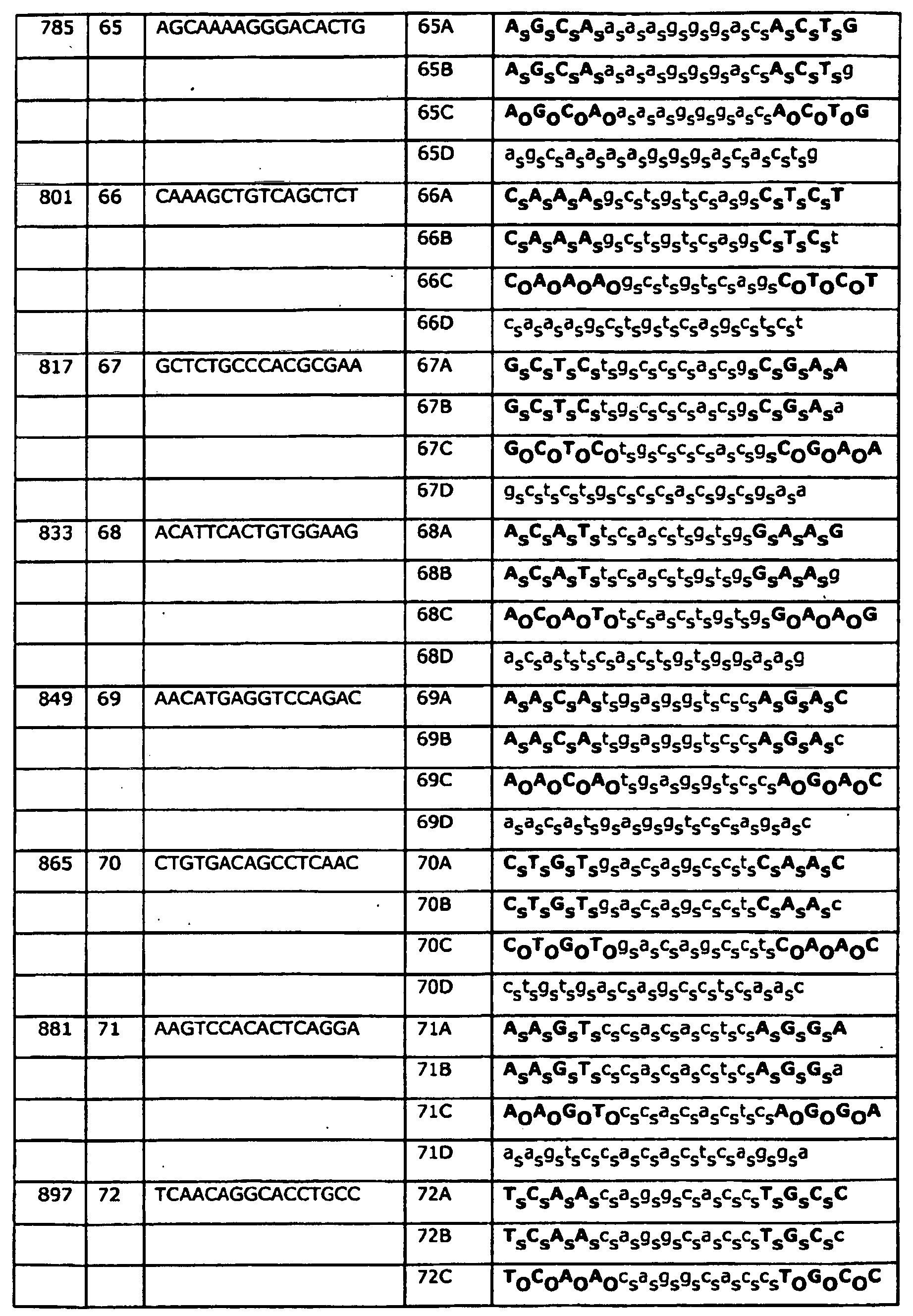

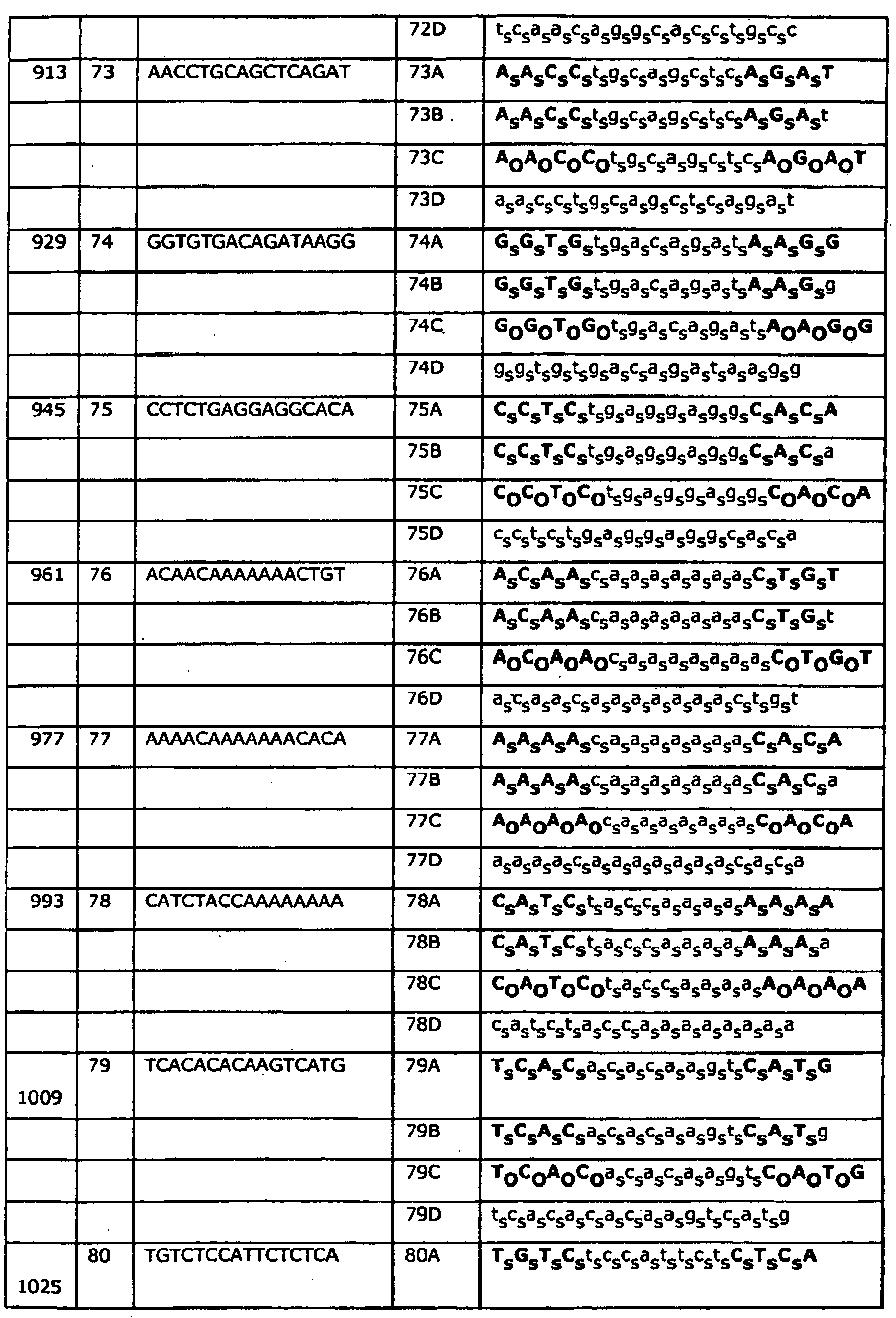

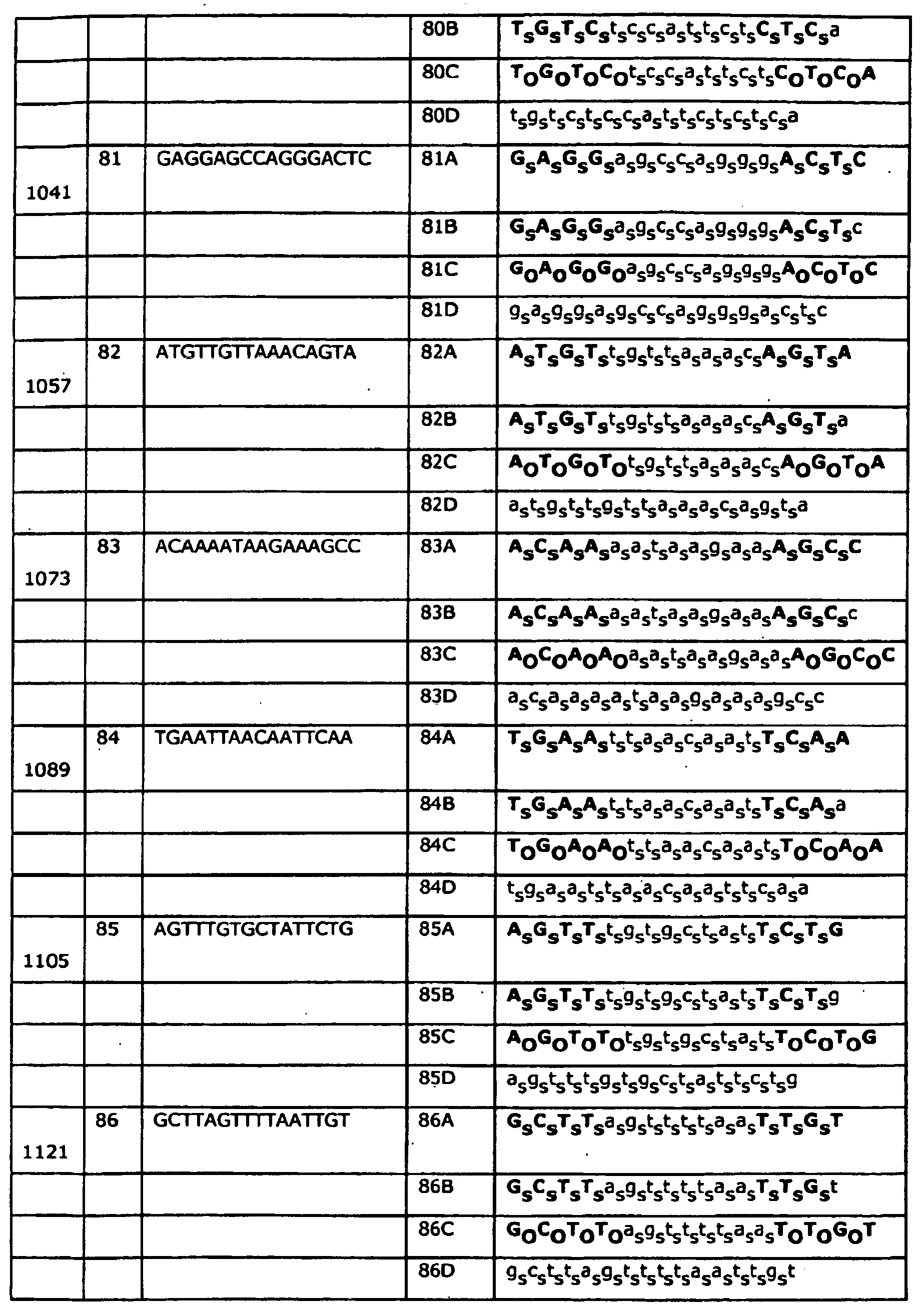

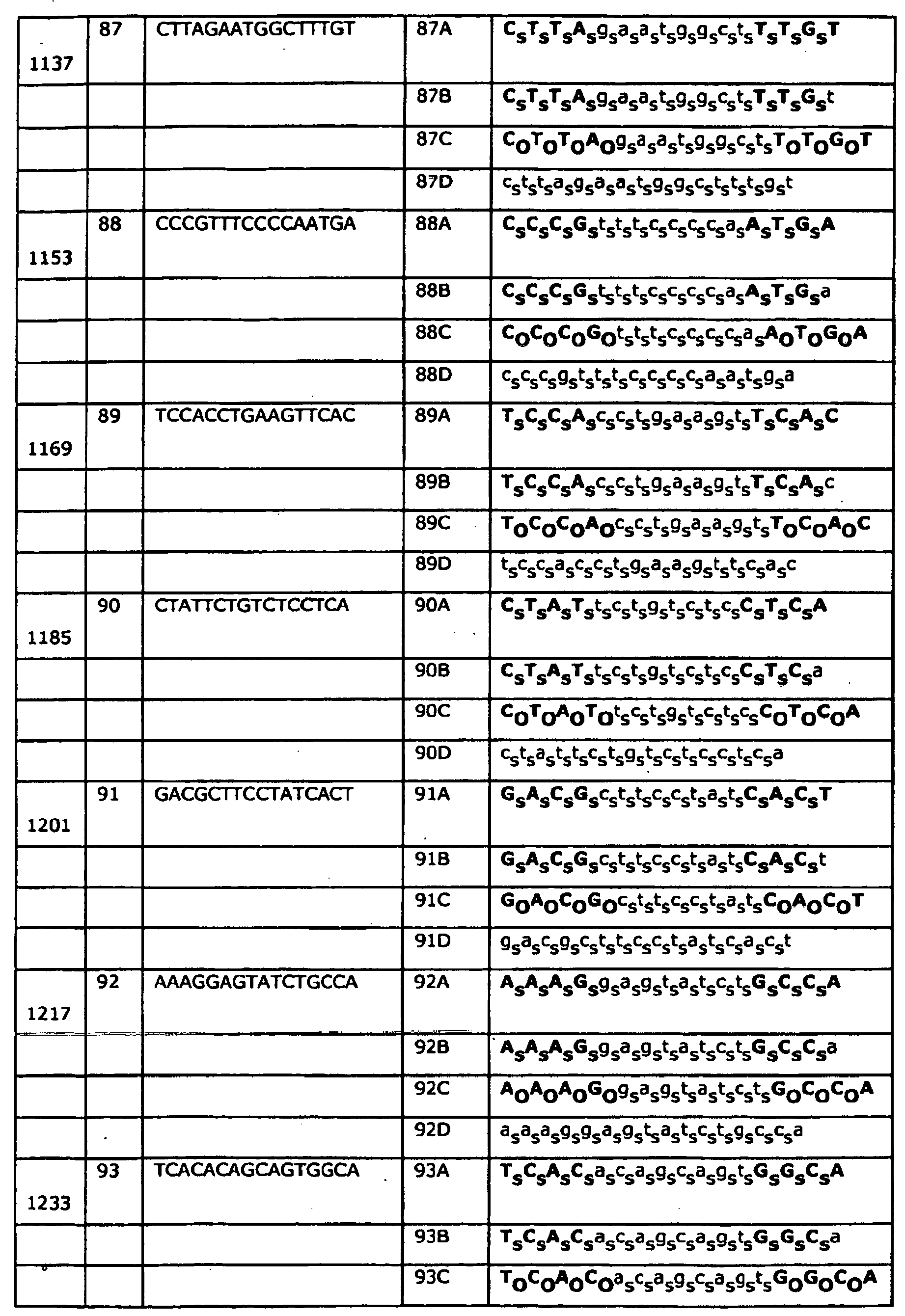

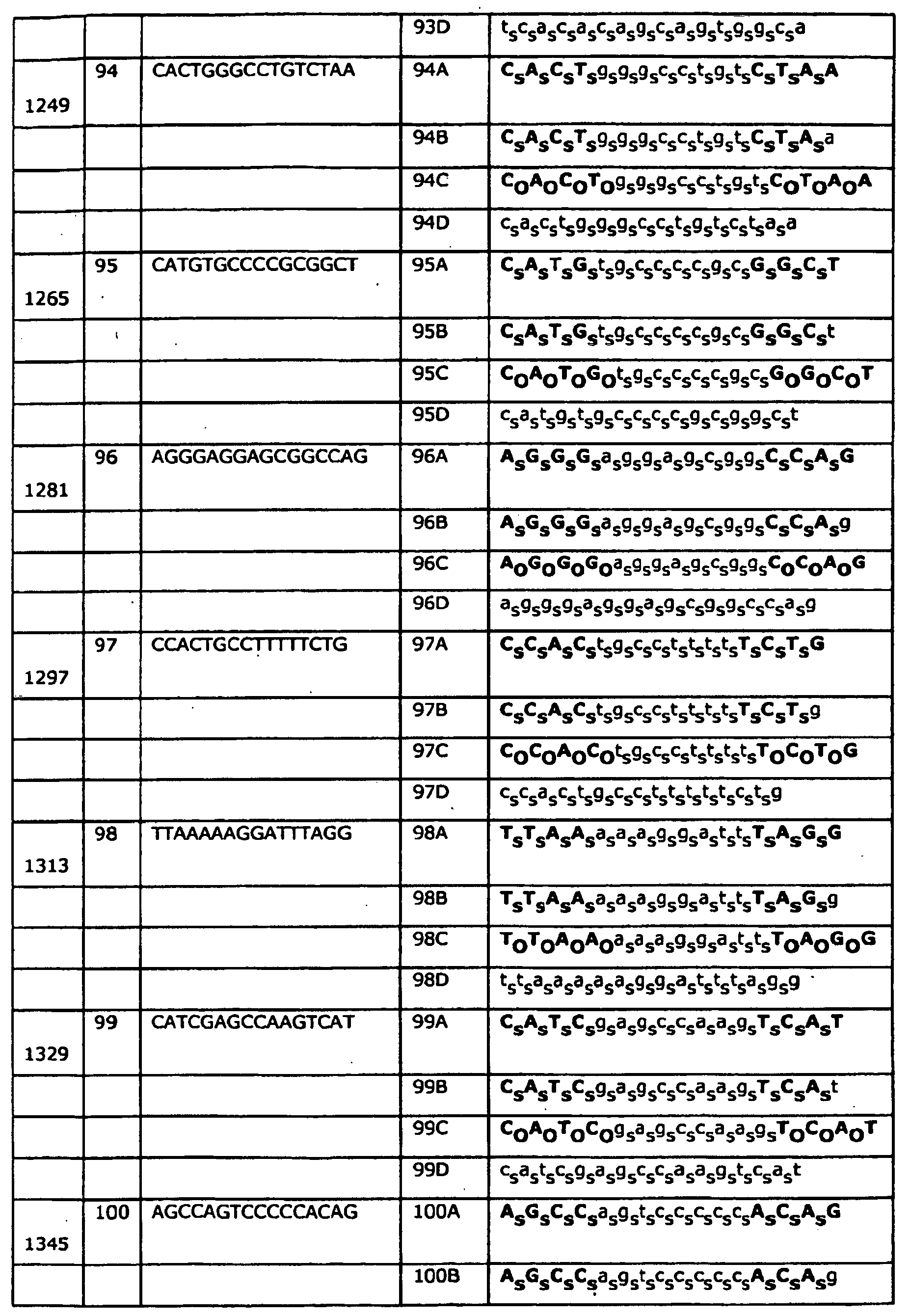

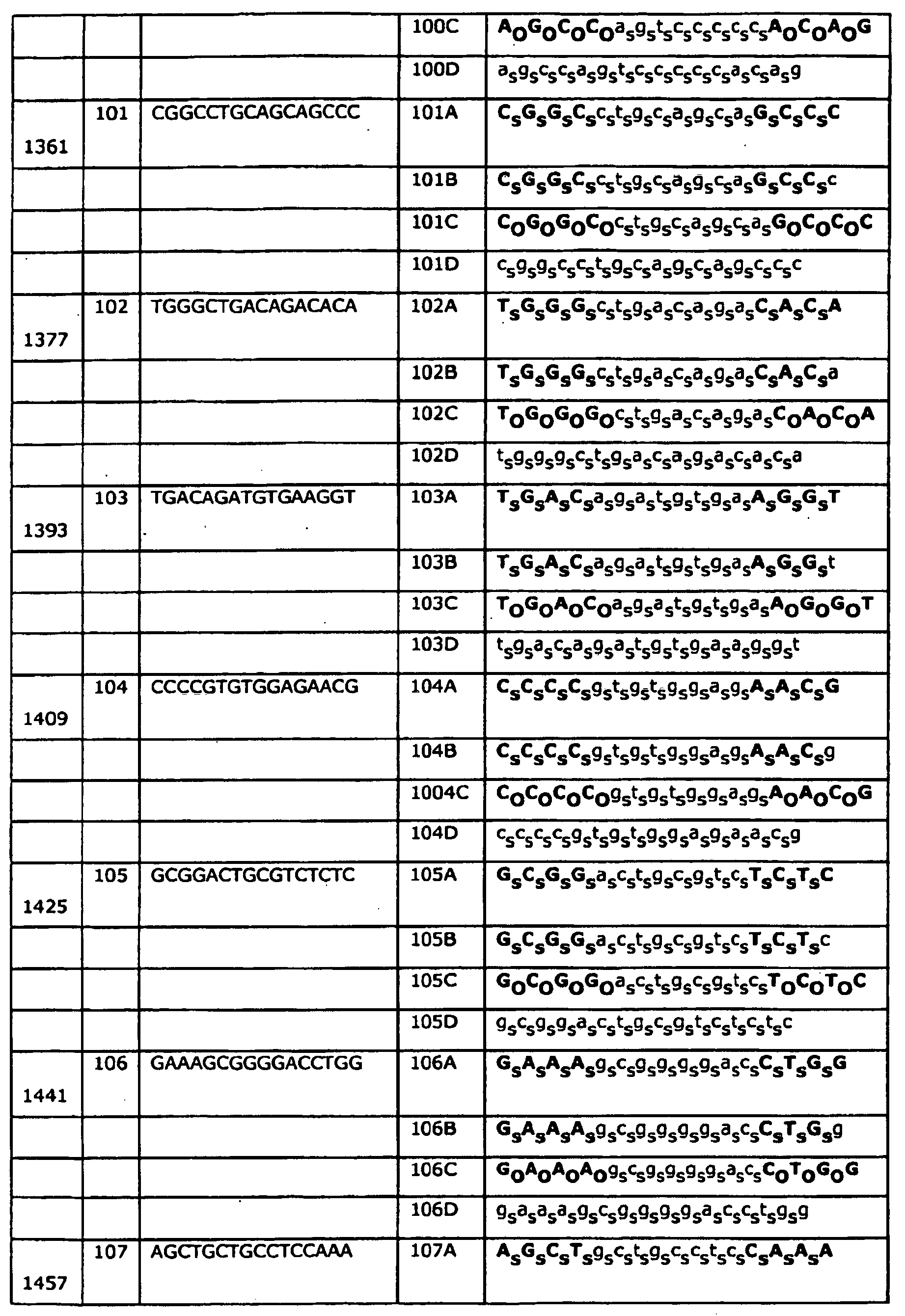

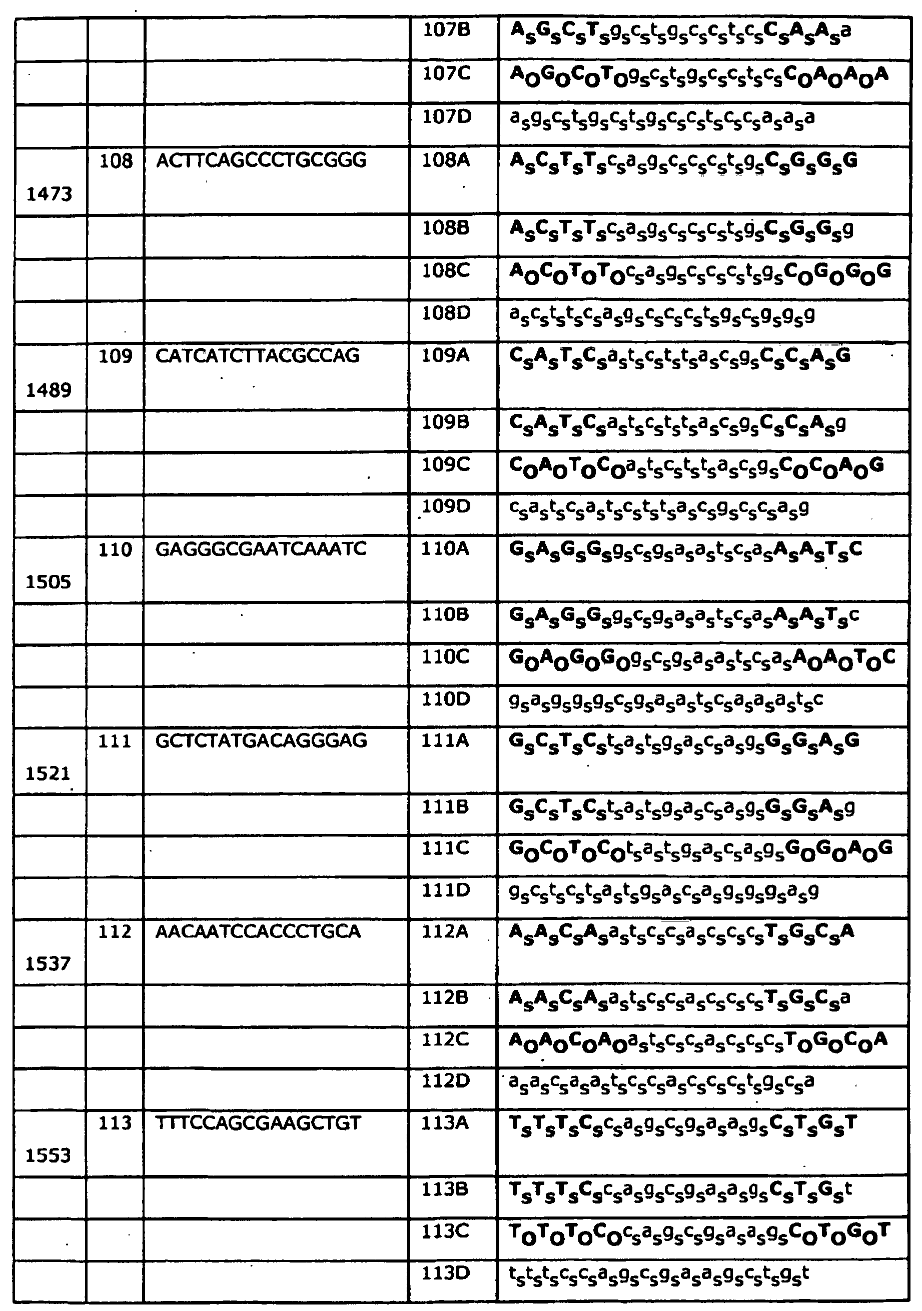

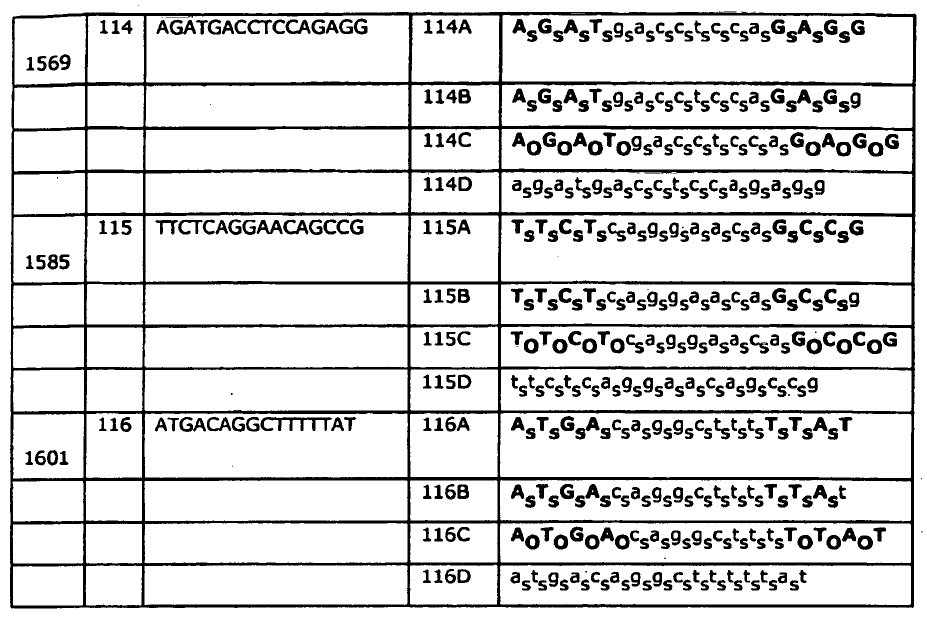

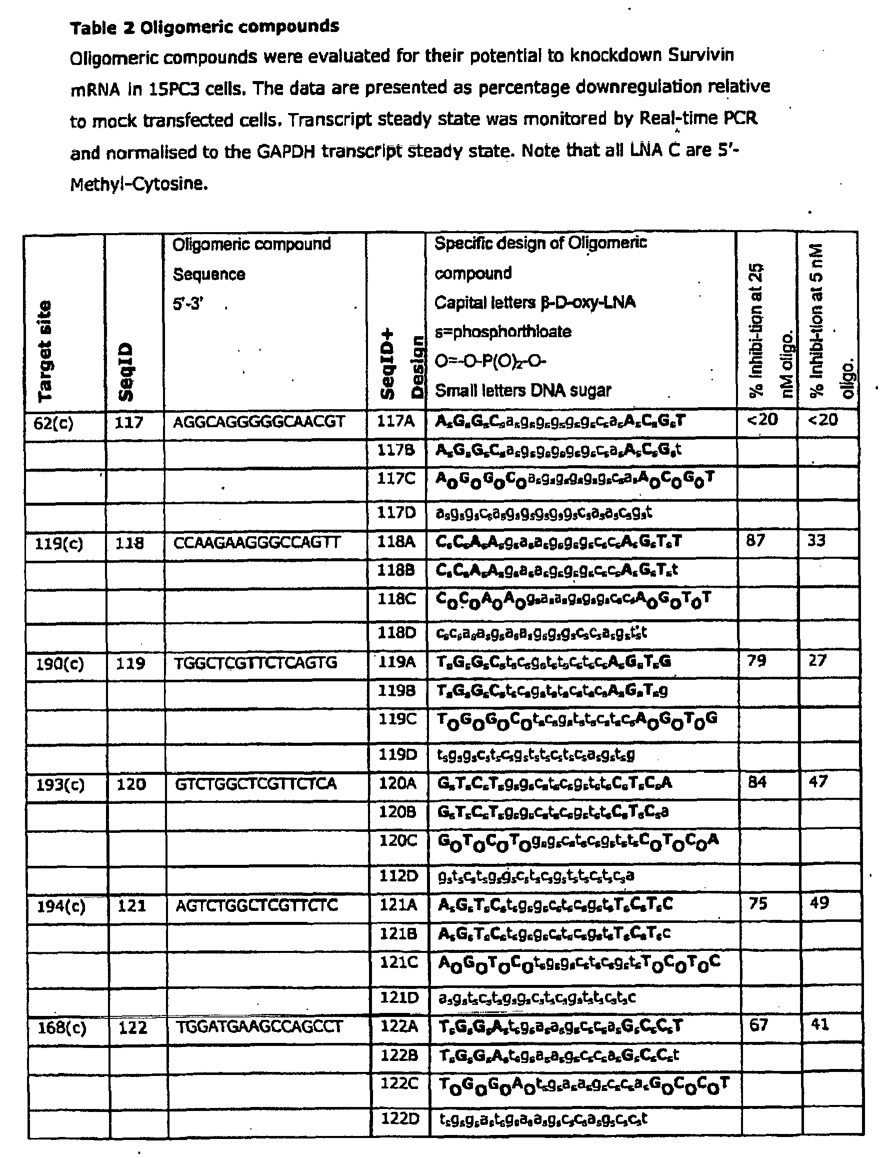

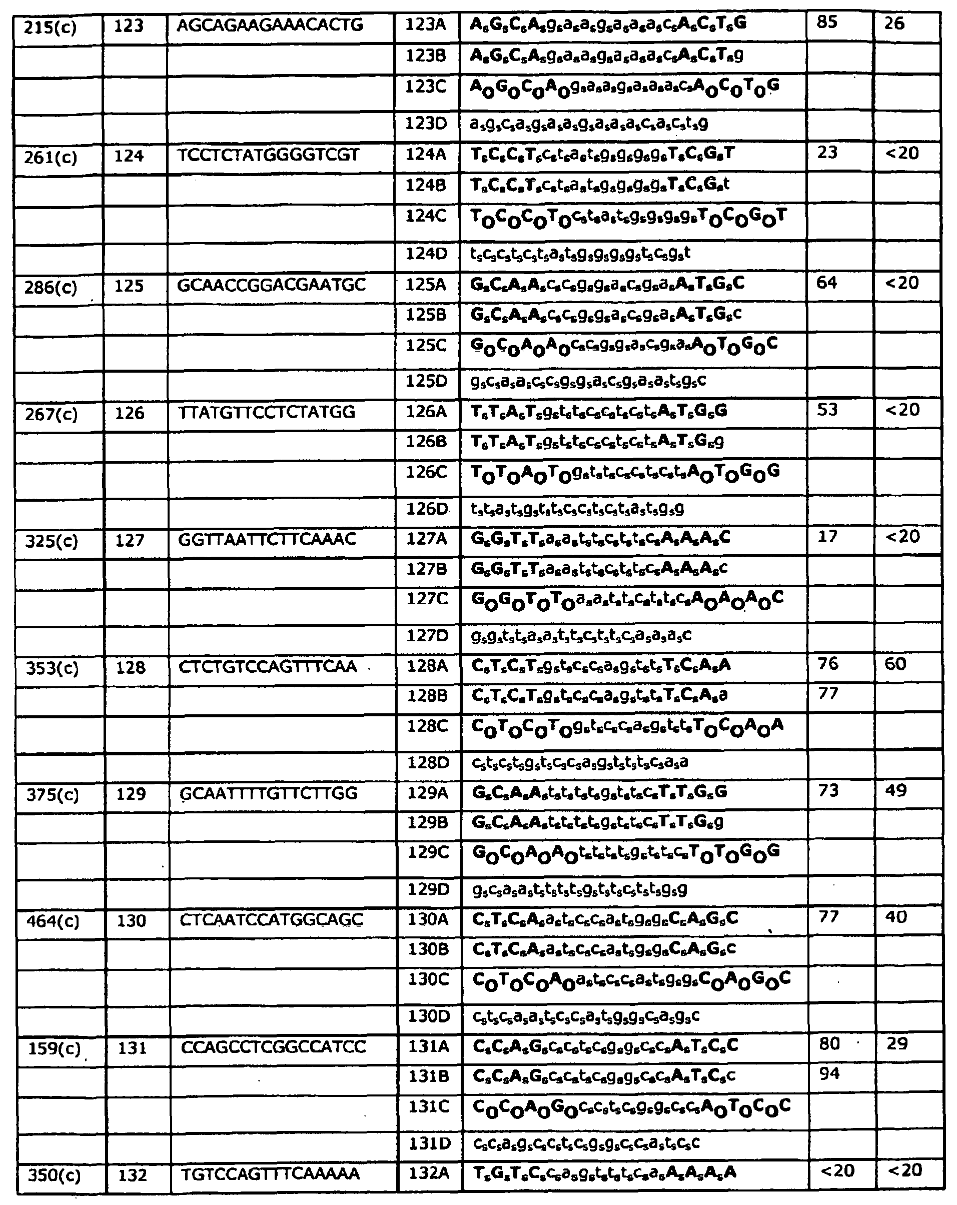

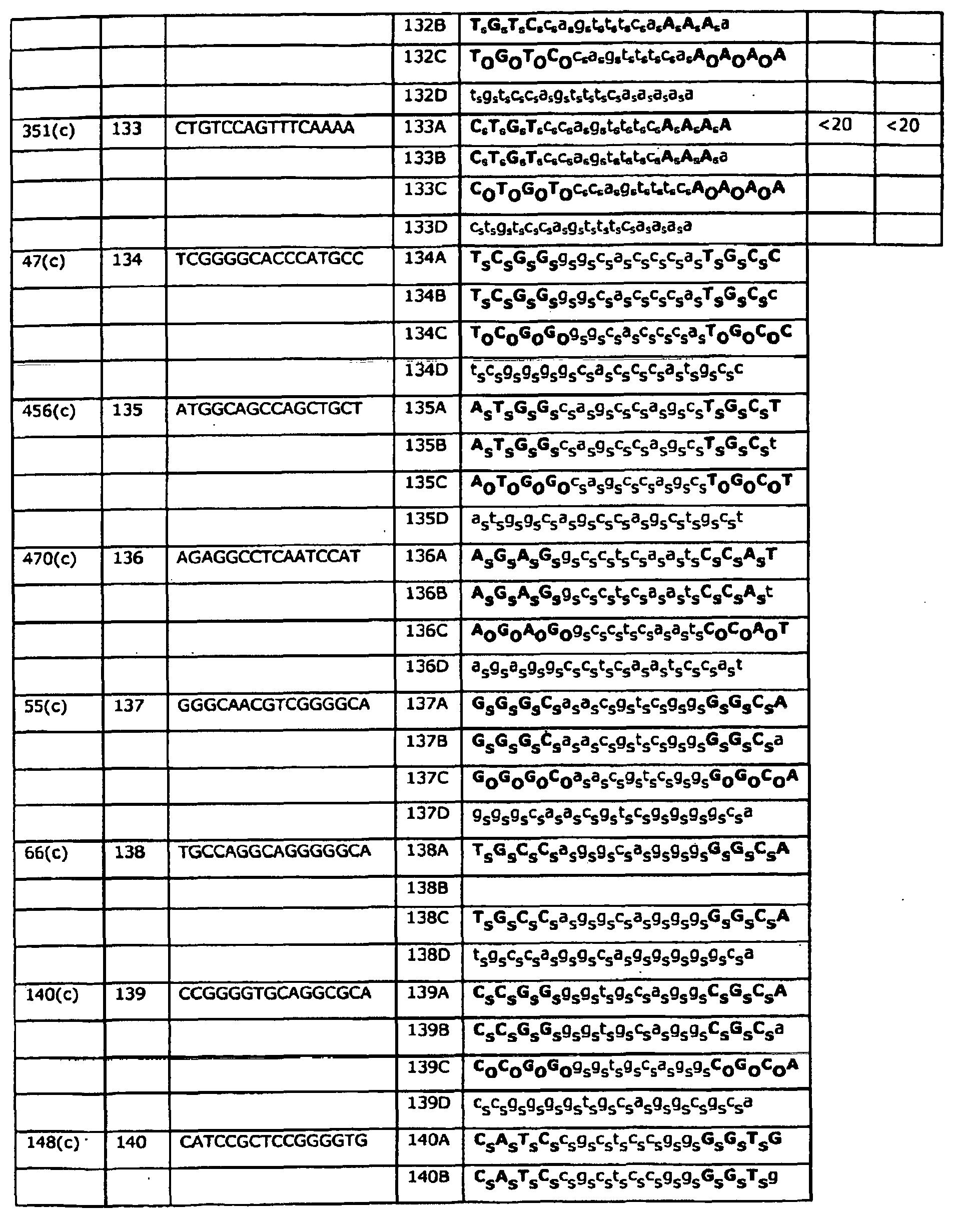

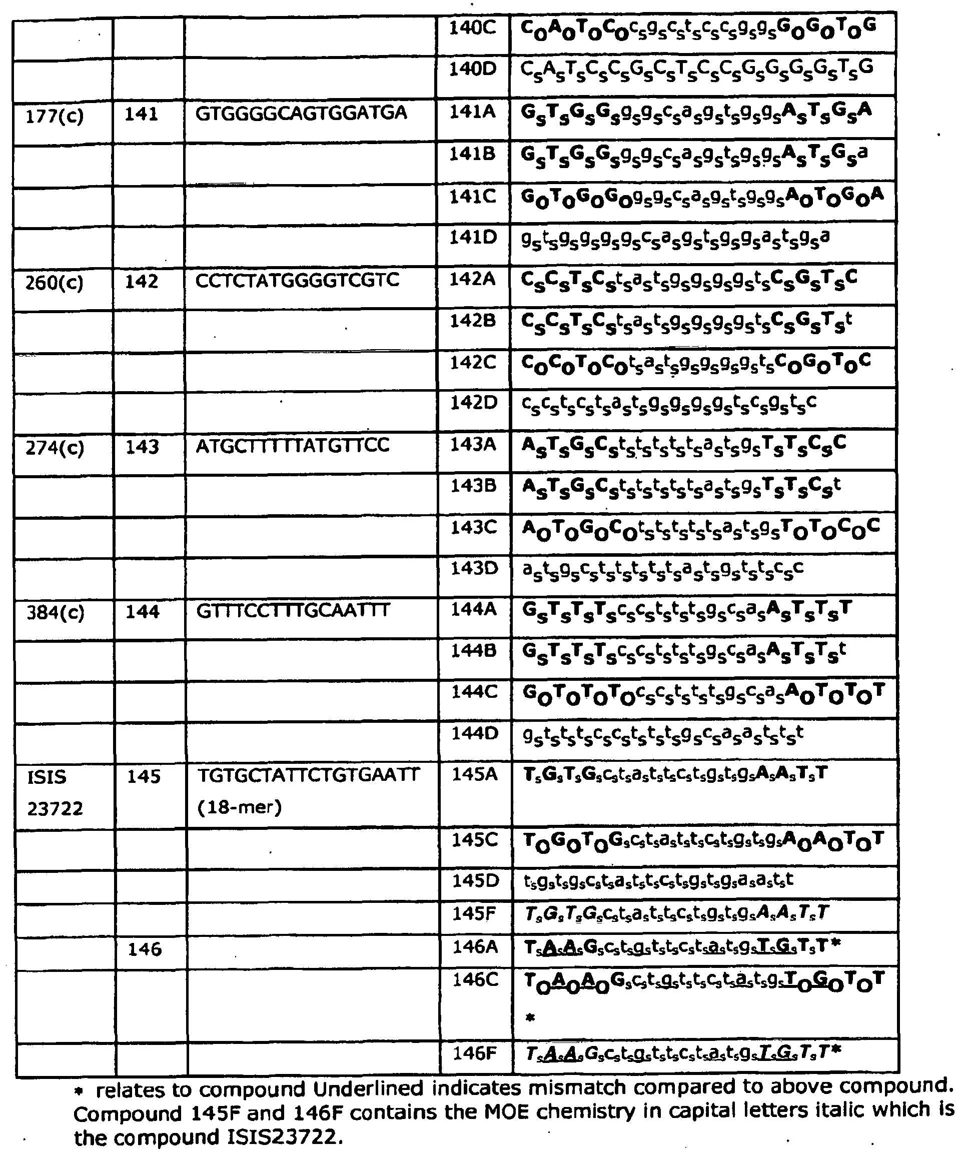

**Table 3 IC₅₀ (nM) of LNA (β-D-oxy-LNA) containing oligomeric in two cell lines of different origin**

| Oligomeric compounds were evaluated for their potential to knockdown Survivin mRNA in 15PC3 and MCF7 cells. Transcript steady state was monitored by Real-time PCR and normalised to the GAPDH transcript steady state. | | |
|---|---|---|
| **Seq ID/design** | **MCF7** | **15PC3** |
| 2A | 28 | 5 |
| 2B | | <5 |
| 4A | | <5 |
| 4B | | 5 |
| 6A | 8 | 3 |
| 6B | | <5 |
| 9A- | 11 | 3 |
| 15A | 1 | <1 |
| 15B | | <1 |
| 15E | | 1 |
| 118A | | <5 |
| 120A | | <25 |
| 123A | | <5 |
| 128A | | <5 |
| 128B | | <25 |
| 129A | | <25 |
| 131A | | <25 |
| 131B | | <5 |

As showed In table 1 and 2, SEQ ID NO 2, 3, 4, 5, 6, 8, 9, 10, 11, 12, 13, 14, 15, 118, 119, 120, 121, 122, 123, 125, 126, 128, 129, 130 and 131 demonstrated at least 30% inhibition of survivin expression at 25 nM in these experiments and are therefore preferred.

Compounds of particular interest are 2A, 2B, 4A, 4B, 6A, 6B, 15A, 15B, 15E, 119A, 119B, 121A, 121B, 128A, 128B, 130A, 130B, 131A and 131B.

### Example 11 Improved inhibition in vitro of survivin expression using LNA antisense oligomeric compounds compared to phosphorothioates and MOE

Comparison of mRNA inhibition by using phosphorothioates and MOE (Calbiochem) versus LNA containing antisense oligomeric compound in 15PC3 cells was performed. A LNA version of ISIS23722 was compared to the MOE containing compound, which is an 18-mer 4MOE/PS+10PS+4MOE/PS and compared to an iso-sequential phosphorthioate. Transfection was performed of 15PC3 with oligonucleotides or media (mock) (see example 5). Survivin mRNA was monitored with realtime PCR and normalised to GAPDH. Survivin mRNA presented relative to mock expression (see Table 4).

**Table 4 Percentage down regulation of mRNA**

| | ***0,2 nM*** | ***1 nM*** | ***5 nM*** | ***25 nM*** | ***100 nM*** |
|---|---|---|---|---|---|
| LNA version of ISIS23722 (4LNA/PS+10PS+4LNA/PS): 145A | <20% | 48% | 79% | 84% | 76% |
| MOE compound ISIS23722 (4MOE/PS+10PS+4MOE/PS): 145F | <20% | <20% | <20% | <20% | 46% |
| Phosphorthioate version of ISIS23722 (18PS) 145D | 22% | <20% | <20% | <20% | <20% |
| LNA version of ISIS23722 with 6 mismatches: 146C | - | - | - | <20% | <20% |
| MOE compound ISIS23722 with 6 mismatches: 146F | - | - | - | <20% | <20% |

In another experiment, the supernatants from each culture well were also included in the analysis in order to allow late apoptotic cells to be analyzed. The 18-mer LNA, PS and MOE compounds above were compared to LNA 16-mers of the invention. 15PC3 cells were transfected with the indicated oligos at the given concentrations (see Example 5). Total RNA was extracted at 24 hours. Cells in the media supernatant were included in the analysis. Survivin mRNA was monitored with realtime PCR and normalised to GAPDH. Survivin mRNA presented relative to mock expression. (see Table 5)

**Table 5 Down regulation of mRNA (percentage of mock expression)**

| Description: Seq ID | *100 nM* | 25 *nM* | 5 *nM* |
|---|---|---|---|
| LNA version of ISIS23722 (4LNA/PS+10PS+4LNA/PS): 145A | 91% | 94% | 89% |
| LNA version of ISIS23722 (4LNA/PO+.10PS+4LNA/PO): 145C | 89% | 88% | 79% |
| MOE compound ISIS23722 (4MOE/PS+10PS+4MOE/PS):145F | 68% | 36% | <20% |
| Phosphorthioate version of ISIS23722 (18PS): 145D | 35% | <20% | <20% |
| LNA compound 2A (16-mer) | 99% | 90% | 66% |
| LNA compound 6A (16-mer) | - | 98% | 90% |
| LNA compound 15B (16-mer) | 97% | 97% | 99% |

### Example 12 Apoptosis induction by LNA antisense oligomeric compounds

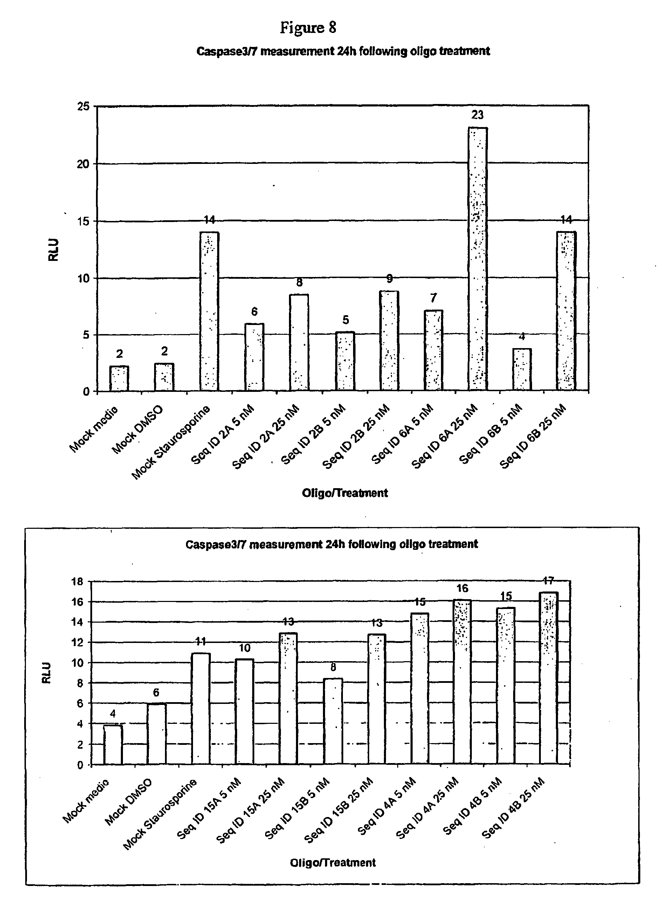

Cells were seeded to a density of 12000 cells per well in white 96 well plate (Nunc 136101) in DMEM the day prior to transfection. The next day cells were washed once in prewarmed OptiMEM followed by addition of 72 µl OptiMEM containing 5 µg/ml Lipofectamine2000 (In vitrogen). Cells were incubated for 7 min before adding 18 µl oligonucleotides diluted in OptiMEM.. The final oligonucleotide concentration ranged from 5 nM to 25 nM. After 4 h of treatment, cells were washed in OptiMEM and 100 µl DMEM containing serum was added. Following oligo treatment cell; were allowed to recover for the period indicated before they were removed from the CO2 incubated and equilibrated to room temperature for 15 min. The highly sensitive Caspase 3/7-Glo^{™} Reagent (Promega) was added directly to the cells in 96well and plates were incubated for 20min. before recording luminescence (luciferase activity) in Luminoskan Ascent instrument from Thermo Labsystems after further 1 min lag period. The luciferase activity is measured as Relative Light Units per seconds (RLU/s). The data was processed in the Ascent software 2.4.2. and graphs were drawn in excel. (see Figure 8)

### Example 13 Improved induction of apoptosis in vitro using LNA antisense oligomeric compounds compared to phosphorothioates and MOE

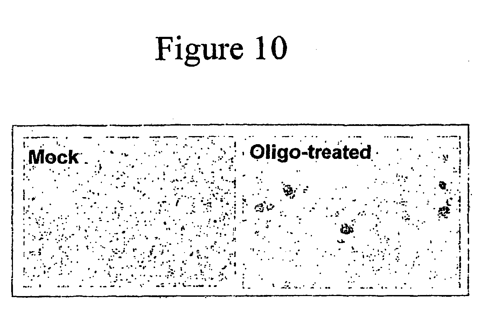

Measurement of apoptosis using BD^{™} cytometric bead array (CBA) (cat. 557816). Cells were transfected using lipofectamine 2000 as described (see Example 5). 24 h following transfection, the cells from the supernatant was spun down and the adherent cells were trypsionised and spun down. The cell pellet was resuspended/washed in PBS and counted to bring cell concentration to 2 x 10⁶ cells/ml lysis buffer containing protease inhibitors. The procedure was proceded as described by manufacturer with the following modifications. When cells were lysed, they were lysed for 40 min and vortexed with a 10 min interval. 1x 10⁵ cells were incubated with Caspase 3, Bcl-2 and PARP beads, mixed briefly and incubated for 1 h at room temperature. Caspase 3 activity, Bcl2 expression and induction of PARP in oligo treated cells were analysed using the using the BD TM CBA software. Data were transferred to excel and graphs were drawn. All data were related to mock (which is set to one). Figure 9 shows that the LNA containing compounds (145A and 145C) improves induction of apoptosis compared to the iso-sequential MOE compound ISIS27322 (here 145F) and the iso-sequential phosphorthioate compound (145D). Mismatch controls of a LNA compound (146C) and the MOE compound (146F) as well as the LNA compound 15A was also included in the study. Furthermore, Caspase 3 activation of compound 15A was detected by immunohistochemical analysis of LNA oligomeric compound treated cells (Figure 10).

### Example 14 Antisense oligonucleotide inhibition of Survivin in proliferating cancer cells

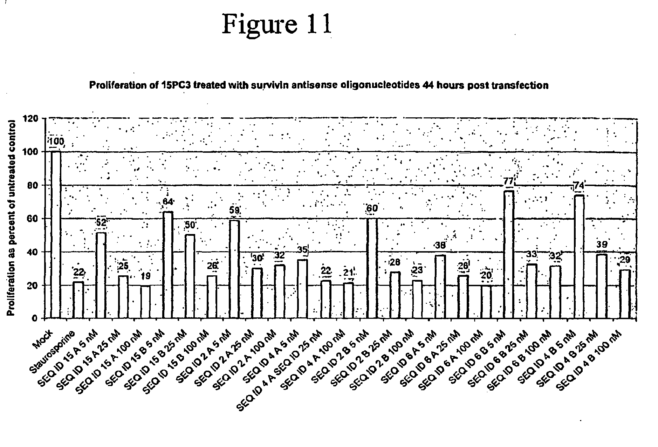

Cells were seeded to a density of 12000 cells per well in white 96 well plate (Nunc 136101) in DMEM the day prior to transfection. The next day cells were washed once in prewarmed OptiMEM followed by addition of 72 µl OptiMEM containing 5 µg/ml Lipofectamine2000 (In vitrogen). Cells were incubated for 7 min before adding 18 µl oligonucleotides diluted in OptiMEM. The final oligonucleotide concentration ranged from 5 nM to 100 nM. After 4 h of treatment, cells were washed in OptiMEM and 100 µl serum containing DMEM was added. Following oligo treatment cells were allowed to recover for the period indicated, viable cells were measured by adding 20 µl the tetrazolium compound [3-(4,5-dimethyl-2-yl)-5-(3-carboxymethoxyphenyl)-2-(4-sulfophenyl)-2H-tetrazolium, inner salt; MTS] and an electron coupling reagent (phenazine ethosulfate; PES) (CellTiter 96^{®} AQᵤₑₒᵤₛ One Solution Cell Proliferation Assay, Promega). Viable cells were measured at 490 nm in a Powerwave (Biotek Instruments). Growth rate (ΔOD/h) were plotted against oligo concentration. (see Figure 11).

### Example 15 Measurement of Ploidy (cell cycle) and DNA degradation (apoptosis) of cells following treatment with oligomeric compounds targeting Survivin

The late stage in the apoptotic cascade leads to large numbers of small fragments of DNA which can be analysed by propidium iodide staining of the cells, furthermore, propidium iodide staining can be used to asses ploidy in treated cells. To assess ploidy/ apoptosis of cells treated with oligomeric compound directed against Survivin, cells were washed in PBA and fixed for 1 h in 70 % EtOH at 4°C. After treatment with 50 µg/ml RNAse (Sigma) for 20 min at room temperature cells were washed with PBS and incubated with 40 µg/ml propidium iodide (Sigma or BD) for 30 min. All samples were analysed using fluorescence activated cell sorter (FACSCalibur, Becton Dickinson) and Cell Quest software. In the DNA histogram the hypodiploid or the sub-G1 peak represented the apoptotic cells.

### Example 16 Measurement of changes in the mitochondrial membrane potential of cells following treatment with oligomeric compounds targeting Survivin

To measure changes in the mitochondrial membrane potential the MitoSensor^{™} reagent method (Becton Dickinson, Cat # K2017-1) was used. MitoSensor^{™} reagent is taken up by healthy cells, in which it forms aggregates that emit red fluorescence. Upon apoptosis the mitochondrial membrane potential changes and does not allow the reagent to aggregate within the mitochondria and therefore it remains in the cytoplasm in its monomeric form where it emits green fluorescence. Cells treated with oligomeric compounds directed against Survivin were washed and incubated in MitoSensor Reagent diluted in Incubation buffer as described by manufacturer. Changes in membrane potential following oligo treatment was detected by fluorescence activated cell sorter (FACSCalibur, Becton Dickinson) and by the use of Cell Quest software.

### Example 17 Inhibition of capillary formation of Endothelial cells following antisense oligo treatment

Endothelial monolayer cells (e.g. HUVEC) were incubated with antisense oligos directed against survivin. Tube formation was analysed by either of the two following methods. The first method was the BD BioCoat angiogenesis tube formation system. Cells were transfected with oligos as described (example 5). Transfected cells were seeded at 2 x 10⁴ cells /96 well onto matrigel polymerized BD Biocoat angiogeneis plates. The plates were incubated for the hours/days indicated with or without PMA (5- 50 nM), VEGF (20-200 ng/ml), Suramin or vechicle. The plates were stained with Cacein AM as stated by the manufacturer and images were taken. Total tube length was measured using MetaMorph. Althernatively, cells were seeded in rat tail type I collagen (3 mg/ml, Becton Dickinson) in 0.1 volumen of 10 x DMEM, neutralised with sterile 1 M NaOH and kept on ice or in matrigel. Cells were added to the collagen suspension at a final concentration of 1x 10⁶ cells/ml collagen. The cell-collagen mixture was added to 6-well or 35 mm plates and placed in a humidified incubator at 37°C. When geled 3 ml of culture medium plus an extra 10 % FBS were added and cells were allow to form capillary-like vascular tubes over the period indicated in the presence or absence of PMA (16nM), VEGF (50 ng/ml). Tube formation was quantified following cryostat sectioning of the gels and examination of sections by phase-contrast microscopy.

### Example 18 Measurement of in vitro cytotoxicity following treatment with oligomeric compounds targeting Survivin

Cells were seeded (0.3 - 1.2 x 10⁴) and treated with antisense oligos as described (example for MTS assay Exampel 12). At the times indicated, 20 - 50 µl medium from the antisense treated cells were transferred to 96-well plates in order to measure the release of LDH to the medium. An equal volume of LHD substrate was added as described by the manufacturer. Released LDH was measured using a 30-minute coupled enzymatic assay, which results in the conversion of a tetrazolium salt (INT) into a red formazan product. The amount of colour formed is proportional to the number of lysed cells. Visible wavelength absorbance data (measured at 490 nm) were collected using a standard 96-well plate reader (Powerwave, Bio-Tek Instruments). As positive control cells were treated for about 45 minutes with 0,9 % Triton X- 100 (=100% lysis). Cytotoxicity was plotted relative to mock and Triton-x 100 treated cells (100 % lysis = 100 % cytotoxicity).

### Example 19: In vivo model: Tumour growth inhibition of human tumour cells grown in vivo by systemic treatment with antisense oligonucleotides

Female NMRI athymic nude mice of 6 weeks old were purchased from M&B, Denmark and allowed to acclimatize for at least one week before entering experiments. Human cancer cells typically 10⁶ cells suspended in 300 µl matrigel (BD Bioscience), were subcutaneously injected into the flanks of 7-8 week old NMRI athymic female nude mice. When the tumour growth was established, typically 7-12 days post tumour cell injection; different antisense oligonucleotides were administrated at 5 mg/kg/day for up to 28 days using ALZET osmotic pumps implanted subcutaneously. Prior to dorsal implantation the pumps were incubated overnight at room temperature in sterile PBS to start the pumps. Control animals received saline alone for the same period. Each experimental group included at least 5 mice. Antitumour activities were estimated by the inhibition of tumour volume. Tumour growth was followed regularly by measuring 2 perpendicular diameters. Tumour volumes were calculated according to the formula (π×L×D²/6), where L represents the largest diameter and D the tumour diameter perpendicular to L. At the end of treatment the animals were sacrificed and tumour weights were measured. Mean tumour volume and weights of groups were compared using Mann-Whitney's test. All analysis was made in SPSS version 11.0 for windows. Optimally a Western blot analysis may also be performed to measure if the antisense oligonucleotides have an inhibitory effect on protein levels. At the end of treatment period mice were therefore anaesthetised and the tumours were excised and immediately frozen in liquid nitrogen. The tumours were homogenized in lysis buffer (i.e. 20 m*M* Tris-Cl [pH 7.5]; 2% Triton X-100; 1/100 vol. Protease Inhibitor Cocktail Set III (Calbiochem); 1/100 vol. Protease Inhibitor Cocktail Set II (Calbiochem)) at 4°C with the use of a motor-driven homogeniser. 500 µl lysis buffer was applied per 100 mg tumour tissue. Tumour lysates from each group of mice were pooled and centrifuged at 13.000 g for 5 min at 4°C to remove tissue debris. Protein concentrations of the tumour extracts were determined using the BCA Protein Assay Reagent Kit (Pierce, Rockford). The protein extracts (50-100 µg) were fractionated on a gradient SDS-PAGE gel spanning from 4-20% and transferred to PVDF membranes and visualized by aminoblack staining. The expression of survivin was detected with anti-human survivin antibody followed by horseradish peroxidase-conjugated anti-goat IgG (DAKO). Immunoreactivity was detected by the ECL Plus (Amersham biotech) and quantitated by a Versadoc 5000 lite system (Bio-Rad).

### Example 20: In vivo model : Tumor growth inhibition of human tumour fragments transplanted in nude mice after intraperetoneal treatment with LNA antisense oligos

Tumour growth inhibiting activity of LNA antisense oligonucleotides was tested in xenotransplanted athymic nude mice, NMRI nu/nu, from Oncotest's (Freiburg, Germany) breeding colony. Human tumour fragments from breast (MDA MB 231), prostate (PC3) or lung tumours (LXFE 397, Oncotest) were obtained from xenografts in serial passage in nude mice. After removal of tumors from donor mice, they were cut into fragments (1-2 mm diameter) and placed in RPMI 1640 culture medium until subcutaneous implantation. Recipient mice were anaesthetized by inhalation of isoflurane. A small incision was made in the skin of the back. The tumor fragments (2 fragments per mouse) were transplanted with tweezers. MDA MB 231 and LXFE 397 tumors were tamsplanted in female mice, PC3 tumors were transplanted in male mice. When a mean tumour diameter 4-6 mm was reached, animals were randomized and treated with oligonucleotides at 20 mg/kg intraperetoneally once a day for three weeks excluding weekends. A vehicle (saline) and positive control group (Taxol, 20 mg/kg/day) were included in all experiments. All groups consisted of 6 mice. The tumour volume was determined by two-dimensional measurement with a caliper on the day of randomization (Day 0) and then twice weekly. Tumor volumes were calculated according to the formula: (a x b²) x 0.5 where a represents the largest and b the perpendicular tumor diameter. Mice were observed daily for 28 days after randomization until tumour volume was doubled. Mice were sacrificed when the tumour diameters exceeded 1.6 cm. For the evaluation of the statistical significance of tumour inhibition, the U-test by Mann-Whitney-Wilcoxon was performed. By convention, p-values <0.05 indicate significance of tumor inhibition.

### Example 21: Biodistribution of oligonucleotides in mice

Female NMRI athymic nude mice of 6 weeks old were purchased from M&B, Denmark and allowed to acclimatize for at least one week before entering experiments. Human cancer cells typically 10⁶ cells suspended in 300 µl matrigel (BD Bioscience) were subcutaneously injected into the flanks of 7-8 week old NMRI athymic female nude mice. When tumour growth was evident, tritium labelled oligonucleotides were administrated at 5 mg/kg/day for 14 days using ALZET osmotic pumps implanted subcutaneously. The oligonucleotides were tritium labeled as described by Graham MJ et al. (J Pharmacol Exp Ther 1998; 286(1): 447-458). Oligonucleotides were quantitated by scintillation counting of tissue extracts from all major organs (liver, kidney, spleen, heart, stomach, lungs, small intestine, large intestine, lymph nodes, skin, muscle, fat, bone, bone marrow) and subcutaneous transplanted human tumour tissue.

### Example 22: Uptake of LNA oligomeric compound in human tumour xenografts

Human 15PC3 xenografted tumors according to Example 13 were homogenized in 10 volumes of 0,5% Igepal CA-630, 25 mM Tris pH 8.0, 25 mM EDTA, 100 mM NaCl, 1mg/ml Proteinase K1 and incubated overnight at 37 degrees celsius followed by phenolchloroform extraction. The concentration of antisense oligonucleotide 2650 in the combined aqueous phase was determined using a sequence specific ELISA assay. Two probes, one labelled with biotin and one labelled with digoxigenin (DIG) with complementary sequences to the antisense oligonucleotide are hybridised to the antisense oligo. The complex is captured by immobilized streptavidin and quantified using a horse raddish peroxidase-conjugated anti-digoxigenin antibody and standard ELISA procedures. Briefly, 10 nM DNA capture probe (5'-aactgtgc-Biotin-3') and 10 nM LNA detection probe (5'-DIG-GATGTTTCgatgtttc-3') were mixed with sample or standards in 1 % blocking reagent (Roche cat. 1 096 176) in PBS. The probes were annealed to the oligo by heating the mixture to 70 degrees celsius and gradual cooling to 20 degrees Celsius. The mixture was transferred to streptavidin-coated wells. The amount of captured DIG-probe is quantified using an HRP-conjugated Anti-DIG antibody fragment (Roche) and standard ELISA procedures. At least 1,3µg/g tumours tissue of the oligomeric compound 15A was detected (data not adjusted for recovery).

The present invention has been described with specificity in accordance with certain of its preferred embodiments. Therefore, the following examples serve only to illustrate the invention and are not intended to limit the same.

## Claims

1. A compound capable of modulating the expression of survivin wherein said compound is selected from the group consisting of
CₛTₛCₛAₛaₛtₛcₛcₛaₛtₛgₛgₛCₛAₛGₛC (SEQ ID NO: 130A),
CₛTₛCₛAₛaₛtₛcₛcₛaₛtₛgₛgₛCₛAₛGₛc (SEQ ID NO, 130B), and
C_{O}T_{O}C_{O}A_{O}aₛtₛcₛcₛaₛtₛgₛgₛC_{O}A_{O}G_{O}C (SEQ ID NO: 130C);
wherein
capital letters indicate beta-D-oxy-LNA,
small letters indicate DNA,
subscript O indicates a phosphate linkage,
subscript s indicates a phosphorothioate linkage, and
wherein each C is 5'-methyl-cytosine

2. The compound according to claim 1, wherein said compound is CₛTₛCₛAₛaₛtₛcₛcₛaₛtₛgₛgₛCₛAₛGₛc (SEQ ID NO: 130B).

3. A conjugate comprising the compound according to any of the preceding claim and at least one non-nucleotide or non-polynucleotide moiety covalently attached to said compound.

4. A pharmaceutical composition comprising a compound according to claim 1 or claim 2 or a conjugate according to claim 3, and a pharmaceutically acceptable diluent, carrier or adjuvant.

5. The pharmaceutical composition according to claim 4, further comprising at least one chemotherapeutic agent.

6. The pharmaceutical composition according to claim 4 or claim 5, wherein said carrier is physiological saline.

7. The pharmaceutical composition according to claim 4 or claim 5, wherein said carrier is phosphate buffered saline.

8. A compound according to claim 1 or claim 2 or a conjugate according to claim 3 for use as a medicament.

9. Use of a compound according to claim 1 or claim 2 or a conjugate according to claim 3 for the manufacture of a medicament for the treatment of cancer.

10. Use of a compound according to claim 1 or claim 2 or a conjugate according to claim 3 in combination with a chemotherapeutic agent for the manufacture of a medicament for the treatment of cancer.

## Patentansprüche

1. Verbindung, die in der Lage ist, die Expression von Survivin zu beeinflussen, wobei die besagte Verbindung aus der Gruppe ausgewählt ist, die aus
CₛTₛCₛAₛaₛtₛcₛcₛaₛtₛgₛgₛCₛAₛGₛC (SEQ ID NR.: 130A),
CₛTₛCₛAₛaₛtₛcₛcₛaₛtₛgₛgₛCₛAₛGₛc (SEQ ID NR.: 130B) und
C_{O}T_{O}C_{O}A_{O}aₛtₛcₛcₛaₛtₛgₛgₛC_{O}A_{O}G_{O}C (SEQ ID NR.: 130C) besteht,
wobei Großbuchstaben beta-D-oxy-LNA,
Kleinbuchstaben DNA,
der Index O eine Phosphatverknüpfung und
der Index s eine Phosphorothioatverknüpfung bezeichnen und
wobei C jeweils 5'-Methylcytosin bedeutet.

2. Verbindung nach Anspruch 1, wobei die besagte Verbindung aus CₛTₛCₛAₛaₛtₛcₛcₛaₛtₛgₛgₛCₛAₛGₛc (SEQ ID NR.: 130B) besteht.

3. Konjugat, welches die Verbindung gemäß einem der vorhergehenden Ansprüche und wenigstens einen Nicht-Nukleotid- oder Nicht-Polynukleotidrest aufweist, der kovalent mit der besagten Verbindung verbunden ist.

4. Pharmazeutische Zusammensetzung enthaltend eine Verbindung gemäß Anspruch 1 oder Anspruch 2 oder ein Konjugat gemäß Anspruch 3 und ein pharmazeutisch verträgliches Verdünnungsmittel, Trägermittel oder Hilfsmittel.

5. Pharmazeutische Zusammensetzung nach Anspruch 4, die ferner wenigstens einen chemotherapeutischen Wirkstoff enthält.

6. Pharmazeutische Zusammensetzung nach Anspruch 4 oder Anspruch 5, wobei das Trägermittel physiologische Kochsalzlösung ist.

7. Pharmazeutische Zusammensetzung nach Anspruch 4 oder Anspruch 5, wobei das Trägermittel mit Phosphat gepufferte Kochsalzlösung ist.

8. Verbindung nach Anspruch 1 oder Anspruch 2 oder ein Konjugat nach Anspruch 3 für eine Verwendung als Medikament.

9. Verwendung einer Verbindung nach Anspruch 1 oder Anspruch 2 oder eines Konjugates nach Anspruch 3 zur Herstellung eines Medikaments zur Behandlung von Krebs.

10. Verwendung einer Verbindung nach Anspruch 1 oder Anspurch 2 oder eines Konjugates nach Anspruch 3 zur Herstellung eines Medikaments zur Behandlung von Krebs in Verbindung mit einem chemotherapeutischen Wirkstoff.

## Revendications

1. Composé susceptible de moduler l'expression de la survivine, ledit composé étant choisi dans le groupe composé de
CₛTₛCₛAₛaₛtₛcₛcₛaₛtₛgₛgₛCₛAₛGₛC (SEQ ID N° 130A),
CₛTₛCₛAₛaₛtₛcₛcₛaₛtₛgₛgₛCₛAₛGₛc (SEQ ID N° 130B) et
C_{O}T_{O}C_{O}A_{O}aₛtₛcₛcₛaₛtₛgₛgₛC_{O}A_{O}G_{O}C (SEQ ID N° 130C),
où les lettres majuscules désignent un bêta-D-oxy-LNA, les lettres minuscules désignent l'ADN,
l'indice 0 désigne une liaison phosphate,
l'indice s désigne une liaison phosphorothioate, et chaque C est la 5'-méthylcytosine.

2. Composé selon la revendication 1, ledit composé étant CₛTₛCₛAₛaₛtₛcₛcₛaₛtₛgₛgₛCₛAₛGₛC (SEQ ID N° 130B).

3. Conjugué comprenant le composé selon l'une quelconque des revendications précédentes et au moins un fragment non nucléotidique ou non polynucléotidique lié de manière covalente audit composé.

4. Composition pharmaceutique comprenant un composé selon la revendication 1 ou la revendication 2 ou un conjugué selon la revendication 3, et un diluant, support ou adjuvant pharmaceutiquement acceptable.

5. Composition pharmaceutique selon la revendication 4, comprenant en outre au moins un agent chimiothérapeutique.

6. Composition pharmaceutique selon la revendication 4 ou la revendication 5, dans laquelle ledit support est une solution saline physiologique.

7. Composition pharmaceutique selon la revendication 4 ou la revendication 5, dans laquelle ledit support est une solution saline tamponnée au phosphate.

8. Composé selon la revendication 1 ou la revendication 2 ou un conjugué selon la revendication 3 pour une utilisation en tant que médicament.

9. Utilisation d'un composé selon la revendication 1 ou la revendication 2 ou d'un conjugué selon la revendication 3 pour la fabrication d'un médicament destiné au traitement du cancer.

10. Utilisation d'un composé selon la revendication 1 ou la revendication 2 ou d'un conjugué selon la revendication 3 en association avec un agent chimiothérapeutique, pour la fabrication d'un médicament destiné au traitement du cancer.
